# EUROPEAN PATENT APPLICATION

(11) **EP 4 821 927 A1**
(43) Date of publication of application: **14.10.2026**
(21) Application number: 24899900.5
(22) Date of filing: 05.12.2024
(51) Int. Cl.: C07D 405/04, C07D 405/14, A61K 31/343, A61K 31/395, A61P 3/10

(54) **BENZO HETEROCYCLIC COMPOUND AND USE THEREOF**

(30) Priority: 06.12.2023 CN 202311665522
(71) Applicant: Cascade Pharmaceuticals, Inc, Shanghai 201321 (CN)
(72) Inventor: SHI, Jingjing, Shanghai 201422 (CN); GU, Yuefei, Shanghai 201422 (CN); WU, Guohui, Shanghai 201422 (CN); ZHANG, Zheng, Shanghai 201422 (CN); GONG, Linpei, Shanghai 201422 (CN); WANG, Peng, Shanghai 201422 (CN); ZHU, Xin, Shanghai 201422 (CN); DU, Xiaolong, Shanghai 201422 (CN); YANG, Shengsheng, Shanghai 201422 (CN)
(74) Representative: De Clercq & Partners
(86) International application number: PCT/CN2024/137037
(87) International publication number: WO 2025/119263

(57) **Abstract**

A benzo heterocyclic compound and a use thereof. The compound has the general formula of formula I. The compound has good GPR40 agonistic activity and has a good proprietary drug prospect.

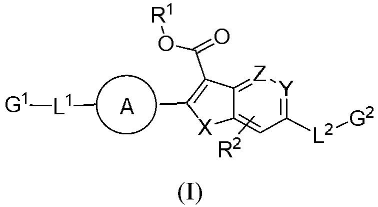

## Description

### SPECIFICATION

The present application claims priority to Chinese Patent Application No. 2023116655229, filed on December 6, 2023. The contents of the above Chinese patent application are incorporated herein by reference in their entirety.

### TECHNICAL FIELD

The present invention relates to a benzoheterocyclic compound and a use thereof.

### BACKGROUND

GPR40 is a member of the GPCR family, also known as the FFA1 receptor, and is a class A G protein-coupled receptor. It can be activated by endogenous medium- and long-chain fatty acids (such as capric acid, palmitic acid, oleic acid, and docosahexaenoic acid) *in vivo.* GPR40 is predominantly expressed at high levels in pancreatic β-cells, intestinal endocrine cells, and the brain, while also being expressed in tissues such as the gastrointestinal tract, liver, heart, skeletal muscle, and taste buds. When activated by its endogenous ligand, GPR40 induces insulin secretion only at elevated blood glucose levels (activation of GPR40 promotes Ca²⁺ influx in pancreatic β-cells, leading to insulin secretion), thereby eliminating the risk of hypoglycemia. Partial agonists of GPR40 activate the Gq/IP3 pathway to promote insulin secretion; full agonists additionally activate the Gs/cAMP pathway, stimulating the release of GLP-1 and GIP, thereby achieving a more potent glucose-lowering effect. These make GPR40 an important therapeutic target for diseases such as diabetes, obesity, cardiovascular disease, and dyslipidemia. The distribution of GPR40 in the brain may be related to pain regulation, neuroprotection, behavior regulation and the like, and is a potential target for treating nervous system diseases.

Given the importance of GPR40, the development of drugs that can activate GPR40 is of great significance.

### SUMMARY OF THE INVENTION

The technical problem to be solved by the present invention is to overcome the limited types of drugs with GPR40 agonist activity in the prior art. To this end, the present invention provides a benzoheterocyclic compound, a preparation method therefor, and a use thereof. The compounds of the present invention have good GPR40 agonist activity, and further have the advantages of good pharmacokinetics and low toxicity.

The present invention solves the above technical problem through the following technical solutions.

The present invention provides a compound of formula I, a pharmaceutically acceptable salt thereof, a solvate thereof, or a solvate of the pharmaceutically acceptable salt thereof: 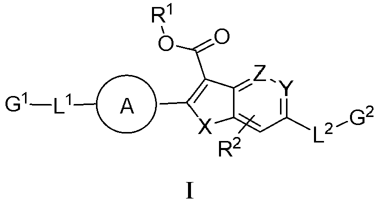
wherein X is O or NR³, and R³ is H or C₁-C₆ alkyl;
Z and Y are independently CH or N;
G¹ is H, C₁-C₆ alkyl, C₁-C₆ alkyl substituted by one or more G¹⁻¹, C₆-C₁₄ aryl, C₆-C₁₄ aryl substituted by one or more G¹⁻², 5- to 10-membered heteroaryl, 5- to 10-membered heteroaryl substituted by one or more G¹⁻³, C₁-C₆ alkoxy, C₁-C₆ alkoxy substituted by one or more G¹⁻⁴, C₂-C₆ alkenyl, C₂-C₆ alkenyl substituted by one or more G¹⁻⁵, C₂-C₆ alkynyl, C₂-C₆ alkynyl substituted by one or more G¹⁻⁶, C₃-C₈ cycloalkyl, C₃-C₈ cycloalkyl substituted by one or more G¹⁻⁷, C₃-C₈ cycloalkenyl, C₃-C₈ cycloalkenyl substituted by one or more G¹⁻⁸, 3- to 8-membered heterocycloalkyl, 3- to 8-membered heterocycloalkyl substituted by one or more G¹⁻⁹, 3- to 8-membered heterocycloalkenyl, or 3- to 8-membered heterocycloalkenyl substituted by one or more G¹⁻¹⁰;
each G¹⁻¹, each G¹⁻², each G¹⁻³, each G¹⁻⁴, each G¹⁻⁵, each G¹⁻⁶, each G¹⁻⁷, each G¹⁻⁸, each G¹⁻⁹, and each G¹⁻¹⁰ is independently deuterium, halogen, cyano, -NG¹⁻¹⁻¹G¹⁻¹⁻², -NC(=O)G¹⁻¹⁻³G¹⁻¹⁻⁴, hydroxyl, -S(=O)₂-C₁-C₆ alkyl, C₁-C₆ alkyl, C₁-C₆ alkyl substituted by one or more G¹⁻¹⁻⁵, C₁-C₆ alkoxy, C₁-C₆ alkoxy substituted by one or more G¹⁻¹⁻⁶, -S-C₁-C₆ alkyl, -S-C₁-C₆ alkyl substituted by one or more G¹⁻¹⁻⁷, C₃-C₈ cycloalkyl, C₃-C₈ cycloalkyl substituted by one or more G¹⁻¹⁻⁸, -O-C₃-C₈ cycloalkyl, -O-C₃-C₈ cycloalkyl substituted by one or more G¹⁻¹⁻⁹, or -C(=O)NG¹⁻¹⁻¹¹G¹⁻¹⁻¹²;
or, any two adjacent G¹⁻², together with the carbon atom to which they are attached, form a 3- to 8-membered heterocycloalkyl, a 3- to 8-membered heterocycloalkyl substituted by one or more G¹⁻¹⁻⁹, a C₃-C₈ cycloalkyl, or a C₃-C₈ cycloalkyl substituted by one or more G¹⁻¹⁻¹⁰;
G¹⁻¹⁻¹, G¹⁻¹⁻², G¹⁻¹⁻³, G¹⁻¹⁻⁴, G¹⁻¹⁻¹¹, and G¹⁻¹⁻¹² are independently H, C₁-C₆ alkyl, C₃-C₈ cycloalkyl, 5- to 10-membered heteroaryl, or 5- to 10-membered heteroaryl substituted by one or more G¹⁻¹⁻¹⁰⁻¹;
each G¹⁻¹⁻¹⁰⁻¹ is independently C₁-C₆ alkyl;
each G¹⁻¹⁻⁵, each G¹⁻¹⁻⁶, each G¹⁻¹⁻⁷, each G¹⁻¹⁻⁸, each G¹⁻¹⁻⁹, and each G¹⁻¹⁻¹⁰ is independently halogen, oxo, C₁-C₆ alkyl, or C₃-C₈ cycloalkyl;
L¹ is a bond or C₁-C₆ alkylene;
ring A is C₄-C₆ cycloalkyl, C₄-C₆ cycloalkyl substituted by one or more A¹, C₄-C₆ cycloalkenyl, C₄-C₆ cycloalkenyl substituted by one or more A², 4- to 6-membered heterocycloalkyl, 4- to 6-membered heterocycloalkyl substituted by one or more A¹, 4-to 6-membered heterocycloalkenyl, or 4- to 6-membered heterocycloalkenyl substituted by one or more A⁴; the 4- to 6-membered heterocycloalkyl, the 4- to 6-membered heterocycloalkyl substituted by one or more A¹, the 4- to 6-membered heterocycloalkenyl, and the 4- to 6-membered heterocycloalkenyl substituted by one or more A⁴ have 1 or 2 heteroatoms independently selected from one or more types of N, S, and O;
each A¹, each A², each A³, and each A⁴ is independently deuterium, halogen, cyano, -NA¹⁻¹A¹⁻², -NC(=O)A¹⁻³A¹⁻⁴, hydroxyl, C₁-C₆ alkyl, C₁-C₆ alkyl substituted by one or more A¹⁻⁵, C₁-C₆ alkoxy, or C₁-C₆ alkoxy substituted by one or more A¹⁻⁶;
A¹⁻¹, A¹⁻², A¹⁻³, and A¹⁻⁴ are independently deuterium, halogen, cyano, C₁-C₆ alkyl, or C₁-C₆ alkoxy;
each A¹⁻⁵ and each A¹⁻⁶ is independently hydrogen, deuterium, halogen, cyano, C₁-C₆ alkyl, or C₁-C₆ alkoxy;
R¹ is C₁-C₆ alkyl, C₇-C₁₂ linear alkyl, C₂-C₁₂ alkenyl, C₂-C₆ alkynyl, C₃-C₁₀ cycloalkyl, C₆-C₁₄ aryl, 5- to 10-membered heteroaryl, C₁-C₆ alkyl substituted by one or more R¹⁻¹, C₇-C₁₂ linear alkyl substituted by one or more R¹⁻¹, C₂-C₁₂ alkenyl substituted by one or more R¹⁻¹, C₂-C₆ alkynyl substituted by one or more R¹⁻¹, C₃-C₁₀ cycloalkyl substituted by one or more R¹⁻², C₆-C₁₄ aryl substituted by one or more R¹⁻³, or 5- to 10-membered heteroaryl substituted by one or more R¹⁻⁴;
each R¹⁻¹, each R¹⁻², each R¹⁻³, and R¹⁻⁴ is independently halogen, C₁-C₆ alkyl, C₃-C₈ cycloalkyl, C₆-C₁₄ aryl, C₁-C₆ alkoxy, 5- to 10-membered heteroaryl, C₁-C₆ alkyl substituted by one or more R¹⁻¹⁻¹, C₃-C₈ cycloalkyl substituted by one or more R¹⁻¹⁻¹, C₆-C₁₄ aryl substituted by one or more R¹⁻¹⁻¹, C₁-C₆ alkoxy substituted by one or more R¹⁻¹⁻¹, or 5- to 10-membered heteroaryl substituted by one or more R¹⁻¹⁻¹;
R¹⁻¹⁻¹ is halogen, C₁-C₆ alkyl, or C₆-C₁₄ aryl;
R² is hydrogen, deuterium, halogen, cyano, C₁-C₆ alkyl, or C₁-C₆ alkoxy;
L² is C₁-C₆ alkylene, C₁-C₆ alkylene substituted by one or more L²⁻¹, C₃-C₈ cycloalkylene, or C₃-C₈ cycloalkylene substituted by one or more L²⁻²;
each L²⁻¹ and each L²⁻² is independently halogen, C₁-C₆ alkyl, C₁-C₆ alkyl substituted by one or more L²⁻¹⁻¹, C₁-C₆ alkoxy, C₁-C₆ alkoxy substituted by one or more L²⁻¹⁻², C₃-C₈ cycloalkyl, C₃-C₈ cycloalkyl substituted by one or more L²⁻¹⁻³, C₂-C₆ alkynyl, or C₂-C₆ alkynyl substituted by one or more L²⁻¹⁻⁴;
each L²⁻¹⁻¹, each L²⁻¹⁻², each L²⁻¹⁻³, and each L²⁻¹⁻⁴ is independently C₃-C₈ cycloalkyl or C₃-C₈ cycloalkyl substituted by one or more L²⁻¹⁻¹⁻¹;
G² is -C(=O)G²⁻¹, -C(=O)NG²⁻²G²⁻³, or 5- to 10-membered heteroaryl;
G²⁻¹ is hydroxyl, C₁-C₆ alkyl, or -O-NH₂;
G²⁻² and G²⁻³ are independently H, -S(=O)₂-C₁-C₆ alkyl, C₃-C₈ cycloalkyl, C₁-C₆ alkyl, or C₁-C₆ alkyl substituted by one or more G²⁻²⁻¹;
each G²⁻²⁻¹ is independently carboxyl or -S(=O)₂OH;
each 5- to 10-membered heteroaryl, each 3- to 8-membered heterocycloalkenyl, and each 3- to 8-membered heterocycloalkyl independently has 1, 2, 3, or 4 heteroatoms independently selected from one or more types of N, S, and O.

In some embodiments, the compound is represented by formula Ia, preferably by formula Ia1: 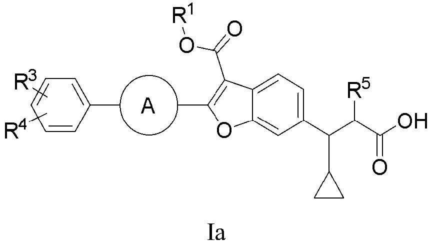 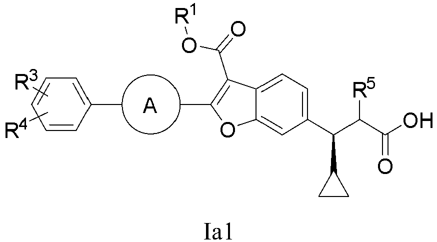 wherein
ring A is C₄-C₆ cycloalkyl, C₄-C₆ cycloalkyl substituted by one or more A¹, C₄-C₆ cycloalkenyl, C₄-C₆ cycloalkenyl substituted by one or more A², 4- to 6-membered heterocycloalkyl, 4- to 6-membered heterocycloalkyl substituted by one or more A¹, 4-to 6-membered heterocycloalkenyl, or 4- to 6-membered heterocycloalkenyl substituted by one or more A⁴; the 4- to 6-membered heterocycloalkyl, the 4- to 6-membered heterocycloalkyl substituted by one or more A¹, the 4- to 6-membered heterocycloalkenyl, and the 4- to 6-membered heterocycloalkenyl substituted by one or more A⁴ have 1 or 2 heteroatoms independently selected from one or more types of N, S, and O;
each A¹, each A², each A³, and each A⁴ is independently deuterium, halogen, cyano, -NA¹⁻¹A¹⁻², -NC(=O)A¹⁻³A¹⁻⁴, hydroxyl, C₁-C₆ alkyl, C₁-C₆ alkyl substituted by one or more A¹⁻⁵, C₁-C₆ alkoxy, or C₁-C₆ alkoxy substituted by one or more A¹⁻⁶;
A¹⁻¹, A¹⁻², A¹⁻³, and A¹⁻⁴ are independently deuterium, halogen, cyano, C₁-C₆ alkyl, or C₁-C₆ alkoxy;
each A¹⁻⁵ and each A¹⁻⁶ is independently hydrogen, deuterium, halogen, cyano, C₁-C₆ alkyl, or C₁-C₆ alkoxy;
R³ and R⁴ are independently H, deuterium, halogen, cyano, -NR³⁻¹R³⁻², - NC(=O)R³⁻³R³⁻⁴, hydroxyl, -S(=O)₂-C₁-C₆ alkyl, C₁-C₆ alkyl, C₁-C₆ alkyl substituted by one or more R³⁻⁵, C₁-C₆ alkoxy, C₁-C₆ alkoxy substituted by one or more R³⁻⁶, -S-C₁-C₆ alkyl, -S-C₁-C₆ alkyl substituted by one or more R³⁻⁷, C₃-C₈ cycloalkyl, C₃-C₈ cycloalkyl substituted by one or more R³⁻⁸, -O-C₃-C₈ cycloalkyl, -O-C₃-C₈ cycloalkyl substituted by one or more R³⁻⁹, or -C(=O)NR³⁻¹⁰R³⁻¹¹
or, any two adjacent R³ and R⁴, together with the carbon atom to which they are attached, form a 3- to 8-membered heterocycloalkyl, a 3- to 8-membered heterocycloalkyl substituted by one or more R³⁻⁸, a C₃-C₈ cycloalkyl, or a C₃-C₈ cycloalkyl substituted by one or more R³⁻⁸;
R³⁻¹, R³⁻², R³⁻³, R³⁻⁴, R³⁻¹⁰, and R³⁻¹¹ are independently H, C₁-C₆ alkyl, C₃-C₈ cycloalkyl, 5- to 10-membered heteroaryl, or 5- to 10-membered heteroaryl substituted by one or more R³⁻¹⁰⁻¹;
each R³⁻¹⁰⁻¹ is independently C₁-C₆ alkyl;
each R³⁻⁵, each R³⁻⁶, each R³⁻⁷, each R³⁻⁸, and each R³⁻⁹ is independently halogen, oxo, C₁-C₆ alkyl, or C₃-C₈ cycloalkyl;
R¹ is C₁-C₆ alkyl, C₇-C₁₂ linear alkyl, C₂-C₁₂ alkenyl, C₂-C₆ alkynyl, C₃-C₁₀ cycloalkyl, C₆-C₁₄ aryl, 5- to 10-membered heteroaryl, C₁-C₆ alkyl substituted by one or more R¹⁻¹, C₇-C₁₂ linear alkyl substituted by one or more R¹⁻¹, C₂-C₁₂ alkenyl substituted by one or more R¹⁻¹, C₂-C₆ alkynyl substituted by one or more R¹⁻¹, C₃-C₁₀ cycloalkyl substituted by one or more R¹⁻², C₆-C₁₄ aryl substituted by one or more R¹⁻³, or 5- to 10-membered heteroaryl substituted by one or more R¹⁻⁴;
each R¹⁻¹, each R¹⁻², each R¹⁻³, and R¹⁻⁴ is independently halogen, C₁-C₆ alkyl, C₃-C₈ cycloalkyl, C₆-C₁₄ aryl, C₁-C₆ alkoxy, 5- to 10-membered heteroaryl, C₁-C₆ alkyl substituted by one or more R¹⁻¹⁻¹, C₃-C₈ cycloalkyl substituted by one or more R¹⁻¹⁻¹, C₆-C₁₄ aryl substituted by one or more R¹⁻¹⁻¹, C₁-C₆ alkoxy substituted by one or more R¹⁻¹⁻¹, or 5- to 10-membered heteroaryl substituted by one or more R¹⁻¹⁻¹;
R¹⁻¹⁻¹ is halogen, C₁-C₆ alkyl, or C₆-C₁₄ aryl;
R⁵ is H, halogen, C₁-C₆ alkyl, C₁-C₆ alkyl substituted by one or more R⁵⁻¹, C₁-C₆ alkoxy, C₁-C₆ alkoxy substituted by one or more R⁵⁻², C₃-C₈ cycloalkyl, C₃-C₈ cycloalkyl substituted by one or more R⁵⁻³, C₂-C₆ alkynyl, or C₂-C₆ alkynyl substituted by one or more R⁵⁻⁴
each R⁵⁻¹, each R⁵⁻², each R⁵⁻³, and each R⁵⁻⁴ is independently C₃-C₈ cycloalkyl.

In some embodiments, ring A is 4- to 6-membered heterocycloalkyl or C₄-C₆ cycloalkenyl.

In some embodiments, R³ and R⁴ are independently H, halogen, C₁-C₆ alkyl, C₁-C₆ alkyl substituted by one or more R³⁻⁵, C₁-C₆ alkoxy, or C₁-C₆ alkoxy substituted by one or more R³⁻⁶; or, any two adjacent R³ and R⁴, together with the carbon atom to which they are attached, form a 3- to 8-membered heterocycloalkyl, for example, the 3- to 8-membered heterocycloalkyl may be dioxolanyl.

In some embodiments, each R³⁻⁵ and each R³⁻⁶ is independently halogen.

In some embodiments, R¹ is C₁-C₆ alkyl, C₁-C₆ alkyl substituted by one or more R¹⁻¹, C₇-C₁₂ linear alkyl, C₂-C₁₂ alkenyl, C₂-C₆ alkynyl, C₁-C₆ alkoxy substituted by one or more R¹⁻¹, C₃-C₁₀ cycloalkyl, C₃-C₁₀ cycloalkyl substituted by one or more R¹⁻², C₆-C₁₄ aryl, or C₆-C₁₄ aryl substituted by one or more R¹⁻³

In some embodiments, each R¹⁻¹, each R¹⁻², and each R¹⁻³ is independently halogen, C₁-C₆ alkyl, C₁-C₆ alkyl substituted by one or more R¹⁻¹⁻¹, C₃-C₈ cycloalkyl, C₆-C₁₄ aryl, C₁-C₆ alkoxy, C₁-C₆ alkoxy substituted by one or more R¹⁻¹⁻¹, or 5- to 10-membered heteroaryl.

In some embodiments, R¹⁻¹⁻¹ is halogen, C₁-C₆ alkyl, or C₆-C₁₄ aryl.

In some embodiments, R⁵ is H or halogen.

In some embodiments, the compound of formula Ia satisfies the following conditions:
(1) ring A is 4- to 6-membered heterocycloalkyl or C₄-C₆ cycloalkenyl;
(2) R³ and R⁴ are independently H, halogen, C₁-C₆ alkyl, C₁-C₆ alkyl substituted by one or more R³⁻⁵, C₁-C₆ alkoxy, or C₁-C₆ alkoxy substituted by one or more R³⁻⁶; or, any two adjacent R³ and R⁴, together with the carbon atom to which they are attached, form a 3-to 8-membered heterocycloalkyl, for example, the 3- to 8-membered heterocycloalkyl may be dioxolanyl;
(3) each R³⁻⁵ and each R³⁻⁶ is independently halogen;
(4) R¹ is C₁-C₆ alkyl, C₁-C₆ alkyl substituted by one or more R¹⁻¹, C₇-C₁₂ linear alkyl, C₂-C₁₂ alkenyl, C₂-C₆ alkynyl, C₁-C₆ alkoxy substituted by one or more R¹⁻¹, C₃-C₁₀ cycloalkyl, C₃-C₁₀ cycloalkyl substituted by one or more R¹⁻², C₆-C₁₄ aryl, or C₆-C₁₄ aryl substituted by one or more R¹⁻³;
(5) each R¹⁻¹, each R¹⁻², and each R¹⁻³ is independently halogen, C₁-C₆ alkyl, C₁-C₆ alkyl substituted by one or more ¹⁻¹⁻¹, C₃-C₈ cycloalkyl, C₆-C₁₄ aryl, C₁-C₆ alkoxy, C₁-C₆ alkoxy substituted by one or more R¹⁻¹⁻¹, or 5- to 10-membered heteroaryl;
(6) R¹⁻¹⁻¹ is halogen, C₁-C₆ alkyl, or C₆-C₁₄ aryl; and
(7) R⁵ is H or halogen.

In some embodiments, ring A is 4- to 6-membered heterocycloalkyl.

In some embodiments, R³ and R⁴ are each independently H or C₁-C₆ alkoxy, and further, the C₁-C₆ alkoxy is located at the meta position.

In some embodiments, R⁵ is H.

In some embodiments, the compound of formula Ia satisfies the following conditions:
(1) ring A is 4- to 6-membered heterocycloalkyl;
(2) R³ and R⁴ are each independently H or C₁-C₆ alkoxy, and further, the C₁-C₆ alkoxy is located at the meta position; and
(3) R⁵ is H.

In some embodiments, in ring A, the C₄-C₆ cycloalkenyl is cyclohexenyl, and the 4- to 6-membered heterocycloalkyl is saturated cycloalkyl containing 1-2 N atoms, which may be, for example, piperidinyl.

In some embodiments, in R³ and R⁴, the "C₁-C₆ alkyl" in the C₁-C₆ alkyl and the C₁-C₆ alkyl substituted by one or more R³⁻⁵ is independently methyl, ethyl, n-propyl, isopropyl, *n*-butyl, isobutyl, *sec*-butyl, or *tert*-butyl.

In some embodiments, in R³ and R⁴, the "C₁-C₆ alkoxy" in the C₁-C₆ alkoxy and the C₁-C₆ alkoxy substituted by one or more R³⁻⁶ is independently methoxy, ethoxy, *n-*propoxy, isopropoxy, *n*-butoxy, isobutoxy, *sec*-butoxy, or *tert*-butoxy.

In some embodiments, in R³ and R⁴, the 3- to 8-membered heterocycloalkyl formed by any two adjacent R³ and R⁵, together with the carbon atom to which they are attached, is a 5- to 6-membered heterocycloalkyl containing 1-2 oxygen atoms, for example, the 5- to 6-membered heterocycloalkyl may be dioxolanyl.

In some embodiments, in R³ and R⁴, the halogen is fluorine or chlorine.

In some embodiments, in each R³⁻⁵ and each R³⁻⁶, the halogen is fluorine.

In some embodiments, in R¹, the "C₁-C₆ alkyl" in the C₁-C₆ alkyl and the C₁-C₆ alkyl substituted by one or more R¹⁻¹ is independently methyl, ethyl, *n*-propyl, isopropyl, *n*-butyl, isobutyl, *sec*-butyl, *tert*-butyl, *-n*-C₅H₁₁, -*n*-C₆H₁₃,

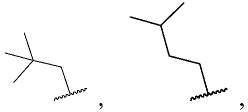

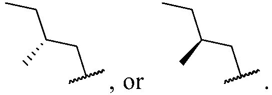

In some embodiments, in R¹, the "C₇-C₁₂ linear alkyl" in the C₇-C₁₂ linear alkyl and the C₇-C₁₂ linear alkyl substituted by one or more R¹⁻¹ is C₈-C₁₂ linear alkyl.

In some embodiments, in R¹, the "C₂-C₁₂ alkenyl" in the C₂-C₁₂ alkenyl and the C₂-C₁₂ alkenyl substituted by one or more R¹⁻¹ is C₄-C₁₀ alkenyl containing 1-2 double bonds.

In some embodiments, in R¹, the "C₂-C₆ alkynyl" in the C₂-C₆ alkynyl and the C₂-C₆ alkynyl substituted by one or more R¹⁻¹ is C₃-C₄ alkynyl containing one triple bond.

In some embodiments, in R¹, the "C₁-C₆ alkoxy" in the C₁-C₆ alkoxy and the C₁-C₆ alkoxy substituted by one or more R¹⁻¹ is methoxy, ethoxy, *n*-propoxy, isopropoxy, *n-*butoxy, isobutoxy, *sec*-butoxy, or *tert-*butoxy*.*

In some embodiments, in R¹, the "C₃-C₁₀ cycloalkyl" in the C₃-C₁₀ cycloalkyl and the C₃-C₁₀ cycloalkyl substituted by one or more R¹⁻² is independently cyclopropyl, cyclobutyl, cyclopentyl, or cyclohexyl.

In some embodiments, in R¹, the "C₆-C₁₄ aryl" in the C₆-C₁₄ aryl and the C₆-C₁₄ aryl substituted by one or more R¹⁻³ is phenyl.

In some embodiments, in each R¹⁻¹, each R¹⁻², each R¹⁻³, and each R¹⁻⁴, the halogen is fluorine.

In some embodiments, in each R¹⁻¹, each R¹⁻², each R¹⁻³, and each R¹⁻⁴, the "C₁-C₆ alkyl" in the C₁-C₆ alkyl and the C₁-C₆ alkyl substituted by one or more R¹⁻¹⁻¹ is independently methyl, ethyl, *n*-propyl, isopropyl, *n*-butyl, isobutyl, *sec*-butyl, or *tert-*butyl.

In some embodiments, in each R¹⁻¹, each R¹⁻², each R¹⁻³, and each R¹⁻⁴, the C₃-C₈ cycloalkyl is cyclopropyl, cyclobutyl, cyclopentyl, or cyclohexyl.

In some embodiments, in each R¹⁻¹, each R¹⁻², each R¹⁻³, and each R¹⁻⁴, the "C₆-C₁₄ aryl" in the C₆-C₁₄ aryl and the C₆-C₁₄ aryl substituted by one or more R¹⁻¹⁻¹ is phenyl.

In some embodiments, in each R¹⁻¹, each R¹⁻², each R¹⁻³, and each R¹⁻⁴, the C₁-C₆ alkoxy is independently methoxy or ethoxy.

In some embodiments, in each R¹⁻¹, each R¹⁻², each R¹⁻³, and each R¹⁻⁴, the 5- to 10-membered heteroaryl is independently 5- to 6-membered heteroaryl containing one S atom or 5- to 6-membered heteroaryl containing one N atom, further optionally thiophenyl, pyridinyl, or benzothiophenyl.

In some embodiments, in each R¹⁻¹⁻¹, the halogen is fluorine, chlorine, or bromine.

In some embodiments, in each R¹⁻¹⁻¹, the C₁-C₆ alkyl is methyl or ethyl.

In some embodiments, in each R¹⁻¹⁻¹, the C₆-C₁₄ aryl is phenyl.

In some embodiments, in R⁵, the halogen is fluorine.

In some embodiments, A is

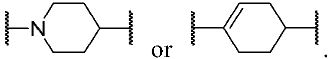

In some embodiments, R³ and R⁴ are independently H, -OCH₃, F, Cl, -CH₃, -CF₃, -OCF₃, or

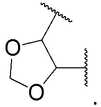

In some embodiments, R¹ is methyl, ethyl, propyl, n-butyl,

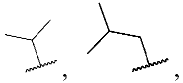

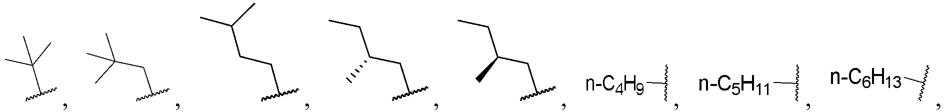

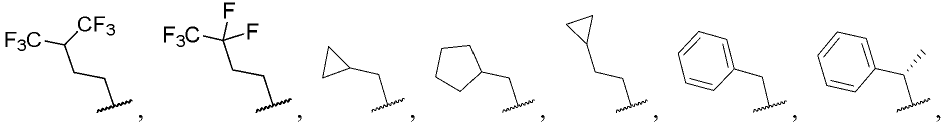

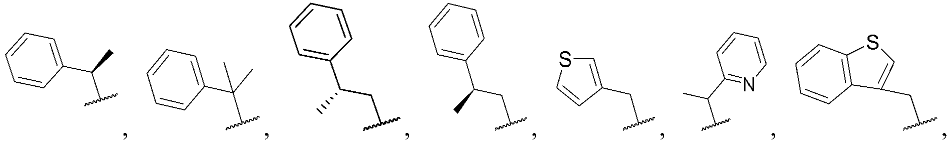

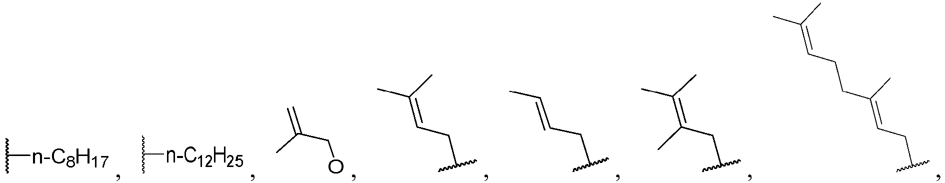

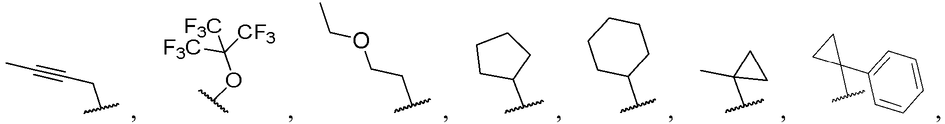

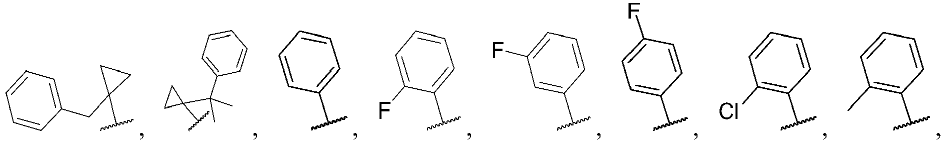

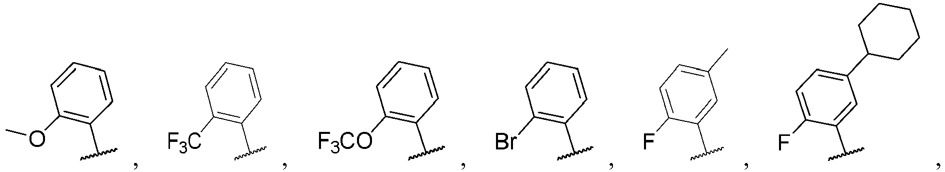

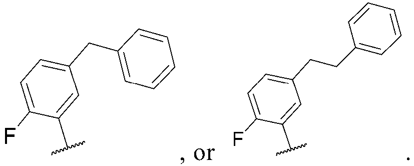

In some embodiments, R⁵ is H.

In some embodiments, the following conditions are satisfied:
(1) A is 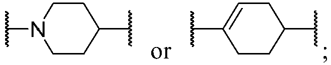
(2) R³ and R⁴ are independently H, -OCH₃, F, Cl, -CH₃, -CF₃, -OCF₃, or 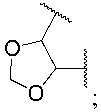
(3) R¹ is methyl, ethyl, propyl, n-butyl, 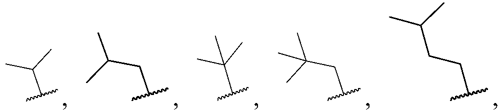 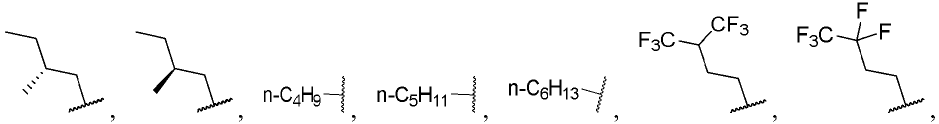 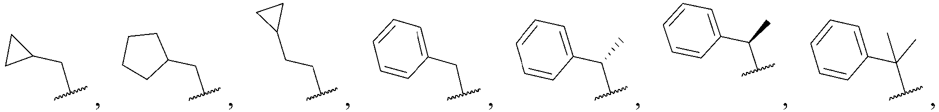 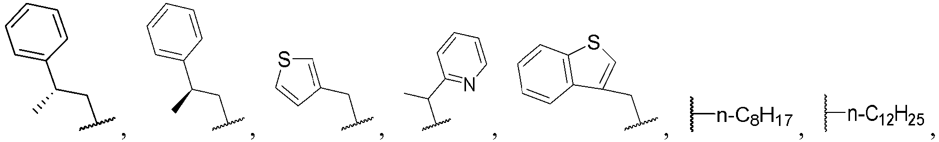 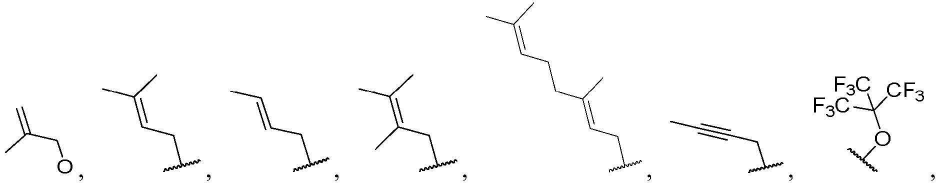 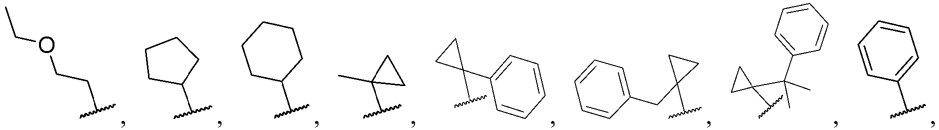 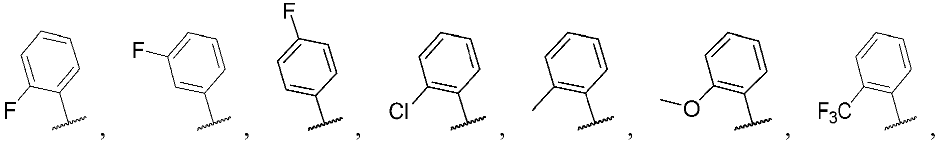 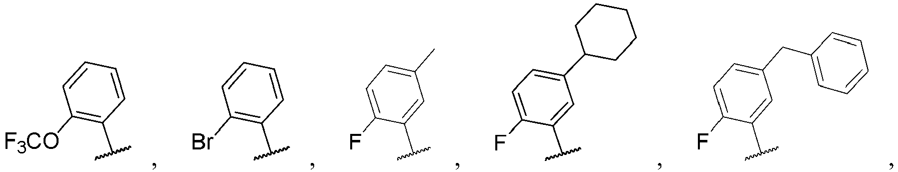 or 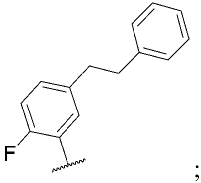 and
(4) R⁵ is H.

In some embodiments, R¹ is C₃-C₆ alkyl, C₁-C₆ alkyl substituted by one or more R¹⁻¹, C₃-C₁₀ cycloalkyl, C₃-C₁₀ cycloalkyl substituted by one or more R¹⁻², phenyl, or phenyl substituted by one or more R¹⁻³.

In some embodiments, each R¹⁻¹ is independently fluorine or phenyl.

In some embodiments, each R¹⁻² is independently C₁-C₃ alkyl or C₁-C₃ alkyl substituted by one or more R¹⁻¹⁻¹.

In some embodiments, each R¹⁻³ is independently fluorine, C₁-C₃ alkyl, or C₁-C₃ alkyl substituted by one or more R¹⁻¹⁻¹.

In some embodiments, each R¹⁻¹⁻¹ is independently phenyl.

In some embodiments, R³ is H, methoxy, or C₁-C₆ alkyl substituted by one or more R³⁻⁵

In some embodiments, R⁴ is methoxy or C₁-C₆ alkyl substituted by one or more R³⁻⁵.

In some embodiments, each R³⁻⁵ is fluorine.

In some embodiments, the following conditions are satisfied:
(1) R¹ is C₃-C₆ alkyl, C₁-C₆ alkyl substituted by one or more R¹⁻¹, C₃-C₁₀ cycloalkyl, C₃-C₁₀ cycloalkyl substituted by one or more R¹⁻², phenyl, or phenyl substituted by one or more R¹⁻³;
(2) each R¹⁻¹ is independently fluorine or phenyl;
(3) each R¹⁻² is independently C₁-C₃ alkyl or C₁-C₃ alkyl substituted by one or more R¹⁻¹⁻¹;
(4) each R¹⁻³ is independently fluorine, C₁-C₃ alkyl, or C₁-C₃ alkyl substituted by one or more R¹⁻¹⁻¹;
(5) each R¹⁻¹⁻¹ is independently phenyl;
(6) R³ is H, methoxy, or C₁-C₆ alkyl substituted by one or more R³⁻⁵;
(7) R⁴ is methoxy or C₁-C₆ alkyl substituted by one or more R³⁻⁵; and
(8) each R³⁻⁵ is fluorine.

In some embodiments, the compound of formula I is any one of the following compounds:

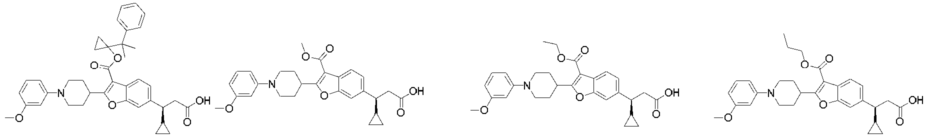

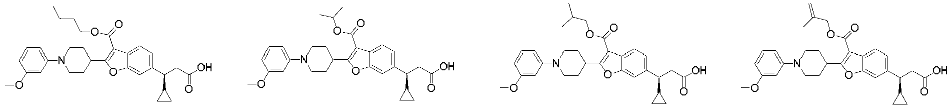

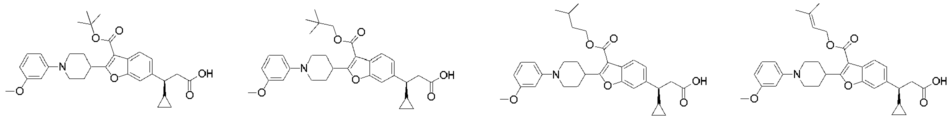

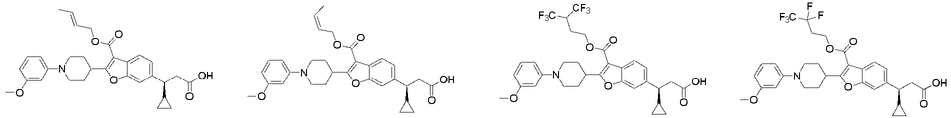

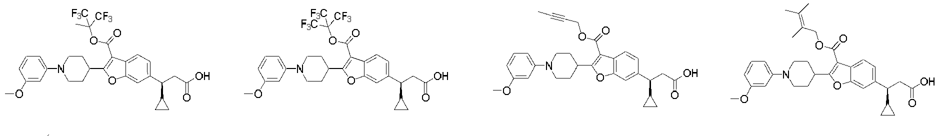

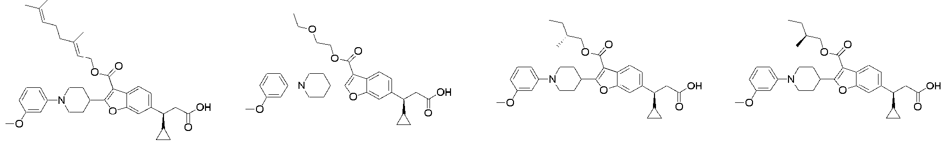

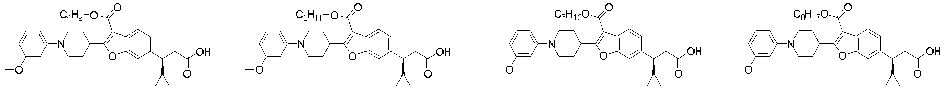

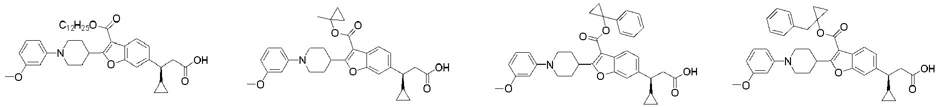

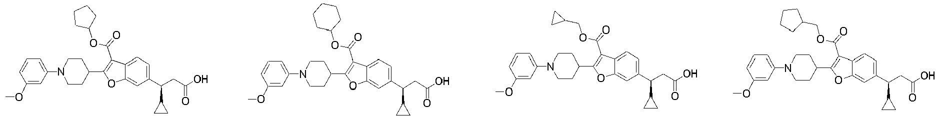

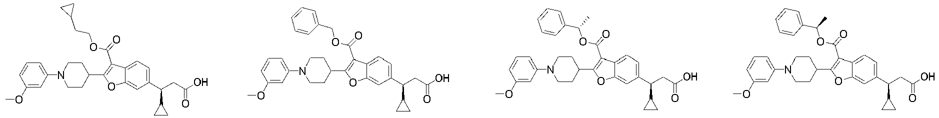

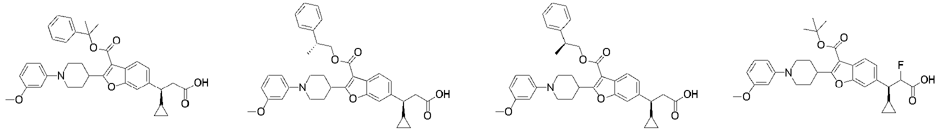

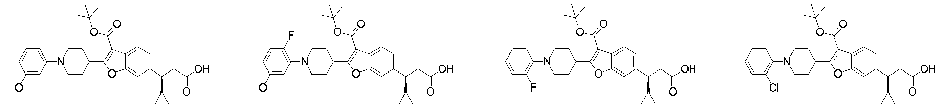

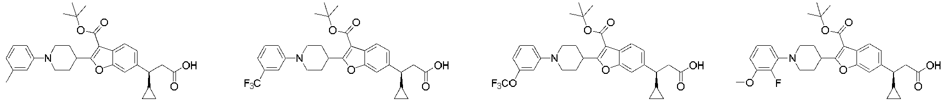

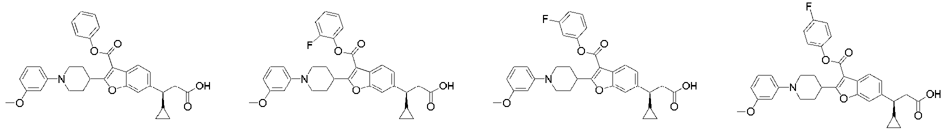

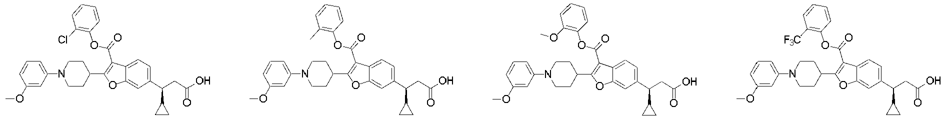

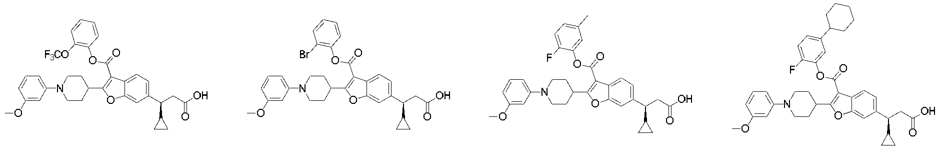

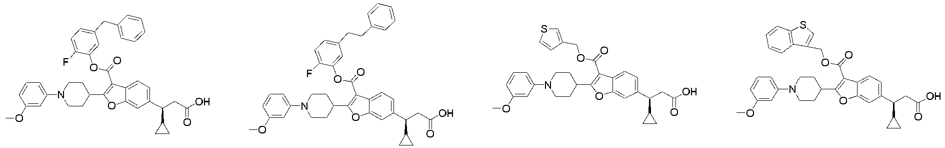

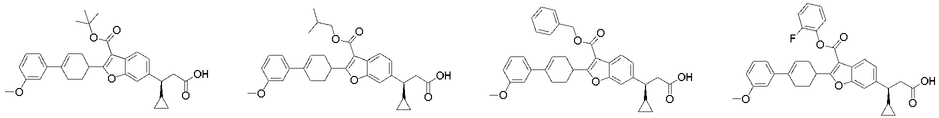

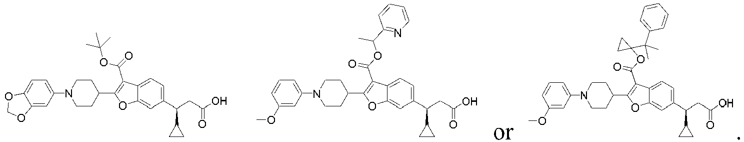

In another aspect, the present invention also provides a pharmaceutical composition comprising the compound of formula I, the pharmaceutically acceptable salt thereof, the solvate thereof, or the solvate of the pharmaceutically acceptable salt thereof, and a pharmaceutically acceptable excipient.

In another aspect, the present invention also provides a use of the compound of formula I or the pharmaceutically acceptable salt thereof in the manufacture of a GPR40 agonist.

In another aspect, the present invention also provides a use of the compound of formula I or the pharmaceutically acceptable salt thereof in the manufacture of a medicament for treating or preventing a GPR40-related disease;
in the use, the GPR40-related disease is, for example, diabetes.

The present invention also provides a use of the compound of formula I or the pharmaceutically acceptable salt thereof in the manufacture of a medicament for treating or preventing diabetes.

The present invention also provides a method of treating a GPR40-related disease (preferably diabetes), comprising administering to a patient an effective amount of the compound of formula I or the pharmaceutically acceptable salt thereof.

The present invention also provides a method of treating diabetes, comprising administering to a patient an effective amount of the compound of formula I or the pharmaceutically acceptable salt thereof.

The term more than one in the expression "group B substituted by one or more group A" refers to 2, 3, 4, or 5. The term "group B substituted by one or more group A" means that 1, 2, 3, 4, or 5 hydrogen atoms in group B are independently substituted by group A. When more than one group A appears simultaneously, unless otherwise specified, their definitions are independent of and do not affect each other. For example, "C₆-C₁₀ aryl substituted by 3 halogens" refers to the C₆-C₁₀ aryl substituted by 3 halogens, where the definitions of the 3 halogens are independent of and do not affect each other.

The term "pharmaceutically acceptable salt" refers to a salt obtained by reacting a compound with a pharmaceutically acceptable acid or base. When the compound contains a relatively acidic functional group, a base addition salt can be obtained by contacting the compound with a sufficient amount of a pharmaceutically acceptable base in a suitable inert solvent. When the compound contains a relatively basic functional group, an acid addition salt can be obtained by contacting the compound with a sufficient amount of a pharmaceutically acceptable acid in a suitable inert solvent. For details, please refer to the "Handbook of Pharmaceutical Salts: Properties, Selection, and Use" (P. Heinrich Stahl, Camille G. Wermuth, 2011, 2nd Revised Edition).

The " 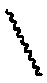 " in a structural moiety means that the structural moiety is connected to other moieties in the molecule through this site. For example, 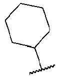 refers to cyclohexyl.

The "-" at the terminus of a group indicates that the group is connected to the rest of the molecule through this site. For example, -C₅H₁₁ refers to n-pentane.

The term "halogen" refers to fluorine, chlorine, bromine, or iodine.

The term "alkyl" refers to a straight or branched, saturated monovalent hydrocarbon group with a specified number of carbon atoms (*e.g.,* C₁-C₆). The alkyl includes, but is not limited to, methyl, ethyl, *n*-propyl, isopropyl, *n*-butyl, isobutyl, *sec-*butyl, *tert*-butyl, *n*-pentyl, *n*-hexyl, *etc.*

The term "heterocycloalkyl" refers to a cyclic group with a specified number of ring atoms (*e.g.,* 5- to 10-membered), a specified number of heteroatoms (*e.g.,* 1, 2, or 3), and a specified type of heteroatoms (one or more types of N, O, and S). The heterocycloalkyl includes, but is not limited to, dioxolanyl, piperidinyl, *etc.*

The term "alkoxy" refers to the group R^{X}-O-, where the definition of R^{X} is the same as that in the term "alkyl". The alkoxy includes, but is not limited to: methoxy, ethoxy, *n*-propoxy, isopropoxy, *etc.*

The term "alkylene" refers to a divalent group connected to the rest of the molecule via two single bonds, with the remaining definition being the same as the term "alkyl".

The term "cycloalkyl" refers to a cyclic, saturated monovalent hydrocarbon group with a specified number of carbon atoms (*e.g.,* C₃-C₈), which is monocyclic. The cycloalkyl includes, but is not limited to: 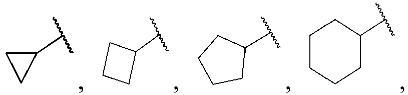 *etc.*

The term "aryl" refers to a cyclic, unsaturated monovalent hydrocarbon group with a specified number of carbon atoms (*e.g.,* C₆-C₁₀), which is a monocyclic ring or a polycyclic ring (*e.g.,* 2 or 3 rings). When the aryl is a polycyclic ring, the monocyclic rings share two atoms and one bond, and each ring is aromatic. The aryl includes, but is not limited to, phenyl, naphthyl, *etc.*

The term "heteroaryl" refers to a cyclic, unsaturated monovalent group with a specified number of ring atoms (e*.g.,* 5- to 10-membered), a specified number of heteroatoms (*e.g*., 1, 2, or 3), and a specified type of heteroatoms (one or more types of P, N, O, and S), which is a monocyclic ring or a polycyclic ring *(e.g.,* 2 or 3 rings), where the monocyclic rings share two atoms and one bond, and each ring is aromatic. The heteroaryl is connected to the rest of the molecule via a carbon atom or a heteroatom; the heteroaryl is connected to the rest of the molecule through a ring with heteroatoms or a ring without heteroatoms.

The term "pharmaceutically acceptable excipient" refers to all substances contained in a pharmaceutical preparation other than the active pharmaceutical ingredient, generally classified into two categories: vehicles and additives. For details, please refer to the "Pharmacopoeia of the People's Republic of China (2020 Edition)" and the "Handbook of Pharmaceutical Excipients" (Paul J Sheskey, Bruno C Hancock, Gary P Moss, David J Goldfarb, 2020, 9th Edition).

On the basis of not violating the common sense in the field, the preferred conditions above can be arbitrarily combined to obtain the preferred examples of the present invention.

The reagents and raw materials used in the present invention are commercially available.

The positive and progressive effects of the present invention are that the compounds of the present invention exhibit excellent GPR40 agonist activity and have good prospects for drug development.

### DETAILED DESCRIPTION OF THE PREFERRED EMBODIMENT

The present invention is further described below by the way of examples, but the present invention is not thereby limited to the scope of the described examples. Experimental methods without specific conditions in the following examples are selected according to conventional methods and conditions, or according to the commercial specification.

### Synthesis of starting material 1-1:

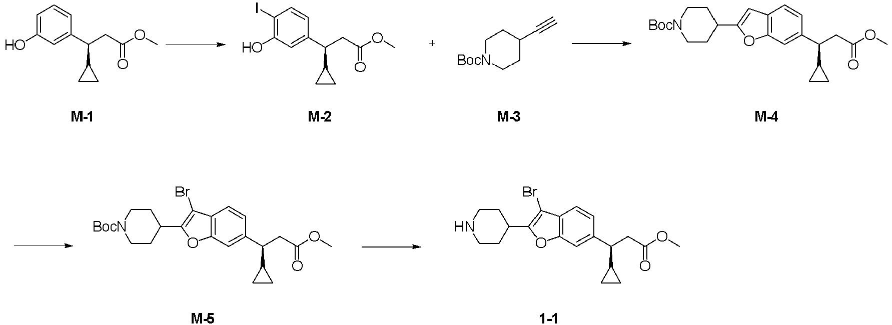

According to the above route, a mixture of commercially available raw material compound **M-1** (*S*)-methyl 3-cyclopropyl-3-(3-hydroxyphenyl)propanoate (10 g, 45.5 mmol, CAS No.: 1142223-08-2), *N*-iodosuccinimide (9.21 g, 41.0 mmol), and dichloromethane (200 mL) was stirred at room temperature for 1 hour. The reaction mixture was concentrated directly, and the resulting crude product was purified by normal-phase column chromatography (dichloromethane: petroleum ether = 50%) to obtain compound **M-2** (9.10 g, yield: 58%) as a yellow oil. LCMS: [M+H]⁺ = 346.8.

A mixture of compound **M-2** (1.35 g, 3.9 mmol), compound **M-3** (815 mg, 3.9 mmol), triethylamine (788 mg, 7.8 mmol), copper(I) iodide (74 mg, 0.39 mmol), bis(triphenylphosphine)palladium(II) chloride (137 mg, 0.195 mmol), and acetonitrile (27 mL) was reacted at 80°C under a nitrogen atmosphere for 4 hours. The reaction mixture was concentrated directly, and the resulting crude product was purified by normal-phase column chromatography (ethyl acetate: petroleum ether = 10%) to obtain compound **M-4** (1.4 g, yield: 84%) as a yellow oil. LCMS: [M-55]⁺ = 372.1.

A mixture of compound **M-4** (1.1 g, 2.58 mmol), N-bromosuccinimide (918 mg, 5.16 mmol), and tetrahydrofuran (20 mL) was stirred at room temperature for 3 hours. The reaction mixture was concentrated directly, and the resulting crude product was purified by normal-phase column chromatography (ethyl acetate: petroleum ether = 10%) to obtain compound **M-5** (982 mg, yield: 75%) as a yellow oil. LCMS: [M-55]⁺ = 449.9.

A mixture of compound **M-5** (760 mg, 1.50 mmol), trifluoroacetic acid (513 mg, 4.50 mmol), and dichloromethane (10 mL) was stirred at room temperature for 2 hours. After the reaction was completed, saturated sodium bicarbonate solution (10 mL) was added, followed by extraction with dichloromethane (20 mL × 3). The organic phase was concentrated, and the resulting crude product was purified by normal-phase column chromatography (ethyl acetate: petroleum ether = 50%) to obtain compound **1-1** (565 mg, yield: 93%) as a yellow oil. LCMS: [M+H]⁺ = 406.2.

### Example 1

Synthesis of compound 1, the synthetic route is as follows:

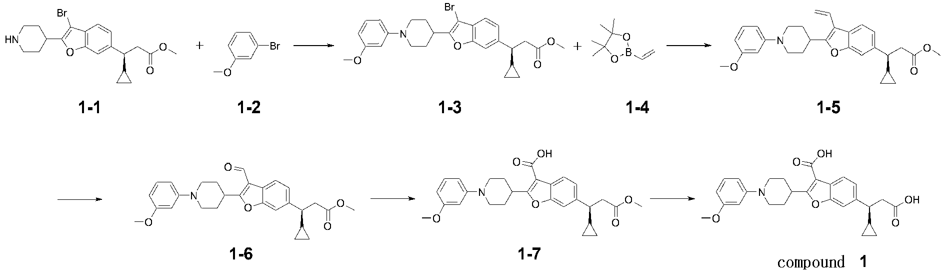

Compound **1-1** (5.65 g, 13.9 mmol), 2,2'-bis(diphenylphosphino)-1,1'-binaphthyl (1.21 g, 1.9 mmol), palladium acetate (400 mg, 1.9 mmol), compound **1-2** (4.28 g, 20.8 mmol), and cesium carbonate (18.12 g, 55.6 mol) were added to toluene (100 mL) and stirred at 100°C overnight. LCMS monitoring confirmed the complete consumption of the starting material. After the reaction was stopped, dichloromethane (200 mL) and water (200 mL) were added to the reaction mixture. The reaction mixture was extracted, and the organic phase was concentrated. The resulting crude product was purified by normal-phase column chromatography (ethyl acetate: petroleum ether = 50%) to obtain compound **1-3** (949 mg, yield: 13%) as a white solid. LCMS: [M+H]⁺ = 526.1.

Compound **1-3** (525 mg, 1.0 mmol), compound **1-4** (385 mg, 2.5 mmol), [1,1'-bis(diphenylphosphino)ferrocene]palladium(II) chloride dichloromethane complex (40 mg, 0.5 mmol), and cesium carbonate (975 mg, 3 mmol) were added to a mixture of 1,4-dioxane: water = 5:1 (25 mL). The reaction mixture was stirred at 80°C for 16 hours. LCMS monitoring confirmed the complete consumption of the starting material. The reaction mixture was extracted with ethyl acetate (50 mL) and concentrated. The resulting crude product was purified by normal-phase column chromatography (ethyl acetate: petroleum ether = 20%) to obtain compound **1-5** (390 mg, yield: 97%) as a yellow oil. LCMS: [M+H]⁺ = 460.2.

Compound **1-5** (390 mg, 0.85 mmol) and 2,6-dimethylpyridine (182 mg, 1.7 mmol) were added to a mixture of 1,4-dioxane: water = 3:1 (40 mL). Potassium osmate dihydrate (15.5 mg, 0.0425 mmol) and sodium periodate (910 mg, 4.25 mmol) were added at 0°C, and the mixture was stirred at 0°C for 4 hours. LCMS monitoring confirmed the complete consumption of the starting material. Saturated sodium thiosulfate aqueous solution (25 mL) was added at 0°C, and the reaction mixture was extracted with ethyl acetate (50 mL) and concentrated. The resulting crude product was purified by normal-phase column chromatography (ethyl acetate: petroleum ether = 20%) to obtain compound **1-6** (195 mg, yield: 49%) as a yellow oil. LCMS: [M+H]⁺ = 462.2.

Compound **1-6** (195 mg, 0.425 mmol) and 2-methyl-2-butene (600 mg, 8.5 mmol) were added to a mixture of tetrahydrofuran: *tert*-butanol = 1:1 (20 mL). An aqueous solution (10 mL) containing sodium dihydrogen phosphate (700 mg, 5.1 mmol) and sodium chlorite (192 mg, 2.15 mmol) was added dropwise at 25°C. The mixture was stirred at 25°C for 4 hours. LCMS monitoring confirmed the complete consumption of the starting material. The reaction mixture was extracted with ethyl acetate (30 mL) and concentrated. The resulting crude product was purified by normal-phase column chromatography (ethyl acetate: petroleum ether = 50%) to obtain compound **1-7** (128 mg, yield: 63%) as a yellow oil. LCMS: [M+H]⁺ = 478.2.

Compound **1-7** (56 mg, 0.117 mmol), lithium hydroxide (15 mg, 0.587 mmol), tetrahydrofuran (5 mL), methanol (5 mL), and water (5 mL) were reacted at 50°C for 2 hours. LCMS monitoring confirmed the complete consumption of the starting material. The reaction mixture was extracted with ethyl acetate (30 mL) and concentrated. The resulting crude product was purified by reverse-phase column chromatography [acetonitrile/water (0.05% formic acid) = 60%] to obtain compound **1** (40 mg, yield: 71%) as a white solid. LCMS: [M+H]⁺ = 464.0.

¹H NMR (400 MHz, CDCl₃) δ 7.90 (d, *J* = 8.0 Hz, 1H), 7.39 (s, 1H), 7.23 - 7.15 (m, 2H), 6.74 - 6.40 (m, 3H), 3.84 - 3.79 (m, 6H), 2.96 - 2.83 (m, 4H), 2.51 - 2.45 (m, 1H), 2.26 - 2.07 (m, 2H), 2.02 - 1.94 (m, 2H), 1.13 - 1.06 (m, 1H), 0.70 - 0.61 (m, 1H), 0.52 - 0.43 (m, 1H), 0.35 - 0.31 (m, 1H), 0.26 - 0.20 (m, 1H).

### Example 2

Synthesis of compound 2, the synthetic route is as follows:

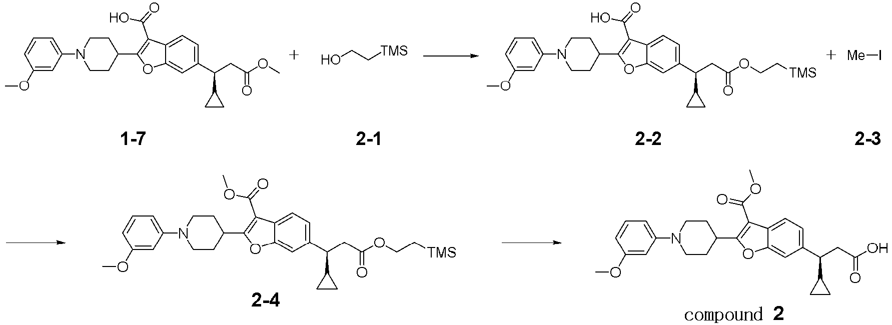

Compound **1-7** (200 mg, 0.420 mmol) was dissolved in toluene (5 mL). Sodium hydride (50 mg, 1.66 mmol) was added at 0°C, and the mixture was stirred at room temperature for 1 hour. Compound **2-1** (346 mg, 2.94 mmol) was added, and the reaction was carried out at 50°C for 1 hour. The reaction was quenched with 1 N hydrochloric acid aqueous solution (30 mL) and extracted with ethyl acetate (30 mL × 3). The organic phase was dried over anhydrous sodium sulfate, filtered, and concentrated. The resulting crude product was purified by normal-phase column chromatography (ethyl acetate: petroleum ether = 50%) to obtain **2-2** (200 mg, yield: 84%) as a white solid. LCMS: [M+H]⁺ = 564.1.

Compound **2-2** (50 mg, 0.105 mmol) was dissolved in *N*,*N*-dimethylformamide (3 mL), then compound **2-3** (50.4 mg, 0.355 mmol) and potassium carbonate (25 mg, 0.176 mmol) were added. The reaction was carried out at 70°C overnight. Water (10 mL) was added to dilute the mixture, followed by extraction with ethyl acetate (10 mL × 3). The organic phase was dried over anhydrous sodium sulfate, filtered, and concentrated. The resulting crude product was purified by normal-phase column chromatography (ethyl acetate: petroleum ether = 15%) to obtain compound **2-4** (33.3 mg, yield: 55%) as a white solid. LCMS: [M+H]⁺ = 578.3.

Compound **2-4** (28.9 mg, 0.05 mmol), tetrahydrofuran (3 mL), and *N,N-*dimethylformamide (1 mL) were added to a reaction tube. Tetrabutylammonium fluoride (0.07 mL, 1 M) was added, and the reaction was carried out at room temperature for 1 hour. The mixture was rotary evaporated to dryness to remove the solvent, and the resulting crude product was purified by reverse-phase column chromatography [acetonitrile/water (0.05% formic acid) = 70%] to obtain compound **2** (11.2 mg, yield: 47%) as a white solid. LCMS: [M+H]⁺ = 478.1.

¹H NMR (400 MHz, CDCl₃) δ 7.90 (d, *J* = 8.0 Hz, 1H), 7.38 (s, 1H), 7.26 - 7.20 (m, 2H), 6.72 - 6.65 (m, 1H), 6.65 - 6.55 (m, 1H), 6.53 - 6.47 (m, 1H), 3.98 (s, 3H), 3.90 - 3.81 (m, 6H), 3.01 - 2.83 (m, 4H), 2.55 - 2.49 (m, 1H), 2.29 - 2.17 (m, 2H), 2.07 - 1.98 (m, 2H), 1.18 - 1.07 (m, 1H), 0.71 - 0.60 (m, 1H), 0.50 - 0.42 (m, 1H), 0.42 - 0.31 (m, 1H), 0.24 - 0.15 (m, 1H).

### Example 3

Synthesis of compound 3, the synthetic route is as follows:

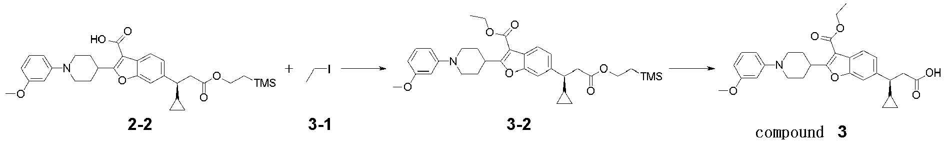

Referring to the synthesis of compound **2,** compound **3** (4.0 mg, yield: 9%) was obtained as a white solid. LCMS: [M+H]⁺ = 492.1.

¹H NMR (400 MHz, CDCl₃) δ 7.91 (d, *J* = 8.0 Hz, 1H), 7.40 (s, 1H), 7.28 - 7.22 (m, 2H), 6.93 - 6.42 (m, 3H), 4.45 (q, *J =* 7.2 Hz, 2H), 3.94 - 3.82 (m, 6H), 3.06 - 2.80 (m, 4H), 2.55 - 2.49 (m, 1H), 2.25 - 2.01 (m, 4H), 1.48 (t, *J =* 7.2 Hz, 3H), 1.15 - 1.08 (m, 1H), 0.68 - 0.62 (m, 1H), 0.51 - 0.45 (m, 1H), 0.39 - 0.34 (m, 1H), 0.26 - 0.18 (m, 1H).

### Example 4

Synthesis of compound 4, the synthetic route is as follows:

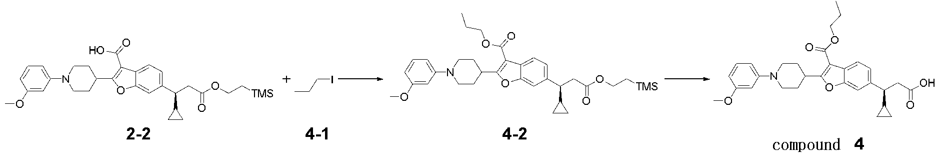

Referring to the synthesis of compound **2,** compound **4** (11.1 mg, yield: 12%) was obtained as a white solid. LCMS: [M+H]⁺ = 506.2.

¹H NMR (400 MHz, CDCl₃) δ 7.92 (d, *J* = 8.0 Hz, 1H), 7.37 (s, 1H), 7.26 - 7.17 (m, 2H), 6.64 (dd, *J =* 8.0, 2.4 Hz, 1H), 6.56 (t, *J =* 2.4 Hz, 1H), 6.46 (dd, *J =* 8.0, 2.4 Hz, 1H), 4.35 (t, *J* = 6.4 Hz, 2H), 3.94 - 3.78 (m, 6H), 2.97 - 2.78 (m, 4H), 2.55 - 2.48 (m, 1H), 2.25 - 2.00 (m, 4H), 1.92 - 1.82 (m, 2H), 1.13 - 1.06 (m, 4H), 0.67 - 0.60 (m, 1H), 0.49 - 0.42 (m, 1H), 0.38 - 0.32 (m, 1H), 0.23 - 0.16 (m, 1H).

### Example 5

Synthesis of compound 5, the synthetic route is as follows:

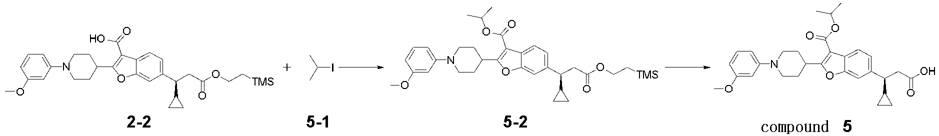

Referring to the synthesis of compound **2,** compound **5** (11.9 mg, yield: 16%) was obtained as a white solid. LCMS: [M+H]⁺ = 506.2.

¹H NMR (400 MHz, CDCl₃) δ 7.90 (d, *J* = 8.0 Hz, 1H), 7.40 (s, 1H), 7.26 - 7.19 (d, *J* = 8.0 Hz, 2H), 6.95 - 6.51 (m, 3H), 5.36 - 5.24 (m, 1H), 3.89 - 3.76 (m, 6H), 3.11 - 2.82 (m, 4H), 2.55 - 2.48 (m, 1H), 2.19 - 2.00 (m, 4H), 1.45 (d, *J* = 6.4 Hz, 6H), 1.12 - 1.04 (m, 1H), 0.70 - 0.60 (m, 1H), 0.51 - 0.44 (m, 1H), 0.39 - 0.33 (m, 1H), 0.24 - 0.17 (m, 1H).

### Example 6

Synthesis of compound 6, the synthetic route is as follows:

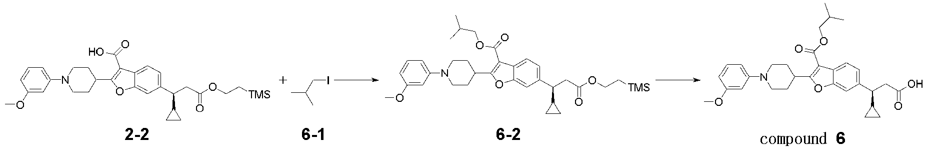

Referring to the synthesis of compound **2,** compound **6** (23.7 mg, yield: 22%) was obtained as a white solid. LCMS: [M+H]⁺ = 520.2.

¹H NMR (400 MHz, CDCl₃) δ 7.94 (d, *J* = 8.0 Hz, 1H), 7.38 (s, 1H), 7.25 - 7.20(m, 2H), 6.65 (dd, *J =* 8.0, 2.4 Hz, 1H), 6.57 (t, *J =* 2.4 Hz, 1H), 6.47 (dd, *J* = 8.0, 2.4 Hz, 1H), 4.19 (d, *J =* 6.4 Hz, 2H), 3.96 - 3.80 (m, 6H), 3.00 - 2.76 (m, 4H), 2.55 - 2.48 (m, 1H), 2.26 - 2.11 (m, 3H), 2.04 - 2.01 (m, 2H), 1.10 (d, *J =* 6.8 Hz, 6H), 1.09 - 1.01 (m, 1H), 0.67 - 0.61 (m, 1H), 0.49 - 0.43 (m, 1H), 0.38 - 0.31 (m, 1H), 0.23 - 0.18 (m, 1H).

### Example 7

Synthesis of compound 7, the synthetic route is as follows:

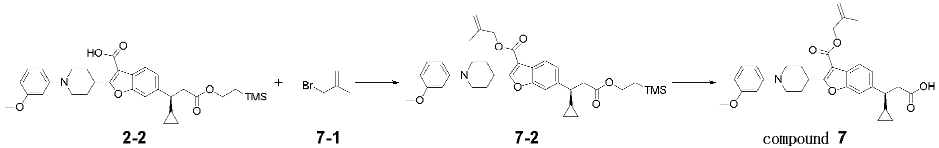

Referring to the synthesis of compound **2,** compound **7** (4.2 mg, yield: 5%) was obtained as a white solid. LCMS: [M+H]⁺ = 518.1.

¹H NMR (400 MHz, CDCl₃) δ 7.94 (d, *J* = 8.0 Hz, 1H), 7.45 (s, 1H), 7.26 - 7.20 (m, 2H), 7.13 (d, *J* = 8.4 Hz, 1H), 6.72 (d, *J* = 2.4 Hz, 1H), 6.64 (dd, *J =* 8.4, 2.4 Hz, 1H), 4.84 (s, 1H), 4.73 (s, 1H), 3.84 - 3.78 (m, 4H), 3.42 (s, 2H), 3.22 (d, *J* = 11.6 Hz, 2H), 2.97 - 2.79 (m, 4H), 2.59 - 2.51 (m, 1H), 2.21 - 2.15 (m, 2H), 1.99 - 1.98 (m, 2H), 1.76 (s, 3H), 1.16 - 1.11 (m, 1H), 0.71 - 0.64 (m, 1H), 0.52 - 0.44 (m, 1H), 0.39 - 0.35 (m, 1H), 0.28 - 0.22 (m, 1H).

### Example 8

Synthesis of compound 8, the synthetic route is as follows:

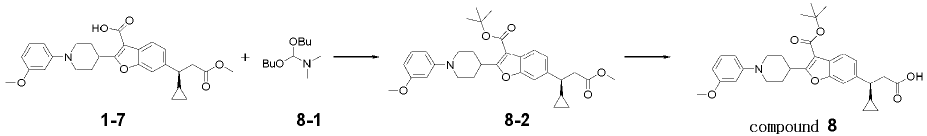

Compound **1-7** (50 mg, 0.105 mmol) was dissolved in toluene (3 mL), and compound **8-1** (212 mg, 1.05 mmol) was added. The reaction was carried out at 80°C for 2 hours. The mixture was rotary evaporated to dryness to remove the solvent, yielding crude compound **8-2,** which was directly used in the next step. LCMS: [M+H]⁺ = 534.0.

Crude compound **8-2** (50 mg, 0.0936 mmol), lithium hydroxide (12 mg, 0.468 mmol), tetrahydrofuran (3 mL), methanol (3 mL), and water (3 mL) were added to a reaction tube. The reaction was carried out at 50°C for 2 hours. After the reaction was completed, the mixture was concentrated directly and purified by reverse-phase column chromatography [acetonitrile/water (0.05% formic acid) = 50% to 70%] to obtain compound **8** (35 mg, yield: 70%) as a white solid. LCMS: [M+H]⁺ = 520.1.

¹H NMR (400 MHz, CDCl₃) δ 7.88 (d, *J =* 8.0 Hz, 1H), 7.34 (s, 1H), 7.22 - 7.15 (m, 2H), 6.72 - 6.36 (m, 3H), 3.84 - 3.81 (m, 6H), 3.02 - 2.75 (m, 4H), 2.52 - 2.44 (m, 1H), 2.27 - 2.06 (m, 2H), 2.05 - 1.98 (m, 2H), 1.66 (s, 9H), 1.11 - 1.06 (m, 1H), 0.64 - 0.58 (m, 1H), 0.46 - 0.40 (m, 1H), 0.35 - 0.29 (m, 1H), 0.20 - 0.16 (m, 1H).

### Example 9

Synthesis of compound 9, the synthetic route is as follows:

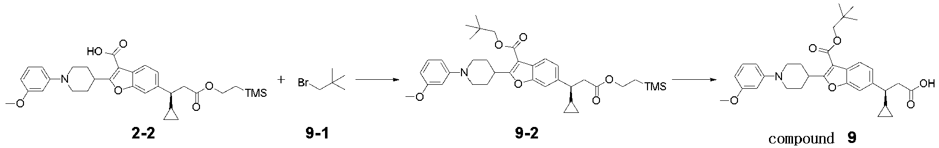

Referring to the synthesis of compound **2,** compound **9** (12.1 mg, yield: 13%) was obtained as a white solid. LCMS: [M+H]⁺ = 534.2.

¹H NMR (400 MHz, CDCl₃) δ 7.94 (d, *J =* 8.0 Hz, 1H), 7.39 - 3.37 (m, 1H), 7.28 - 7.18 (m, 2H), 6.64 (dd, *J =* 8.0, 2.4 Hz, 1H), 6.57 (t, *J =* 2.4 Hz, 1H), 6.46 (dd, *J =* 8.0, 2.4 Hz, 1H), 4.11 (s, 2H), 3.95 -3.84 (m, 6H), 3.01 - 2.78 (m, 4H), 2.54 - 2.48 (m, 1H), 2.28 - 2.12 (m, 2H), 2.05 - 2.02 (m, 2H), 1.20 - 0.95 (m, 10H), 0.68 - 0.61 (m, 1H), 0.50 - 0.43 (m, 1H), 0.39 - 0.33 (m, 1H), 0.23 - 0.17 (m, 1H).

### Example 10

Synthesis of compound 10, the synthetic route is as follows:

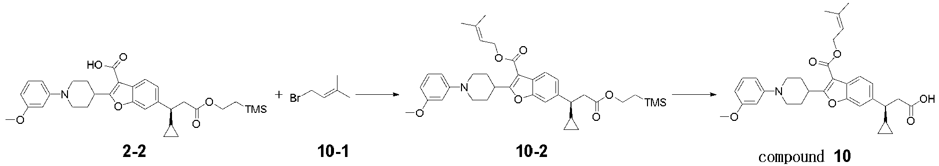

Referring to the synthesis of compound **2,** compound **10** (52 mg, yield: 61%) was obtained as a white solid. LCMS: [M+H]⁺ = 532.1.

¹H NMR (400 MHz, DMSO-*d*₆) δ 7.77 (d, *J =* 8.0 Hz, 1H), 7.55 (s, 1H), 7.26 (d, *J =* 8.0 Hz, 1H), 7.13 (t, *J =* 8.0 Hz, 1H), 6.58 (dd, *J* = 8.0, 2.0 Hz, 1H), 6.50 (t, *J* = 2.0 Hz, 1H), 6.37 (dd, *J =* 8.0, 2.0 Hz, 1H), 5.51 (t, *J =* 7.2 Hz, 1H), 4.82 (d, *J =* 7.2 Hz, 2H), 3.91 - 3.77 (m, 3H), 3.73 (s, 3H), 2.87 - 2.77 (m, 2H), 2.75 - 2.65 (m, 2H), 2.43 - 2.34 (m, 1H), 2.0 - 1.88 (m, 4H), 1.77 (s, 3H), 1.76 (s, 3H), 1.12 - 1.02 (m, 1H), 0.56 - 0.48 (m, 1H), 0.34 - 0.22 (m, 2H), 0.18 - 0.10 (m, 1H).

### Example 11

Synthesis of compound 11, the synthetic route is as follows:

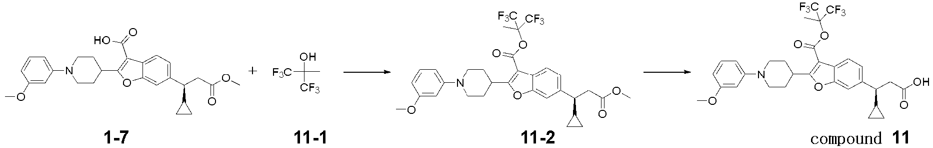

Compound **1-7** (40 mg, 0.083 mmol), compound **11-1** (76 mg, 0.42 mmol), 1-(3-dimethylaminopropyl)-3-ethylcarbodiimide hydrochloride (48 mg, 0.25 mmol), 4-dimethylaminopyridine (15 mg, 0.13 mmol), and dichloromethane (4 mL) were added to a reaction tube under a nitrogen atmosphere. The reaction was stirred at room temperature overnight. After cooling to room temperature, the mixture was diluted with ethyl acetate (30 mL) and washed with saturated brine (30 mL). The organic phase was dried over anhydrous sodium sulfate, filtered, concentrated, and purified by normal-phase column chromatography (ethyl acetate: petroleum ether = 30%) to obtain compound **11-2** (53.7 mg, yield: 99%) as an oil. LCMS: [M+H]⁺ = 642.2.

Compound **11-2** (53.7 mg, 0.083 mmol) and trimethyltin hydroxide (30 mg, 0.17 mmol) in 1,2-dichloroethane (5 mL) were added to a reaction tube. The mixture was heated to 90°C and stirred overnight. After the reaction was completed, dilute hydrochloric acid was added to adjust the pH to 3. The mixture was extracted with ethyl acetate (30 mL), and the organic phase was dried over anhydrous sodium sulfate, filtered, concentrated, and purified by reversed-phase column chromatography [acetonitrile/water (0.05% formic acid) = 50% to 70%] to obtain compound **20** (23 mg, yield: 44%) as a white solid. LCMS: [M+H]⁺ = 628.2.

¹H NMR (400 MHz, CDCl₃) δ 7.87 (d, *J* = 8.0 Hz, 1H), 7.40 (s, 1H), 7.28 - 7.21 (m, 2H), 6.64 (dd, *J =* 8.0 Hz, 2.0 Hz, 1H), 6.56 (t, *J =* 2.0 Hz, 1H), 6.47 (dd, *J =* 8.0 Hz, 2.0 Hz, 1H), 3.89 - 3.72 (m, 6H), 2.98 - 2.81 (m, 4H), 2.54 - 2.48 (m, 1H), 2.25 - 2.14 (m, 5H), 2.05 - 1.97 (m, 2H), 1.15 - 1.06 (m, 1H), 0.69 - 0.62 (m, 1H), 0.50 - 0.44 (m, 1H), 0.39 - 0.33 (m, 1H), 0.23 - 0.17 (m, 1H).

### Example 12

Synthesis of compound 12, the synthetic route is as follows:

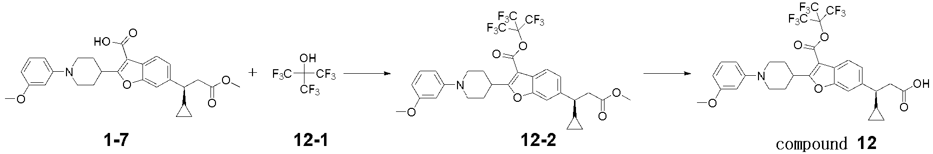

Referring to the synthesis of compound **11,** compound **12** (16.5 mg, yield: 14%) was obtained as a white solid. LCMS: [M+H]⁺ = 682.2.

¹H NMR (400 MHz, CDCl₃) δ 7.83 (d, *J* = 8.0 Hz, 1H), 7.42 (s, 1H), 7.31 - 7.27 (m, 1H), 7.23 (t, *J =* 8.0 Hz, 1H), 6.63 (dd, *J =* 8.0 Hz, 2.0 Hz, 1H), 6.56 (t, *J =* 2.0 Hz, 1H), 6.47 (dd, *J =* 8.0 Hz, 2.0 Hz, 1H), 3.90 - 3.72 (m, 6H), 2.98 - 2.81 (m, 4H), 2.54 - 2.47 (m, 1H), 2.24 - 2.15 (m, 2H), 2.05 - 2.00 (m, 2H), 1.15 - 1.05 (m, 1H), 0.69 - 0.62 (m, 1H), 0.50 - 0.44 (m, 1H), 0.39 - 0.33 (m, 1H), 0.23 - 0.17 (m, 1H).

### Example 13

Synthesis of compound 13, the synthetic route is as follows:

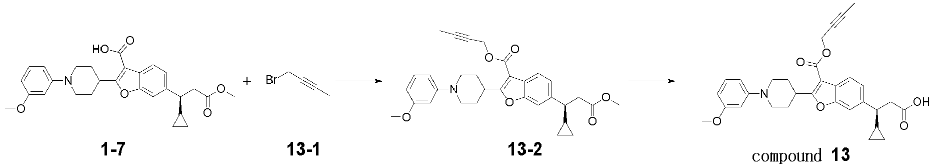

Compound **1-7** (105 mg, 0.22 mmol), compound **13-1** (35 mg, 0.26 mmol), anhydrous potassium carbonate (36 mg, 0.26 mmol), and DMF (2 mL) were added to a reaction tube under a nitrogen atmosphere. The reaction was stirred at room temperature overnight. After cooling to room temperature, the mixture was diluted with ethyl acetate (30 mL) and washed with saturated brine (30 mL). The organic phase was dried over anhydrous sodium sulfate, filtered, concentrated, and purified by normal-phase column chromatography (ethyl acetate: petroleum ether = 1:2) to obtain compound **13-2** (98 mg, 84%) as an oil. LCMS: [M+H]⁺ = 530.1.

Compound **13-2** (98 mg, 0.185 mmol) and THF (2 mL) were added to a reaction tube. Then, 0.4 mL of LiOH aqueous solution (9 mg, 0.37 mmol) was added dropwise to the reaction, and the mixture was stirred at room temperature overnight. The pH was adjusted to 3 with dilute hydrochloric acid, and the mixture was extracted with ethyl acetate (20 mL). The organic phase was dried over anhydrous sodium sulfate, filtered, and concentrated. The crude product was purified by reverse-phase column chromatography [acetonitrile/water (0.05% formic acid) = 50% to 70%] to obtain compound **13** (17.5 mg, 19%) as a white solid. LCMS: [M+H]⁺ = 516.1.

¹H NMR (400 MHz, CDCl₃) δ 7.92 (d, *J* = 8.0 Hz, 1H), 7.34 (s, 1H), 7.24 - 7.15 (m, 2H), 6.60 (dd, *J* = 8.0, 2.4 Hz, 1H), 6.53 (t, *J* = 2.4 Hz, 1H), 6.43 (dd, *J* = 8.0, 2.4 Hz, 1H), 4.93 (s, 2H), 3.92 - 3.77 (m, 6H), 2.97 - 2.74 (m, 4H), 2.53 - 2.46 (m, 1H), 2.21 - 2.08 (m, 2H), 2.05 - 1.96 (m, 2H), 1.89 (s, 3H), 1.12 - 1.05 (m, 1H), 0.49 - 0.42 (m, 2H), 0.36 - 0.27 (m, 1H), 0.20 - 0.14 (m, 1H).

### Example 14

Synthesis of compound 14, the synthetic route is as follows:

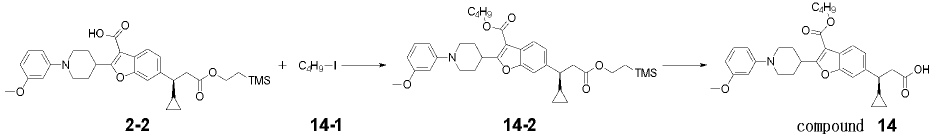

Referring to the synthesis of compound **2,** compound **14** (49 mg, yield: 50%) was obtained as a white solid. LCMS: [M+H]⁺ = 520.1.

¹H NMR (400 MHz, DMSO-*d*₆) δ 7.85 - 7.72 (m, 1H), 7.55 (s, 1H), 7.27 (d, *J* = 8.0 Hz, 1H), 7.13 (t, *J* = 8.0 Hz, 1H), 6.58 (dd, *J =* 8.4, 2.0 Hz, 1H), 6.51 (t, *J =* 2.0 Hz, 1H), 6.37 (dd, *J =* 8.4, 2.0 Hz, 1H), 4.32 (t, *J =* 6.4 Hz, 2H), 3.91 - 3.75 (m, 3H), 3.73 (s, 3H), 2.88 - 2.76 (m, 2H), 2.73 - 2.66 (m, 2H), 2.42 - 2.35 (m, 1H), 2.03 - 1.87 (m, 4H), 1.79 - 1.70 (m, 2H), 1.52 - 1.42 (m, 2H), 1.10 - 1.02 (m, 1H), 0.96 (t, *J =* 6.4 Hz, 3H), 0.56 - 0.47 (m, 1H), 0.33 - 0.23 (m, 2H), 0.19 - 0.08 (m, 1H).

### Example 15

Synthesis of compound 15, the synthetic route is as follows:

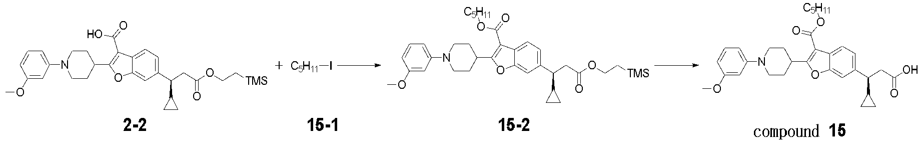

Referring to the synthesis of compound **2,** compound **15** (41 mg, yield: 63%) was obtained as a white solid. LCMS: [M+H]⁺ = 534.1.

¹H NMR (400 MHz, DMSO-*d*₆) δ 7.79 (d, *J =* 7.6 Hz, 1H), 7.55 (s, 1H), 7.26 (d, *J =* 7.6 Hz, 1H), 7.12 (t, *J* = 8.0 Hz, 1H), 6.58 (dd, *J* = 8.0, 2.0 Hz, 1H), 6.50 (t, *J =* 2.0 Hz, 1H), 6.40 - 6.34 (m, 1H), 4.31 (t, *J=* 6.4 Hz, 2H), 3.92 - 3.74 (m, 3H), 3.73 (s, 3H), 2.87 - 2.77 (m, 2H), 2.74 - 2.68 (m, 2H), 2.42 - 2.34 (m, 1H), 2.03 - 1.88 (m, 4H), 1.80 - 1.71 (m, 2H), 1.46 - 1.34 (m, 4H), 1.10 - 1.02 (m, 1H), 0.90 (t, *J =* 6.4 Hz, 3H), 0.55 - 0.47 (m, 1H), 0.33 - 0.23 (m, 2H), 0.18 - 0.10 (m, 1H).

### Example 16

Synthesis of compound 16, the synthetic route is as follows:

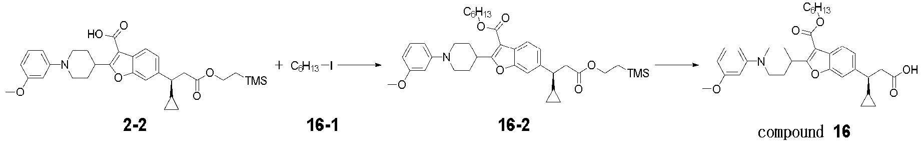

Referring to the synthesis of compound **2,** compound **16** (14 mg, yield: 13%) was obtained as a white solid. LCMS: [M+H]⁺ = 548.1.

¹H NMR (400 MHz, CDCl₃) δ 7.88 (d, *J* = 8.0 Hz, 1H), 7.34 (s, 1H), 7.24 - 7.18 (m, 2H), 6.62 (d, *J =* 7.6 Hz, 1H), 6.55 (s, 1H), 6.44 (d, *J =* 7.6 Hz, 1H), 4.35 (t, *J =* 6.4 Hz, 2H), 3.91 - 3.78 (m, 6H), 2.97 - 2.76 (m, 4H), 2.54 - 2.47 (m, 1H), 2.22 - 2.15 (m, 2H), 2.04 - 1.97 (m, 2H), 1.85 - 1.79 (m, 2H), 1.55 - 1.47 (m, 2H), 1.42 - 1.30 (m, 4H), 1.10 - 1.03 (m, 1H), 0.91 (t, *J* = 6.4 Hz, 3H), 0.65 - 0.57 (m, 1H), 0.47 - 0.40 (m, 1H), 0.35 - 0.28 (m, 1H), 0.21 - 0.14 (m, 1H).

### Example 17

Synthesis of compound 17, the synthetic route is as follows:

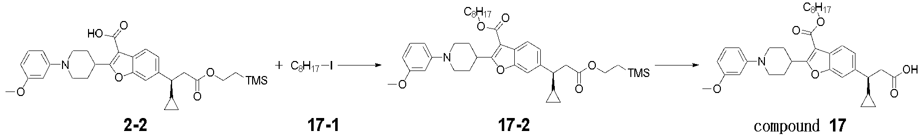

Referring to the synthesis of compound **2,** compound **17** (17 mg, yield: 16%) was obtained as a white solid. LCMS: [M+H]⁺ = 576.1.

¹H NMR (400 MHz, CDCl₃) δ 7.88 (d, *J* = 8.0 Hz, 1H), 7.34 (s, 1H), 7.24 - 7.17 (m, 2H), 6.62 (d, *J =* 7.6 Hz, 1H), 6.54 (s, 1H), 6.44 (d, *J* = 7.6 Hz, 1H), 4.35 (t, *J* = 6.4 Hz, 2H), 3.91 - 3.77 (m, 6H), 2.98 - 2.75 (m, 4H), 2.54 - 2.43 (m, 1H), 2.23 - 2.11 (m, 2H), 2.04 - 1.95 (m, 2H), 1.86 - 1.75 (m, 2H), 1.55 - 1.44 (m, 2H), 1.39 - 1.26 (m, 8H), 1.13 - 1.01 (m, 1H), 0.88 (t, *J =* 6.4 Hz, 3H), 0.65 - 0.57 (m, 1H), 0.47 - 0.39 (m, 1H), 0.35 - 0.28 (m, 1H), 0.21 - 0.13 (m, 1H).

### Example 18

Synthesis of compound 18, the synthetic route is as follows:

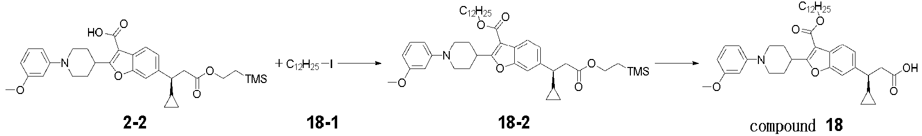

Referring to the synthesis of compound **2,** compound **18** (16 mg, yield: 13%) was obtained as a white solid. LCMS: [M+H]⁺ = 632.1.

¹H NMR (400 MHz, CDCl₃) δ 7.88 (d, *J* = 8.0 Hz, 1H), 7.34 (s, 1H), 7.23 - 7.16 (m, 2H), 6.62 (d, *J* = 8.8 Hz, 1H), 6.54 (s, 1H), 6.44 (d, *J =* 8.8 Hz, 1H), 4.35 (t, *J* = 6.4 Hz, 2H), 3.93 - 3.77 (m, 6H), 2.98 - 2.76 (m, 4H), 2.53 - 2.45 (m, 1H), 2.25 - 2.10 (m, 2H), 2.03 - 1.95 (m, 2H), 1.87 - 1.76 (m, 2H), 1.54 - 1.44 (m, 2H), 1.35 - 1.21 (m, 16H), 1.14 - 1.01 (m, 1H), 0.88 (t, *J =* 6.4 Hz, 3H), 0.65 - 0.57 (m, 1H), 0.48 - 0.39 (m, 1H), 0.35 - 0.28 (m, 1H), 0.22 - 0.14 (m, 1H).

### Example 19

Synthesis of compound 19, the synthetic route is as follows:

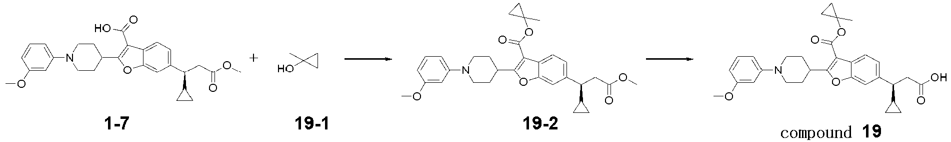

Referring to the synthesis of compound **11,** compound **19** (7.9 mg, yield: 13%) was obtained as a white solid. LCMS: [M+H]⁺ = 518.2.

¹H NMR (400 MHz, CDCl₃) δ 7.87 (d, *J* = 8.0 Hz, 1H), 7.36 (s, 1H), 7.25 - 7.20 (m, 2H), 6.64 (dd, *J =* 8.4 Hz, 2.0 Hz, 1H), 6.56 (t, *J =* 2.0 Hz, 1H), 6.46 (dd, *J =* 8.0 Hz, 2.0 Hz, 1H), 3.91 - 3.81 (m, 6H), 2.99 - 2.79 (m, 4H), 2.55 - 2.47 (m, 1H), 2.22 - 2.11 (m, 2H), 2.04 - 1.99 (m, 2H), 1.72 (s, 3H), 1.14 - 1.04 (m, 3H), 0.84 - 0.78 (m, 2H), 0.68 - 0.61 (m, 1H), 0.49 - 0.42 (m, 1H), 0.38 - 0.32 (m, 1H), 0.22 - 0.16 (m, 1H).

### Example 20

Synthesis of compound 20, the synthetic route is as follows:

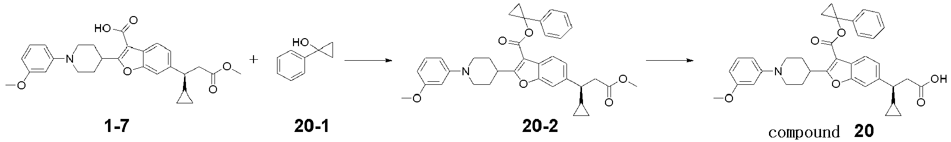

Compounds **1-7** and **20-1** were taken, and referring to the synthesis of compound **11,** compound **20** (17.5 mg, 50%) was obtained as a white solid. LCMS: [M+H]⁺ = 580.1.

¹H NMR (400 MHz, CDCl₃) δ 7.99 (d, *J =* 8.0 Hz, 1H), 7.40 - 7.33 (m, 5H), 7.29 - 7.18 (m, 3H), 6.67 - 6.59 (m, 1H), 6.59 - 6.51 (m, 1H), 6.50 - 6.40 (m, 1H), 3.89 - 3.80 (m, 6H), 2.97 - 2.80 (m, 4H), 2.57 - 2.48 (m, 1H), 2.24 - 2.09 (m, 2H), 1.99 (d, *J =* 12.9 Hz, 2H), 1.53 - 1.49 (m, 2H), 1.45 - 1.41 (m, 2H), 1.18 - 1.07 (m, 1H), 0.72 - 0.59 (m, 1H), 0.53 - 0.43 (m, *J* = 4.8 Hz, 1H), 0.41 - 0.32 (m, 1H), 0.25 - 0.18 (m, 1H).

### Example 21

Synthesis of compound 21, the synthetic route is as follows:

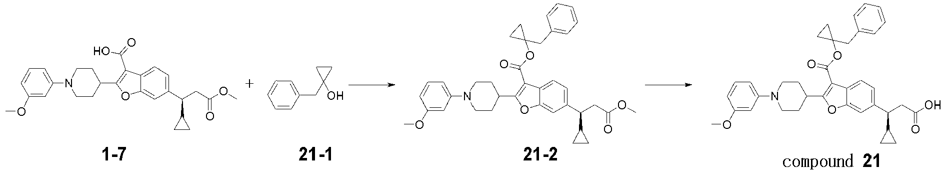

Referring to the synthesis of compound **11,** compound **21** (15.4 mg, yield: 18%) was obtained as a white solid. LCMS: [M+H]⁺ = 594.2.

¹H NMR (400 MHz, CDCl₃) δ 7.75 (d, *J* = 8.0 Hz, 1H), 7.34 (s, 1H), 7.30 - 7.21 (m, 6H), 7.17 (d, *J =* 8.0 Hz, 1H), 6.62 (dd, *J* = 8.0 Hz, 1.6 Hz, 1H), 6.55 (t, *J* = 1.6 Hz, 1H), 6.47 (dd, *J =* 8.0 Hz, 1.6 Hz, 1H), 3.87 - 3.75 (m, 5H), 3.64 - 3.55 (m, 1H), 3.31 (s, 2H), 2.91 - 2.71 (m, 4H), 2.52 - 2.46 (m, 1H), 2.14 - 2.02 (m, 2H), 1.91 - 1.83 (m, 2H), 1.13 - 1.03 (m, 3H), 0.99 - 0.94 (m, 2H), 0.67 - 0.60 (m, 1H), 0.48 - 0.42 (m, 1H), 0.37 - 0.31 (m, 1H), 0.22 - 0.15 (m, 1H).

### Example 22

Synthesis of compound 22, the synthetic route is as follows:

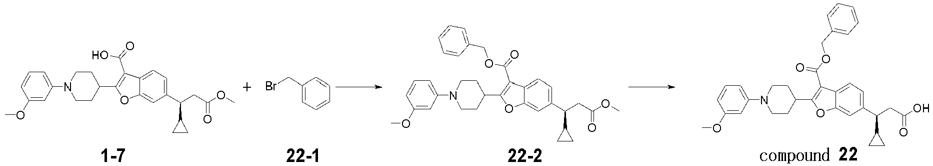

Referring to the synthesis of compound **13,** compound **22** (8.8 mg, yield: 13%) was obtained as a white solid. LCMS: [M+H]⁺ = 554.1.

¹H NMR (400 MHz, CDCl₃) δ 7.90 (d, *J* = 8.0 Hz, 1H), 7.51 (d, *J* = 6.8 Hz, 2H), 7.48 - 7.34 (m, 4H), 7.27 - 7.16 (m, 2H), 6.78 - 6.35 (m, 3H), 5.43 (s, 2H), 3.94 - 3.81 (m, 6H), 2.92 - 2.80 (m, 4H), 2.53 - 2.46 (m, 1H), 2.26 - 2.14 (m, 2H), 2.06 - 1.98 (m, 2H), 1.13 - 1.04 (m, 1H), 0.67 - 0.61 (m, 1H), 0.49 - 0.43 (m, 1H), 0.37 - 0.32 (m, 1H), 0.22 - 0.16 (m, 1H).

### Example 23

Synthesis of compound 23, the synthetic route is as follows:

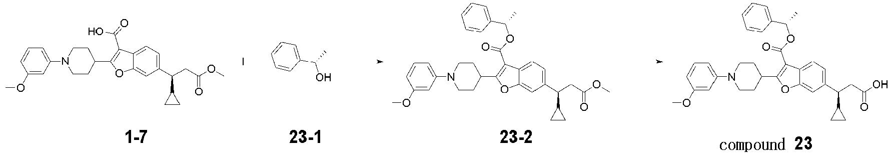

Compound **1-7** (72 mg, 0.151 mmol) was dissolved in dichloromethane (3 mL), and oxalyl chloride (28 mg, 0.226 mmol) was added at 0°C. The reaction was stirred at room temperature for 2 hours. The solvent was then concentrated to dryness, and the residue was dissolved in dichloromethane (2 mL). This solution was added dropwise to compound **23-1** (27.6 mg, 0.226 mmol), and the mixture was stirred at room temperature overnight. After concentration, the crude product was purified by normal-phase column chromatography (ethyl acetate: petroleum ether = 10%) to obtain **23-2** (52.6 mg, yield: 60%) as a white solid. LCMS: [M+H]⁺ = 582.2.

Compound **23-2** (35 mg, 0.06 mmol) and trimethyltin hydroxide (22 mg, 0.12 mmol) were added to 1,2-dichloroethane (5 mL). The mixture was heated to 90°C and stirred overnight. After the reaction was completed, the mixture was filtered and concentrated, then purified by reverse-phase column chromatography [acetonitrile/water (0.05% formic acid) = 50% to 70%] to obtain compound **23** (6.0 mg, yield: 17.5%) as a white solid, LCMS: [M+H]⁺ = 568.2.

¹H NMR (400 MHz, CDCl₃) δ 7.95 (d, *J =* 8.0 Hz, 1H), 7.47 - 7.44 (m, 2H), 7.40 - 7.31 (m, 4H), 7.23 - 7.16 (m, 2H), 6.59 (dd, *J =* 8.0 Hz, 1.6Hz, 1H), 6.53 - 6.50 (m, 1H), 6.42 (dd, *J =* 8.4 Hz, 2.0 Hz, 1H), 6.16 (q, *J =* 6.4 Hz, 1H), 3.87 - 3.75 (m, 6H), 2.90 - 2.81 (m, 4H), 2.51 - 2.45 (m, 1H), 2.17 - 2.09 (m, 2H), 1.98 - 1.95 (m, 2H), 1.72 (d, *J* = 6.4 Hz, 6H), 1.11 - 1.05 (m, 1H), 0.66 - 0.59 (m, 1H), 0.47 - 0.40 (m, 1H), 0.36 - 0.30 (m, 1H), 0.21 - 0.15 (m, 1H).

### Example 24

Synthesis of compound 24, the synthetic route is as follows:

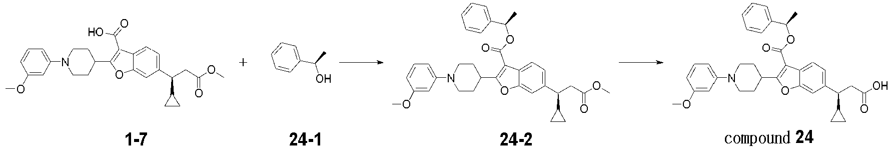

Referring to the synthesis of compound **23,** compound **24** (8.8 mg, yield: 13%) was obtained as a white solid. LCMS: [M+H]⁺ = 568.2.

¹H NMR (400 MHz, CDCl₃) δ 7.98 (d, *J =* 8.0 Hz, 1H), 7.52 - 7.47 (m, 2H), 7.43 - 7.32 (m, 4H), 7.26 - 7.20 (m, 2H), 6.63 (dd, *J* = 8.0 Hz, 2.0 Hz, 1H), 6.55 (t, *J* = 2.0 Hz, 1H), 6.46 (dd, *J =* 8.4 Hz, 2.0 Hz, 1H), 6.20 (q, *J =* 6.4 Hz, 1H), 3.92 - 3.78 (m, 6H), 2.94 - 2.82 (m, 4H), 2.55 - 2.49 (m, 1H), 2.19 - 2.10 (m, 2H), 2.02 - 1.97 (m, 2H), 1.76 (d, *J* = 6.4 Hz, 3H), 1.15 - 1.07 (m, 1H), 0.70 - 0.61 (m, 1H), 0.51 - 0.43 (m, 1H), 0.39 - 0.33 (m, 1H), 0.24 - 0.18 (m, 1H).

### Example 25

Synthesis of compound 25, the synthetic route is as follows:

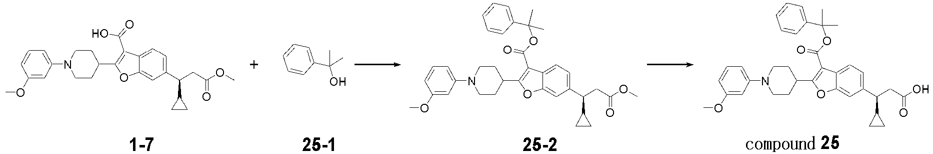

Referring to the synthesis of compound **23,** compound **25** (20.8 mg, yield: 32%) was obtained as a white solid. LCMS: [M+H]⁺ = 582.2.

¹H NMR (400 MHz, CDCl₃) δ 8.00 (d, *J =* 8.0 Hz, 1H), 7.49 - 7.44 (m, 2H), 7.41 - 7.35 (m, 3H), 7.31 - 7.28 (m, 1H), 7.26 - 7.18 (m, 2H), 6.62 (d, *J =* 8.0 Hz, 1H), 6.56 (s, 1H), 6.46 (d, *J =* 8.0 Hz, 1H), 3.87 - 3.76 (m, 6H), 2.94 - 2.80 (m, 4H), 2.55 - 2.49 (m, 1H), 2.24 -2.09 (m, 2H), 2.04 - 1.94 (m, 8H), 1.16 - 1.07 (m, 1H), 0.69 - 0.62 (m, 1H), 0.51 - 0.44 (m, 1H), 0.39 - 0.33 (m, 1H), 0.24 - 0.18 (m, 1H).

### Example 26

Synthesis of compound 26, the synthetic route is as follows:

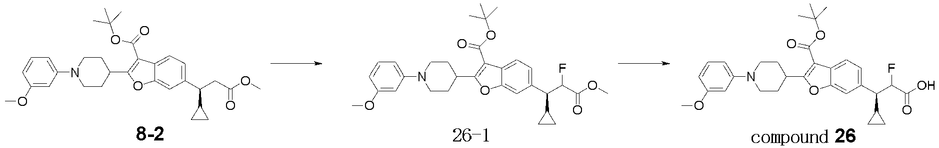

Under a nitrogen atmosphere, compound **8-2** (118 mg, 0.22 mmol) was added to a solution of tetrahydrofuran (5 mL). At -78°C, lithium bis(trimethylsilyl)amide (0.44 mL, 0.44 mmol, 1 mol/L) was slowly added to the reaction mixture and stirred at -78°C for 1 hour. Then, a solution of N-fluorodibenzenesulfonimide (139 mg, 0.44 mmol) in tetrahydrofuran (2 mL) was added to the reaction mixture and stirred for 2 hours. After the reaction was completed, ethyl acetate (50 mL) was added. The organic phase was washed with saturated brine (30 mL) and concentrated to obtain the crude product, which was further purified by normal-phase column chromatography (ethyl acetate: petroleum ether = 10%) to obtain **26-1** (85 mg, yield: 70%) as a yellow oil, MS (ESI, m/z): 552.2 [M+H]⁺.

Compound **26-1** (85 mg, 0.15 mmol) was added to a mixed solution of tetrahydrofuran (4 mL) and methanol (4 mL), followed by the addition of an aqueous solution (1 mL) of lithium hydroxide (43 mg, 1.54 mmol) to the reaction mixture, which was then heated to 60°C and stirred for 2 hours. After the reaction was completed, the mixture was directly concentrated and purified by reverse-phase column chromatography [acetonitrile/water (0.05% formic acid) = 50% to 70%] to obtain compound **26** (10.1 mg, yield: 12%) as a white solid. MS(ESI, m/z): 538.2 [M+H]⁺.

¹H NMR (400 MHz, CDCl₃) δ 7.94 (d, *J* = 8.0 Hz, 1H), 7.53 (s, 1H), 7.32 (d, *J* = 8.8 Hz, 1H), 7.27 (t, *J =* 8.0 Hz, 1H), 6.70 (dd, *J =* 8.4 Hz, 2.0 Hz, 1H), 6.65 (t, *J =* 2.0 Hz, 1H), 6.52 (dd, *J =* 8.4 Hz, 2.0 Hz, 1H), 5.21 (d, *J =* 48.4 Hz, 1H), 3.93 - 3.83 (m, 6H), 3.01 - 2.95 (m, 2H), 2.74 - 2.58 (m, 1H), 2.32 - 2.18 (m, 2H), 2.10 - 2.02 (m, 2H), 1.69 (s, 9H), 1.52 - 1.47 (m, 1H), 0.79 - 0.70 (m, 1H), 0.64 - 0.54 (m, 1H), 0.53 - 0.44 (m, 2H), 0.16 - 0.09 (m, 1H).

### Example 27

Synthesis of compound 27, the synthetic route is as follows:

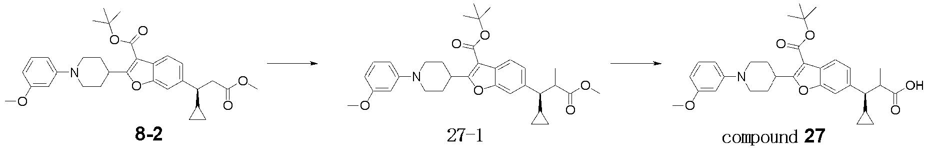

Under a nitrogen atmosphere, compound **8-2** (133 mg, 0.25 mmol) was added to a solution of tetrahydrofuran (5 mL). At -78°C, lithium bis(trimethylsilyl)amide (0.75 mL, 0.75 mmol, 1 mol/L) was slowly added to the reaction mixture and stirred at -78°C for 1 hour. Then, iodomethane (177 mg, 1.24 mmol) was added to the reaction mixture and stirred for 2 hours. After the reaction was completed, ethyl acetate (50 mL) was added. The organic phase was washed with saturated brine (30 mL) and concentrated to obtain the crude product, which was further purified by normal-phase column chromatography (ethyl acetate: petroleum ether = 10%) to obtain **27-1** (110 mg, yield: 81%) as a yellow oil, MS (ESI, m/z): 548.2 [M+H]⁺.

Compound **27-1** (110 mg, 0.2 mmol) was added to a mixed solution of tetrahydrofuran (4 mL) and methanol (4 mL), followed by the addition of an aqueous solution (1 mL) of lithium hydroxide (56 mg, 2.0 mmol) to the reaction mixture, which was then heated to 60°C and stirred for 2 hours. After the reaction was completed, the reaction mixture was directly concentrated and purified by reverse-phase column chromatography [acetonitrile/water (0.05% formic acid) = 50% to 70%] to obtain compound **27** (5.2 mg, yield: 5%) as a white solid. MS (ESI, m/z): 534.2 [M+H]⁺.

¹H NMR (400 MHz, CDCl₃) δ 7.89 (d, *J* = 8.0 Hz, 1H), 7.35 (s, 1H), 7.25 - 7.19 (m, 2H), 6.64 (dd, *J =* 8.0 Hz, 2.0 Hz, 1H), 6.56 (t, *J =* 2.0 Hz, 1H), 6.46 (dd, *J =* 8.0 Hz, 2.0 Hz, 1H), 3.90 - 3.82 (m, 6H), 3.02 - 2.89 (m, 3H), 2.38 - 2.33 (m, 1H), 2.20 - 2.12 (m, 2H), 2.05 - 2.00 (m, 2H), 1.67 (s, 9H), 1.33 (d, *J =* 6.8 Hz, 3H), 1.16 - 1.10 (m, 1H), 0.72 - 0.68 (m, 1H), 0.47 - 0.38 (m, 2H), 0.04 - 0.02 (m, 1H).

### Example 28

Synthesis of compound 28, the synthetic route is as follows:

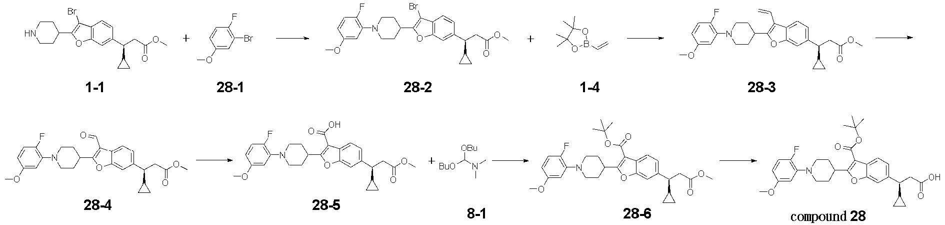

Referring to the synthesis of compound **1,** compound **28-5** was obtained, and then referring to the synthesis of compound **8,** compound **28** (42 mg, yield: 57%) was obtained as a white solid. LCMS: [M+H]⁺ = 538.2.

¹H NMR (400 MHz, CDCl₃) δ 7.90 (d, *J* = 8.0 Hz, 1H), 7.36 (s, 1H), 7.21 (d, *J* = 8.0 Hz, 1H), 7.00 - 6.95 (m, 1H), 6.63 - 6.55 (m, 1H), 6.50 - 6.42 (m, 1H), 3.90 - 3.78 (m, 4H), 3.62 (d, *J =* 11.2 Hz, 2H), 2.95 - 2.80 (m, 4H), 2.54 - 2.48 (m, 1H), 2.34 - 2.19 (m, 2H), 2.08 - 1.99 (m, 2H), 1.68 (s, 9H), 1.16 - 1.04 (m, 1H), 0.68 - 0.58 (m, 1H), 0.50 - 0.41 (m, 1H), 0.39 - 0.30 (m, 1H), 0.24 - 0.15 (m, 1H).

### Example 29

Synthesis of compound 29, the synthetic route is as follows:

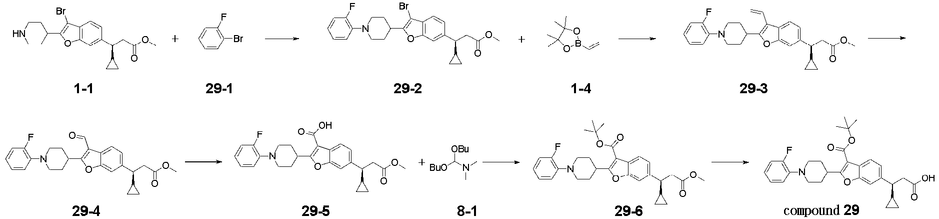

Referring to the synthesis of compound **1,** compound **29-5** was obtained, and then referring to the synthesis of compound **8,** compound **29** (25 mg, yield: 50%) was obtained as a white solid. LCMS: [M+H]⁺ = 508.2.

¹H NMR (400 MHz, CDCl₃) δ 7.91 (d, *J* = 8.0 Hz, 1H), 7.37 (s, 1H), 7.22 (d, *J* = 8.0 Hz, 1H), 7.14 - 7.02 (m, 3H), 7.01 - 6.94 (m, 1H), 3.92 - 3.79 (m, 1H), 3.66 - 3.59 (m, 2H), 2.96 - 2.82 (m, 4H), 2.56 - 2.48 (m, 1H), 2.33 - 2.20 (m, 2H), 2.09 - 2.01 (m, 2H), 1.68 (s, 9H), 1.17 - 1.08 (m, 1H), 0.69 - 0.62 (m, 1H), 0.51 - 0.43 (m, 1H), 0.41 - 0.33 (m, 1H), 0.27 - 0.17 (m, 1H).

### Example 30

Synthesis of compound 30, the synthetic route is as follows:

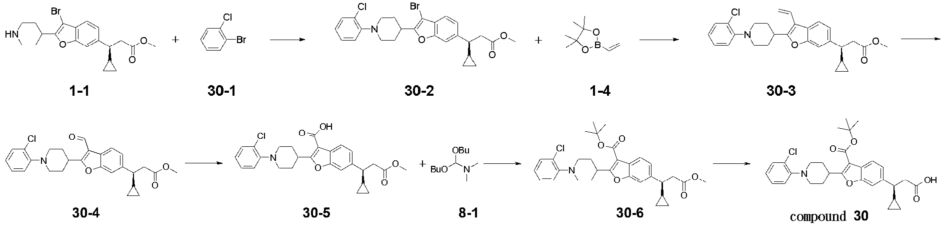

Referring to the synthesis of compound **1,** compound **30-5** was obtained, and then referring to the synthesis of compound **8,** compound **30** (20 mg, yield: 38%) was obtained as a white solid. LCMS: [M+H]⁺ = 524.2.

¹H NMR (400 MHz, CDCl₃) δ 7.91 (d, *J =* 8.0 Hz, 1H), 7.43 - 7.38 (m, 2H), 7.27 - 7.19 (m, 2H), 7.12 (d, *J =* 8.0 Hz, 1H), 7.01 (t, *J =* 7.6 Hz, 1H), 3.90 - 3.82 (m, 1H), 3.58 - 3.52 (m, 2H), 2.92 - 2.81 (m, 4H), 2.56 - 2.48 (m, 1H), 2.35 - 2.23 (m, 2H), 2.09 - 2.02 (m, 2H), 1.68 (s, 9H), 1.17 - 1.07 (m, 1H), 0.68 - 0.58 (m, 1H), 0.52 - 0.42 (m, 1H), 0.40 - 0.31 (m, 1H), 0.25 - 0.18 (m, 1H).

### Example 31

Synthesis of compound 31, the synthetic route is as follows:

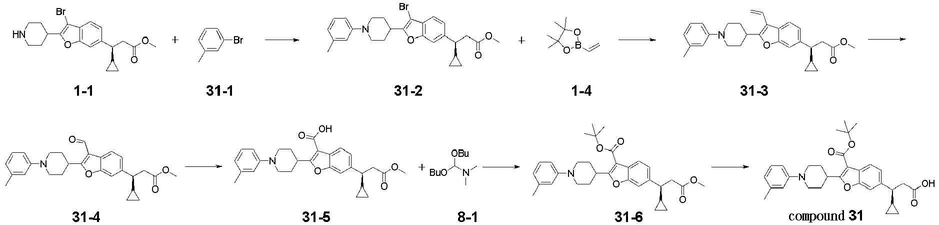

Referring to the synthesis of compound **1,** compound **31-5** was obtained, and then referring to the synthesis of compound **8,** compound **31** (17.2 mg, yield: 19%) was obtained as a white solid. LCMS: [M+H]⁺ = 504.2.

¹H NMR (400 MHz, CDCl₃) δ 8.01 (d, *J =* 8.0 Hz, 1H), 7.47 - 7.38 (m, 2H), 7.31 - 7.22 (m, 2H), 7.18 - 7.06 (m, 2H), 3.95 - 3.83 (m, 3H), 3.15 - 2.87 (m, 4H), 2.65 - 2.45 (m, 1H), 2.32 (s, 3H), 2.23 - 2.03 (m, 2H), 2.09 - 2.04 (m, 2H), 1.71 (s, 9H), 1.17 - 1.08 (m, 1H), 0.72 - 0.63 (m, 1H), 0.55 - 0.43 (m, 1H), 0.43 - 0.32 (m, 1H), 0.30 - 0.16 (m, 1H).

### Example 32:

Synthesis of compound 32, the synthetic route is as follows:

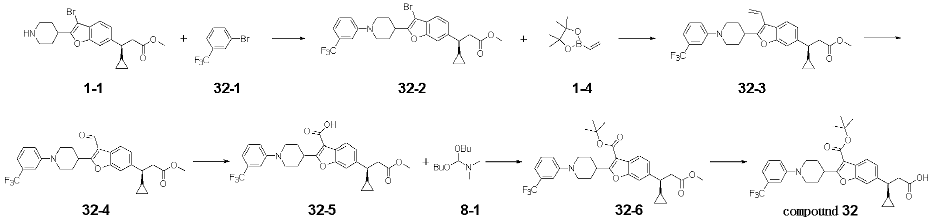

Referring to the synthesis of compound **1,** compound **32-5** was obtained, and then referring to the synthesis of compound **8,** compound **32** (20 mg, yield: 29%) was obtained as a white solid. LCMS: [M+H]⁺ = 558.2.

¹H NMR (400 MHz, CDCl₃) δ 7.91 (d, *J =* 8.0 Hz, 1H), 7.42 - 7.36 (m, 2H), 7.25 - 7.19 (m, 2H), 7.18 - 7.09 (m, 2H), 3.95 - 3.86 (m, 3H), 3.05 - 2.81 (m, 4H), 2.57 - 2.47 (m, 1H), 2.23 - 2.13 (m, 2H), 2.09 - 2.04 (m, 2H), 1.69 (s, 9H), 1.13 - 1.08 (m, 1H), 0.69 - 0.61 (m, 1H), 0.50 - 0.43 (m, 1H), 0.40 - 0.32 (m, 1H), 0.29 - 0.16 (m, 1H).

### Example 33

Synthesis of compound 33, the synthetic route is as follows:

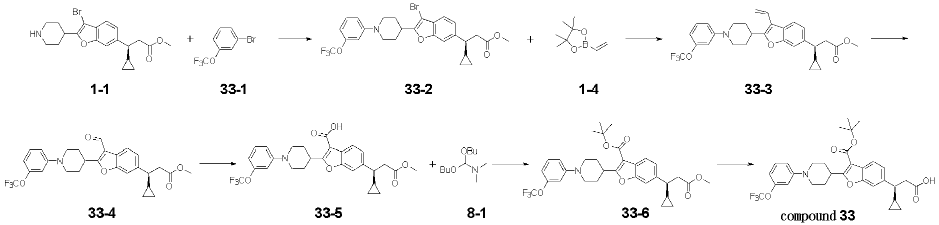

Referring to the synthesis of compound **1,** compound **33-5** was obtained, and then referring to the synthesis of compound **8,** compound **33** (18 mg, yield: 32%) was obtained as a white solid. LCMS: [M+H]⁺ = 574.3.

¹H NMR (400 MHz, CDCl₃) δ 7.91 (d, *J* = 8.0 Hz, 1H), 7.37 (s, 1H), 7.30 - 7.27 (m, 1H), 7.22 (d, *J =* 8.0 Hz, 1H), 6.91 (dd, *J =* 8.4, 2.0 Hz, 1H), 6.83 - 6.79 (m, 1H), 6.73 (d, *J =* 8.0 Hz, 1H), 3.92 - 3.83 (m, 3H), 3.04 - 2.83 (m, 4H), 2.57 - 2.48 (m, 1H), 2.21 - 2.12 (m, 2H), 2.08 - 2.02 (m, 2H), 1.69 (s, 9H), 1.14 - 1.08 (m, 1H), 0.68 - 0.62 (m, 1H), 0.50 - 0.43 (m, 1H), 0.41 - 0.33 (m, 1H), 0.23 - 0.15 (m, 1H).

### Example 34

Synthesis of compound 34, the synthetic route is as follows:

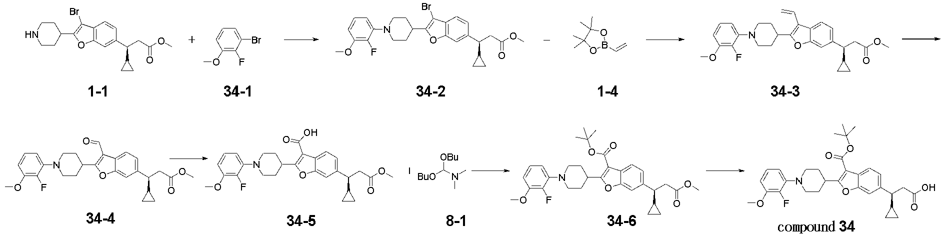

Referring to the synthesis of compound **1,** compound **34-5** was obtained, and then referring to the synthesis of compound **8,** compound **34** (11 mg, yield: 11%) was obtained as a white solid. LCMS: [M+H]⁺ = 538.3.

¹H NMR (400 MHz, CDCl₃) δ 7.91 (d, *J* = 8.0 Hz, 1H), 7.37 (s, 1H), 7.22 (d, *J* = 8.0 Hz, 1H), 7.02 (t, *J =* 8.0 Hz, 1H), 6.71 - 6.66 (m, 2H), 3.92 (s, 3H), 3.90 - 3.80 (m, 1H), 3.62 (d, *J =* 12.0 Hz, 2H), 3.02 - 2.78 (m, 4H), 2.56 - 2.48 (m, 1H), 2.32 - 2.22 (m, 2H), 2.08 - 2.00 (m, 2H), 1.68 (s, 9H), 1.13 - 1.08 (m, 1H), 0.71 - 0.56 (m, 1H), 0.48 - 0.42 (m, 1H), 0.41 - 0.31 (m, 1H), 0.28 - 0.14 (m, 1H).

### Example 35

Synthesis of compound 35, the synthetic route is as follows:

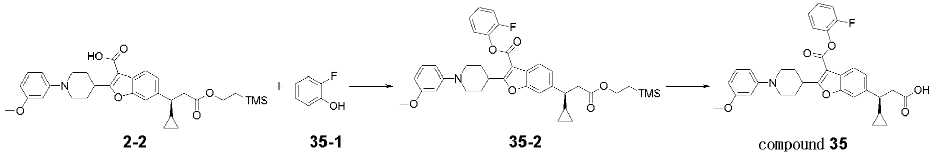

Referring to the synthesis of compound **23,** compound **35-2** was obtained, and then referring to the synthesis of compound **2,** compound **35** (12 mg, yield: 20%) was obtained as a white solid. LCMS: [M+H]⁺ = 558.2.

¹H NMR (400 MHz, CDCl₃) δ 8.02 (d, *J* = 8.0 Hz, 1H), 7.45 (s, 1H), 7.38 - 7.30 (m, 2H), 7.29 - 7.20 (m, 4H), 6.80 - 6.39 (m, 3H), 4.00 - 3.90 (m, 1H), 3.88 - 3.80 (m, 5H), 3.03 - 2.84 (m, 4H), 2.58 - 2.50 (m, 1H), 2.40 - 2.18 (m, 2H), 2.16 - 2.06 (m, 2H), 1.17 - 1.07 (m, 1H), 0.70 - 0.63 (m, 1H), 0.51 - 0.45 (m, 1H), 0.40 - 0.33 (m, 1H), 0.25 - 0.18 (m, 1H).

### Example 36

Synthesis of compound 36, the synthetic route is as follows:

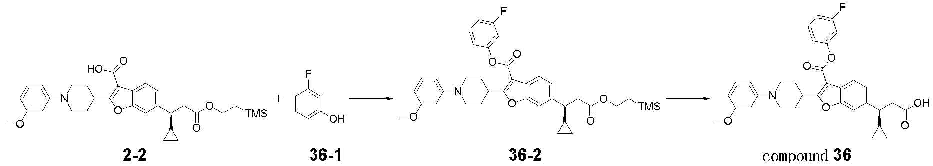

Referring to the synthesis of compound **35,** compound **36** (30 mg, yield: 58%) was obtained as a white solid. LCMS: [M+H]⁺ = 557.2.

¹H NMR (400 MHz, CDCl₃) δ 7.99 (d, *J =* 8.0 Hz, 1H), 7.48 - 7.41 (m, 2H), 7.28 (d, *J* = 8.0 Hz, 1H), 7.22 (t, *J* = 8.0 Hz, 1H), 7.13 - 7.03 (m, 3H), 6.67 - 6.61 (m, 1H), 6.60 - 6.55 (m, 1H), 6.50 - 6.45 (m, 1H), 3.93 - 3.82 (m, 6H), 2.98 - 2.85 (m, 4H), 2.59 - 2.51 (m, 1H), 2.29 - 2.17 (m, 2H), 2.12 - 2.05 (m, 2H), 1.18 - 1.11 (m, 1H), 0.71 - 0.64 (m, 1H), 0.54 - 0.47 (m, 1H), 0.40 - 0.33 (m, 1H), 0.27 - 0.19 (m, 1H).

### Example 37

Synthesis of compound 37, the synthetic route is as follows:

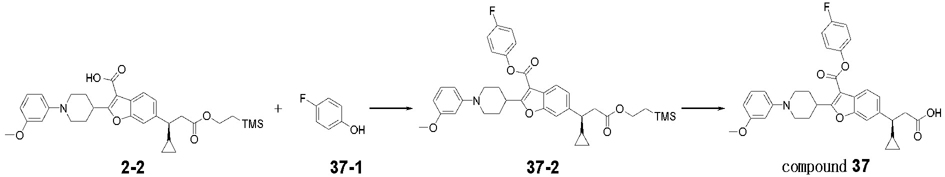

Referring to the synthesis of compound **35,** compound **37** (12 mg, yield: 53%) was obtained as a white solid. LCMS: [M+H]⁺ = 558.2.

¹H NMR (400 MHz, CDCl₃) δ 7.97 (d, *J* = 8.0 Hz, 1H), 7.45 (s, 1H), 7.29 - 7.11 (m, 6H), 6.62 (dd, *J =* 8.0, 2.0 Hz, 1H), 6.57 - 6.52 (m, 1H), 6.46 (dd, *J =* 8.0, 2.0 Hz, 1H), 3.94 - 3.83 (m, 6H), 2.93 - 2.85 (m, 5H), 2.61 - 2.53 (m, 1H), 2.27 - 2.14 (m, 2H), 2.08 - 2.01 (m, 2H), 1.15 - 1.05 (m, 1H), 0.68 - 0.58 (m, 1H), 0.49 - 0.43 (m, 1H), 0.40 - 0.34 (m, 1H), 0.23 - 0.16 (m, 1H).

### Example 38:

Synthesis of compound 38, the synthetic route is as follows:

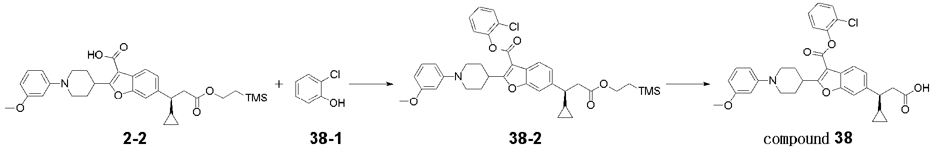

Referring to the synthesis of compound **35,** compound **38** (12 mg, yield: 40%) was obtained as a white solid. LCMS: [M+H]⁺ = 574.2.

¹H NMR (400 MHz, CDCl₃) δ 8.06 (d, *J* = 8.0 Hz, 1H), 7.56 (d, *J* = 7.2 Hz, 1H), 7.44 (s, 1H), 7.42 - 7.30 (m, 3H), 7.29 - 7.26 (m, 1H), 7.22 (t, *J* = 8.0 Hz, 1H), 6.69 - 6.60 (m, 1H), 6.59 - 6.54 (m, 1H), 6.50 - 6.43 (m, 1H), 4.00 - 3.82 (m, 6H), 2.97 - 2.85 (m, 4H), 2.58 - 2.51 (m, 1H), 2.29 - 2.17 (m, 2H), 2.14 - 2.07 (m, 2H), 1.17 - 1.09 (m, 1H), 0.69 - 0.61 (m, 1H), 0.51 - 0.45 (m, 1H), 0.42 - 0.33 (m, 1H), 0.28 - 0.19 (m, 1H).

### Example 39

Synthesis of compound 39, the synthetic route is as follows:

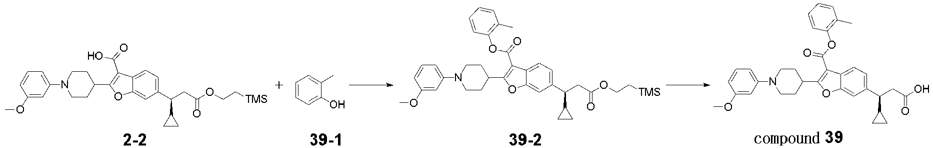

Referring to the synthesis of compound **35,** compound **39** (20 mg, yield: 60%) was obtained as a white solid. LCMS: [M+H]⁺ = 574.2.

¹H NMR (400 MHz, CDCl₃) δ 8.07 (d, *J* = 8.0 Hz, 1H), 7.48 (s, 1H), 7.40 - 7.34 (m, 2H), 7.32 - 7.19 (m, 4H), 6.70 - 6.64 (m, 1H), 6.61 - 6.57 (m, 1H), 6.53 - 6.48 (m, 1H), 4.01 - 3.84 (m, 6H), 3.02 - 2.86 (m, 4H), 2.63 - 2.52 (m, 1H), 2.35 - 2.23 (m, 5H), 2.16 - 2.09 (m, 2H), 1.20 - 1.12 (m, 1H), 0.73 - 0.67 (m, 1H), 0.57 - 0.47 (m, 1H), 0.43 - 0.38 (m, 1H), 0.31 - 0.23 (m, 1H).

### Example 40

Synthesis of compound 40, the synthetic route is as follows:

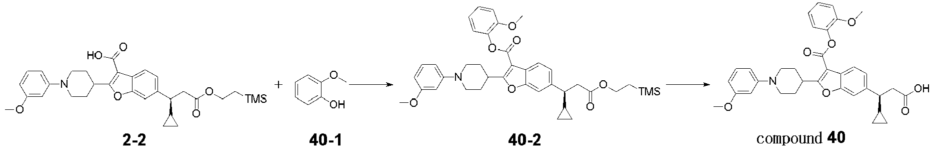

Referring to the synthesis of compound **35,** compound **40** (10 mg, yield: 69%) was obtained as a white solid. LCMS: [M+H]⁺ = 570.3.

¹H NMR (400 MHz, CDCl₃) δ 8.05 (d, *J* = 8.0 Hz, 1H), 7.43 (s, 1H), 7.30 - 7.19 (m, 4H), 7.10 - 7.01 (m, 2H), 6.63 (dd, *J =* 8.0, 2.0 Hz, 1H), 6.57 - 6.54 (m, 1H), 6.46 (dd, *J =* 8.0, 2.0 Hz, 1H), 3.97 - 3.83 (m, 9H), 2.98 - 2.87 (m, 4H), 2.57 - 2.49 (m, 1H), 2.29 - 2.17 (m, 2H), 2.12 - 2.05 (m, 2H), 1.17 - 1.07 (m, 1H), 0.70 - 0.62 (m, 1H), 0.53 - 0.42 (m, 1H), 0.40 - 0.35 (m, 1H), 0.26 - 0.20 (m, 1H).

### Example 41:

Synthesis of compound 41, the synthetic route is as follows:

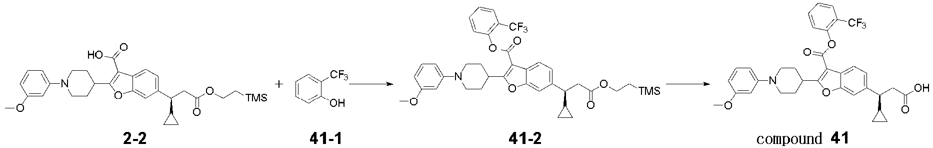

Referring to the synthesis of compound **35,** compound **41** (11 mg, yield: 49%) was obtained as a white solid. LCMS: [M+H]⁺ = 608.3.

¹H NMR (400 MHz, CDCl₃) δ 8.02 (d, *J* = 8.0 Hz, 1H), 7.79 (d, *J* = 8.0 Hz, 1H), 7.69 (t, *J* = 8.0 Hz, 1H), 7.48 - 7.42 (m, 2H), 7.40 (d, *J* = 8.0 Hz, 1H), 7.29 - 7.26 (m, 1H), 7.22 (t, *J* = 8.0 Hz, 1H), 6.65 - 6.61 (m, 1H), 6.57 - 6.53 (m, 1H), 6.49 - 6.43 (m, 1H), 3.94 - 3.82 (m, 6H), 2.98 - 2.87 (m, 4H), 2.59 - 2.50 (m, 1H), 2.27 - 2.16 (m, 2H), 2.09 - 2.03 (m, 2H), 1.16 - 1.09 (m, 1H), 0.72 - 0.63 (m, 1H), 0.54 - 0.46 (m, 1H), 0.43 - 0.34 (m, 1H), 0.28 - 0.20 (m, 1H).

### Example 42

Synthesis of compound 42, the synthetic route is as follows:

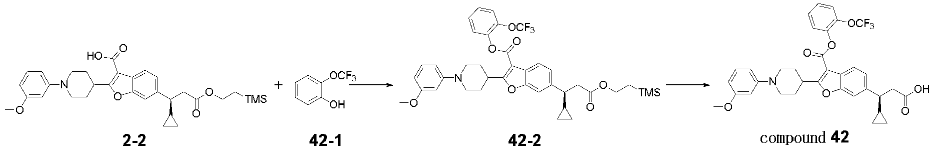

Referring to the synthesis of compound **35,** compound **42** (12 mg, yield: 56%) was obtained as a white solid. LCMS: [M+H]⁺ = 624.3.

¹H NMR (400 MHz, CDCl₃) δ 8.00 (d, *J =* 8.0 Hz, 1H), 7.48 - 7.34 (m, 5H), 7.28 (d, *J =* 8.4 Hz, 1H), 7.21 (t, *J* = 8.0 Hz, 1H), 6.63 (dd, *J =* 8.0, 2.0 Hz, 1H), 6.58 - 6.55 (m, 1H), 6.46 (dd, *J =* 8.0, 2.0 Hz, 1H), 3.93 - 3.82 (m, 6H), 2.95 - 2.84 (m, 4H), 2.57 - 2.50 (m, 1H), 2.26 - 2.19 (m, 2H), 2.10 - 2.04 (m, 2H), 1.18 - 1.10 (m, 1H), 0.70 - 0.63 (m, 1H), 0.54 - 0.45 (m, 1H), 0.41 - 0.35 (m, 1H), 0.27 - 0.18 (m, 1H).

### Example 43

Synthesis of compound 43, the synthetic route is as follows:

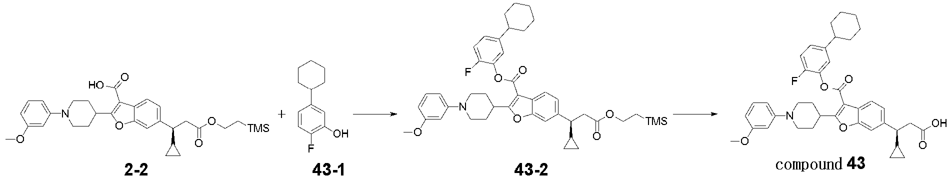

Referring to the synthesis of compound **35,** compound **43** (22 mg, yield: 41%) was obtained as a white solid. LCMS: [M+H]⁺ = 640.3.

¹H NMR (400 MHz, CDCl₃) δ 8.02 (d, *J* = 8.0 Hz, 1H), 7.44 (s, 1H), 7.29 - 7.08 (m, 5H), 6.63 (dd, *J* = 8.0, 2.0 Hz, 1H), 6.57 - 6.55 (m, 1H), 6.46 (dd, *J* = 8.0, 2.0 Hz, 1H), 3.96 - 3.81 (m, 6H), 2.99 - 2.84 (m, 4H), 2.60 - 2.50 (m, 2H), 2.29 - 2.17 (m, 2H), 2.13 - 2.06 (m, 2H), 1.97 - 1.87 (m, 4H), 1.81 - 1.75 (m, 1H), 1.47 - 1.40 (m, 4H), 1.39 - 1.35 (m, 1H), 1.17 - 1.10 (m, 1H), 0.70 - 0.63 (m, 1H), 0.55 - 0.44 (m, 1H), 0.41 - 0.32 (m, 1H), 0.29 - 0.18 (m, 1H).

### Example 44

Synthesis of compound 44, the synthetic route is as follows:

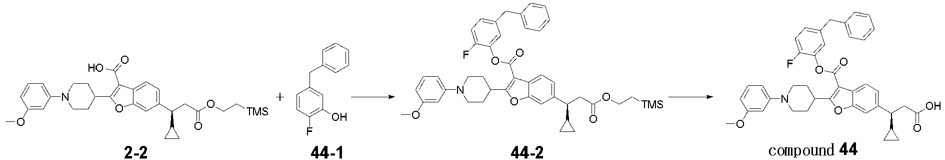

Referring to the synthesis of compound **35,** compound **44** (15 mg, yield: 34%) was obtained as a white solid. LCMS: [M+H]⁺ = 648.3.

¹H NMR (400 MHz, CDCl₃) δ 7.99 (d, *J* = 8.0 Hz, 1H), 7.43 (s, 1H), 7.39 - 7.32 (m, 2H), 7.27 - 7.09 (m, 8H), 6.67 - 6.60 (m, 1H), 6.58 - 6.53 (m, 1H), 6.50 - 6.44 (m, 1H), 4.03 (s, 2H), 3.92 - 3.83 (m, 6H), 2.97 - 2.85 (m, 4H), 2.59 - 2.50 (m, 1H), 2.26 - 2.16 (m, 2H), 2.11 - 2.05 (m, 2H), 1.18 - 1.09 (m, 1H), 0.69 - 0.63 (m, 1H), 0.51 - 0.44 (m, 1H), 0.40 - 0.34 (m, 1H), 0.24 - 0.19 (m, 1H).

### Example 45

Synthesis of compound 45, the synthetic route is as follows:

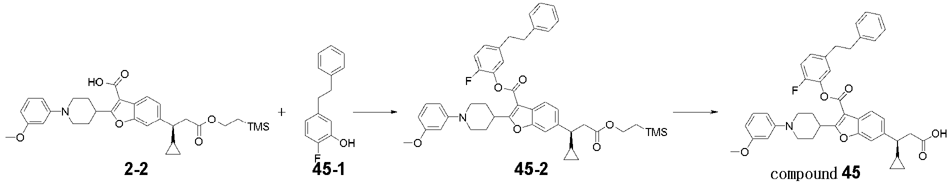

Referring to the synthesis of compound **35,** compound **45** (18 mg, yield: 24%) was obtained as a white solid. LCMS: [M+H]⁺ = 662.3.

¹H NMR (400 MHz, CDCl₃) δ 8.02 (d, *J* = 8.0 Hz, 1H), 7.44 (s, 1H), 7.36 - 7.31 (m, 2H), 7.28 - 7.12 (m, 7H), 7.10 - 7.04 (m, 1H), 6.66 - 6.62 (m, 1H), 6.57 - 6.55 (m, 1H), 6.49 - 6.44 (m, 1H), 3.93 - 3.82 (m, 6H), 2.98 - 2.97 (m, 4H), 2.96 - 2.85 (m, 4H), 2.60 - 2.51 (m, 1H), 2.30 - 2.18 (m, 2H), 2.12 - 2.06 (m, 2H), 1.17 - 1.10 (m, 1H), 0.71 - 0.63 (m, 1H), 0.53 - 0.46 (m, 1H), 0.44 - 0.35 (m, 1H), 0.27 - 0.20 (m, 1H).

### Example 46

Synthesis of compound 46, the synthetic route is as follows:

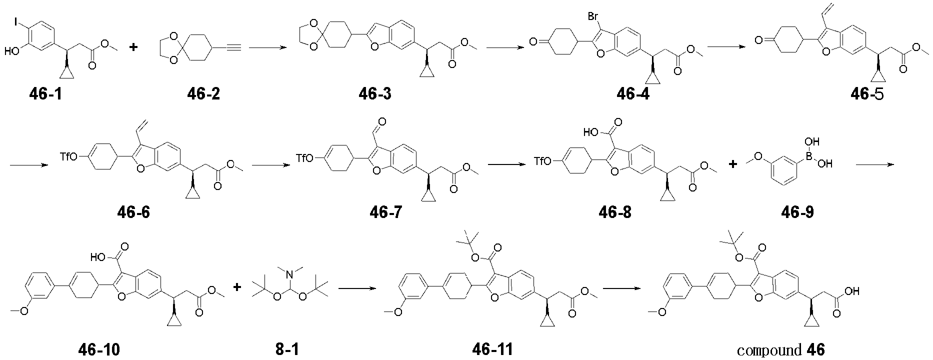

A mixture of compound **46-1** (9.48 g, 27.4 mmol), compound **46-2** (4.55 g, 27.4 mmol), copper(I) iodide (521 mg, 2.74 mmol), bis(triphenylphosphine)palladium(II) chloride (962 mg, 1.37 mmol), triethylamine (8.3 g, 82.2 mmol), and acetonitrile (150 mL) was reacted at 100°C under a nitrogen atmosphere for 16 hours. After the reaction mixture was concentrated, the crude product was purified by normal-phase column chromatography (ethyl acetate: petroleum ether = 20%) to obtain **46-3** (8.75 g, yield: 83%) as a yellow oil. LCMS: [M+H]⁺ = 385.2.

Compound **46-3** (8.75 g, 22.8 mmol) and N-bromosuccinimide (8.1 g, 45.6 mmol) were added to tetrahydrofuran (150 mL) and stirred at room temperature for 1 hour. After the reaction was completed, the reaction mixture was washed with saturated sodium bicarbonate (150 mL). The organic phase was dried, filtered, and concentrated, then purified by normal-phase column chromatography (ethyl acetate: petroleum ether = 20%) to obtain **46-4** (9.12 g, yield: 96%) as a yellow oil. LCMS: [M+H]⁺ = 418.9.

A mixture of compound **46-4** (1.8 g, 4.31 mmol), potassium vinyltrifluoroborate (1.18 g, 8.62 mmol), bis(triphenylphosphine)palladium(II) chloride (7.02 mg), potassium carbonate (1.8 g, 12.93 mmol), *N,N*-dimethylformamide (15 mL), and water (5 mL) was reacted at 100°C under a nitrogen atmosphere for 16 hours. The reaction mixture was diluted with ethyl acetate (100 mL) and filtered. The filtrate was washed with water (100 mL × 2). The organic phase was dried, filtered, and concentrated, then purified by normal-phase column chromatography (ethyl acetate: petroleum ether = 20%) to obtain **46-5** (910 mg, yield: 58%) as a yellow oil. LCMS: [M+H]⁺ = 367.0.

At -78°C under a nitrogen atmosphere, lithium bis(trimethylsilyl)amide (1 M, 3 mL) was slowly added dropwise to a solution of compound **46-5** (910 mg, 2.49 mmol) in tetrahydrofuran (9 mL). After stirring the mixture at -78°C for 1 hour, a solution of *N-*phenyl-bis(trifluoromethanesulfonimide) (978 mg, 2.74 mmol) in tetrahydrofuran (6 mL) was added dropwise. The mixture was first reacted at -78°C for 1 hour, then at room temperature for 1 hour. The reaction mixture was quenched with saturated sodium bicarbonate solution (60 mL) and extracted with ethyl acetate (60 mL × 2). The organic phase was dried, filtered, and concentrated, then purified by normal-phase column chromatography (ethyl acetate: petroleum ether = 30%) to obtain **46-6** (1.2 g, yield: 97%) as a yellow oil. LCMS: [M+H]⁺ = 498.9.

A mixture of compound **46-6** (1.2 g, 2.4 mmol), N-methylmorpholine N-oxide (421 mg, 3.6 mmol), osmium tetroxide dihydrate (9 mg, 0.024 mmol), acetone (15 mL), and water (5 mL) was stirred at room temperature for 16 hours. After adding an aqueous solution (5 mL) of sodium periodate (842 mg, 7.2 mmol) to the reaction mixture, stirring was continued for one hour. The reaction mixture was diluted with ethyl acetate (100 mL) and washed with saturated brine (100 mL). The organic phase was dried, filtered, and concentrated, then purified by normal-phase column chromatography (ethyl acetate: petroleum ether = 30%) to obtain **46-7** (1.2 g, yield: 100%) as a yellow oil. LCMS: [M+H]⁺ = 500.9.

To a mixture of compound **46-7** (1.2 g, 2.4 mmol), 2-methyl-2-butene (3.36 g, 48 mmol), *tert*-butanol (10 mL), and tetrahydrofuran (10 mL) was added an aqueous solution (10 mL) of sodium hydrogen phosphate (4.62 g, 33.6 mmol) and sodium chlorite (1.13 g, 12 mmol). The reaction mixture was stirred at room temperature for 1 hour. The reaction mixture was diluted with water (50 mL) and extracted with ethyl acetate (50 mL × 2). The organic phase was dried, filtered, and concentrated, then purified by normal-phase column chromatography (ethyl acetate: petroleum ether = 50%) to obtain **46-8** (1.2 g, yield: 97%) as a yellow oil. LCMS: [M+H]⁺ = 516.8.

Compound **46-8** (1.2 g, 2.32 mmol), compound **46-9** (527 mg, 3.49 mmol), potassium phosphate (1.48 g, 6.96 mmol), tetrakis(triphenylphosphine)palladium (268 mg, 0.232 mmol), dioxane (10 mL), and water (2 mL) were stirred at 100°C for 2 hours. The reaction mixture was filtered and concentrated, then purified by normal-phase column chromatography (ethyl acetate: petroleum ether = 50%) to obtain compound **46-10** (810 mg, yield: 74%) as a yellow solid. LCMS: [M+H]⁺ = 474.8.

Referring to the synthesis of compound **8,** compound **46** (11 mg, yield: 34%) was obtained as a white solid. LCMS: [M+H]⁺ = 539.3.

¹H NMR (400 MHz, CDCl₃) δ 7.92 (d, *J* = 8.0 Hz, 1H), 7.37 (s, 1H), 7.29 - 7.26 (m, 1H), 7.22 (d, *J* = 8.0 Hz, 1H), 7.05 (d, *J =* 7.6 Hz, 1H), 7.01 - 6.97 (m, 1H), 6.87 - 6.80 (m, 1H), 6.22 (d, *J =* 4.4 Hz, 1H), 4.09 - 3.93 (m, 1H), 3.87 (s, 3H), 2.94 - 2.80 (m, 2H), 2.73 - 2.57 (m, 4H), 2.55 - 2.47 (m, 1H), 2.25 - 2.12 (m, 2H), 1.67 (s, 9H), 1.17 - 1.04 (m, 1H), 0.69 - 0.59 (m, 1H), 0.51 - 0.42 (m, 1H), 0.39 - 0.31 (m, 1H), 0.25 - 0.17 (m, 1H).

### Example 47

Synthesis of compound 47, the synthetic route is as follows:

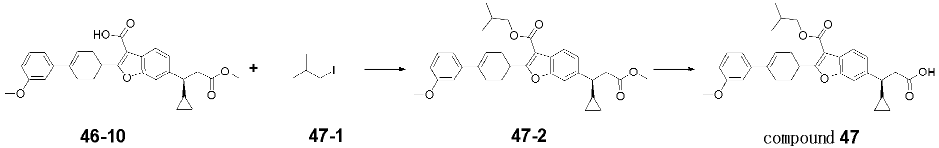

Referring to the synthesis of compound **13,** compound **47** (86 mg, yield: 74%) was obtained as a white solid. LCMS: [M+H]⁺ = 517.1.

¹H NMR (400 MHz, CDCl₃) δ 7.95 (d, *J* = 8.0 Hz, 1H), 7.39 (s, 1H), 7.30 - 7.28 (m, 1H), 7.24 (d, *J =* 8.0 Hz, 1H), 7.05 (d, *J =* 7.6 Hz, 1H), 7.01 - 6.98 (m, 1H), 6.87 - 6.82 (m, 1H), 6.22 (d, *J =* 4.4 Hz, 1H), 4.19 (d, *J =* 6.4 Hz, 2H), 4.11 - 4.00 (m, 1H), 3.87 (s, 3H), 2.95 - 2.81 (m, 2H), 2.74 - 2.57 (m, 4H), 2.56 - 2.49 (m, 1H), 2.26 - 2.18 (m, 2H), 2.17 - 2.12 (m, 1H), 1.17 - 1.07 (m, 7H), 0.70 - 0.61 (m, 1H), 0.52 - 0.43 (m, 1H), 0.40 - 0.32 (m, 1H), 0.26 - 0.18 (m, 1H).

### Example 48

Synthesis of compound 48, the synthetic route is as follows:

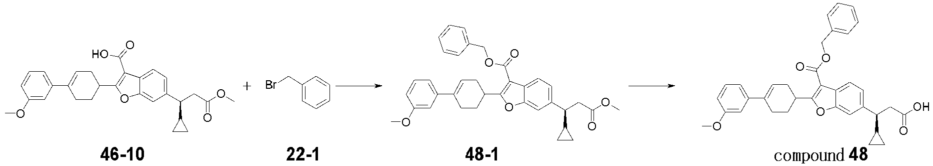

Referring to the synthesis of compound **22,** compound **48** (4.9 mg, yield: 11%) was obtained as a white solid. LCMS: [M+H]⁺ = 551.1.

¹H NMR (400 MHz, CDCl₃) δ 7.86 (d, *J =* 8.0 Hz, 1H), 7.48 - 7.42 (m, 2H), 7.40 - 7.32 (m, 4H), 7.24 - 7.21 (m, 1H), 7.16 (d, *J =* 8.0 Hz, 1H), 6.99 (d, *J =* 7.6 Hz, 1H), 6.93 (s, 1H), 6.79 (d, *J =* 7.6 Hz, 1H), 6.19 - 6.12 (m, 1H), 5.38 (s, 2H), 4.06 - 3.94 (m, 1H), 3.82 (s, 3H), 2.91 - 2.74 (m, 2H), 2.68 - 2.51 (m, 4H), 2.49 - 2.42 (m, 1H), 2.19 - 2.06 (m, 2H), 1.12 - 0.98 (m, 1H), 0.66 - 0.54 (m, 1H), 0.48 - 0.37 (m, 1H), 0.35 - 0.26 (m, 1H), 0.20 - 0.09 (m, 1H).

### Example 49

Synthesis of compound 49, the synthetic route is as follows:

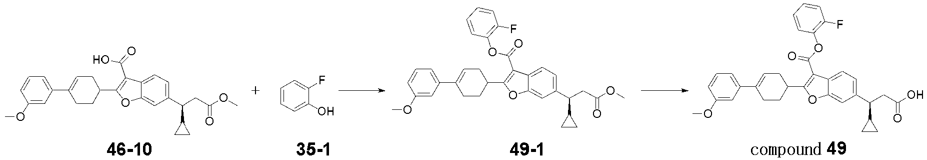

Referring to the synthesis of compound **23,** compound **49** (14 mg, yield: 16%) was obtained as a white solid. LCMS: [M+H]⁺ = 554.2.

¹H NMR (400 MHz, CDCl₃) δ 8.03 (d, *J* = 8.0 Hz, 1H), 7.45 (s, 1H), 7.34 (t,*J* = 8.0 Hz, 1H), 7.29 - 7.20 (m, 5H), 7.03 (d, *J =* 8.0 Hz, 1H), 6.99 - 6.95 (m, 1H), 6.85 - 6.80 (m, 1H), 6.24 - 6.18 (m, 1H), 4.13 - 4.00 (m, 1H), 3.85 (s, 3H), 2.98 - 2.82 (m, 2H), 2.77 - 2.59 (m, 4H), 2.58 - 2.49 (m, 1H), 2.32 - 2.14 (m, 2H), 1.18 - 1.07 (m, 1H), 0.71 - 0.61 (m, 1H), 0.53 - 0.44 (m, 1H), 0.42 - 0.33 (m, 1H), 0.27 - 0.18 (m, 1H).

### Example 50

Synthesis of compound 50, the synthetic route is as follows:

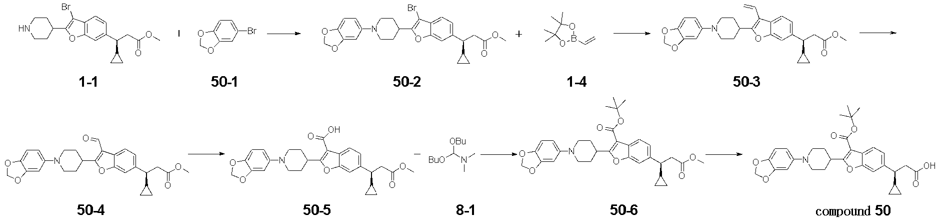

Referring to the synthesis of compound **1,** compound **50-5** was obtained, and then referring to the synthesis of compound **8,** compound **50** (55 mg, yield: 25%) was obtained as a white solid. LCMS: [M+H]⁺ = 534.3.

¹H NMR (400 MHz, CDCl₃) δ 7.90 (d, *J* = 8.0 Hz, 1H), 7.36 (s, 1H), 7.21 (dd, *J* = 8.0, 1.6 Hz, 1H), 6.77 (d, *J =* 8.4 Hz, 1H), 6.67 (d, *J =* 2.4 Hz, 1H), 6.48 (d, *J =* 8.4 Hz, 1H), 5.94 (s, 2H), 3.86 - 3.74 (m, 1H), 3.63 (d, *J* = 12.0 Hz, 2H), 2.93 - 2.78 (m, 4H), 2.56 - 2.48 (m, 1H), 2.26 - 2.16 (m, 2H), 2.06 - 1.98 (m, 2H), 1.67 (s, 9H), 1.14 - 1.06 (m, 1H), 0.67 - 0.60 (m, 1H), 0.48 - 0.42 (m, 1H), 0.38 - 0.32 (m, 1H), 0.22 - 0.16 (m, 1H).

### Example 51

Synthesis of compound 51, the synthetic route is as follows:

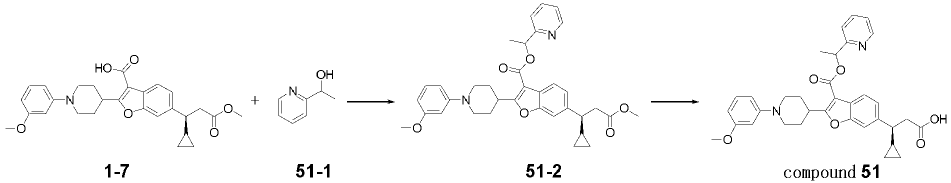

Referring to the synthesis of compound **20,** compound **51** (51.6 mg, yield: 46%) was obtained as a white solid. LCMS: [M+H]⁺ = 569.3.

¹H NMR (400 MHz, CDCl₃) δ 8.71 - 8.66 (m, 1H), 8.03 (d, *J =* 8.0 Hz, 1H), 7.74 (t, *J =* 8.0 Hz, 1H), 7.47 (d, *J =* 7.6 Hz, 1H), 7.39 (s, 1H), 7.29 - 7.17 (m, 3H), 6.67 - 6.60 (m, 1H), 6.58 - 6.53 (m, 1H), 6.49 - 6.43 (m, 1H), 6.26 - 6.18 (m, 1H), 3.94 - 3.79 (m, 6H), 2.95 - 2.81 (m, 4H), 2.57 - 2.48 (m, 1H), 2.22 - 2.12 (m, 2H), 2.06 - 1.97 (m, 2H), 1.81 (d, *J* = 6.0 Hz, 3H), 1.17 - 1.03 (m, 1H), 0.72 - 0.60 (m, 1H), 0.51 - 0.42 (m, 1H), 0.41 - 0.31 (m, 1H), 0.25 - 0.14 (m, 1H).

### Example 52

Synthesis of compound 52, the synthetic route is as follows:

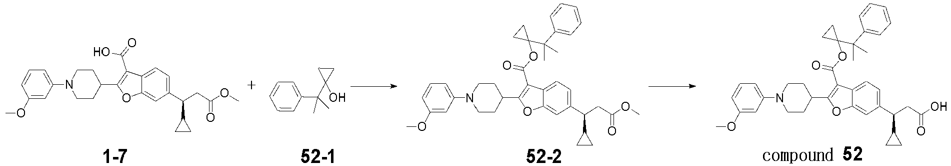

Referring to the synthesis of compound **11,** compound **52** (12.7 mg, yield: 14%) was obtained as a white solid. LCMS: [M+H]⁺ = 622.2.

¹H NMR (400 MHz, CDCl₃) δ 7.56 - 7.48 (m, 3H), 7.32 - 7.26 (m, 3H), 7.23 - 7.16 (m, 2H), 7.07 (dd, *J =* 8.0, 1.2 Hz, 1H), 6.62 - 6.56 (m, 1H), 6.53 - 6.48 (m, 1H), 6.45 - 6.39 (m, 1H), 3.81 (s, 3H), 3.77 - 3.71 (m, 2H), 3.59 - 3.51 (m, 1H), 2.87 - 2.75 (m, 4H), 2.48 - 2.42 (m, 1H), 2.08 - 1.98 (m, 2H), 1.85 - 1.78 (m, 2H), 1.45 (s, 6H), 1.12 - 1.00 (m, 5H), 0.63 - 0.57 (m, 1H), 0.45 - 0.38 (m, 1H), 0.33 - 0.27 (m, 1H), 0.18 - 0.12 (m, 1H).

### Experimental Example

This experimental example is based on NFAT-RE reporter gene activity assay to detect the *in vitro* agonist activity of compounds on GPR40.

### 1. Method

### 1.1 Construction and preparation of plasmid pcDNA3.0-flag-FFAR1 (GPR40)

The pcDNA3.0-flag-FFAR1 (GPR40) plasmid was constructed using conventional molecular cloning methods. The main steps were as follows: The full-length cDNA sequence of human FFAR1(GPR40) (NM_005303.3) was inserted into the *Hind*III and *Xba*I restriction sites of the pcDNA3.0 vector via PCR technology, resulting in the pcDNA3.0-flag-FFAR1(GPR40) plasmid; pGL4.30[luc2P NFAT-RE](# E8481) and pRL-TK (#E2241) plasmids were both purchased from Promega; the plasmids were transformed into DH5α *E. coli* using the CaCl₂ method, further cultured and amplified, and then purified using a plasmid extraction kit (TIANGEN, # DP117) to obtain the corresponding plasmid DNA.

### 1.2 Co-transfection of HEK293T cells with plasmids and compound treatment

HEK293T cells were seeded in a 96-well plate at a density of 1 × 10⁴/well one day prior to plasmid transfection. Cell transfection was performed according to the instructions of the transfection reagent FuGENE^{®} HD (Promega, # E2311). The main steps were as follows: Taking one well as an example, plasmids pcDNA3.0-flag-FFAR1 (GPR40), pGL4.30 [luc2P NFAT-RE], and pRL-TK were added at ratios of 50 ng, 50 ng, and 5 ng, respectively, into 10 µL of Opti-MEM^{™} I medium (Gibco, #11058021) and mixed thoroughly; then, 0.2 µL of FuGENE^{®} HD was added, mixed thoroughly, and incubated at room temperature for 5 minutes; finally, this 10 µL mixture was added to the cell well containing 100 µL of culture medium. 24 hours after co-transfection, the compounds were serially diluted at half-logarithmic intervals with 1 µM as the highest concentration, resulting in 10 concentration gradients, which were added to the cell culture medium for a 4-hour treatment. Two replicate wells were set up for each condition, with compound TAK875 ( 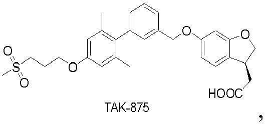 previously entered Phase III clinical trials) serving as the positive control.

### 1.3 Dual-Glo Luciferase assay

After 4 hours of compound treatment, the cells were assayed according to the instructions of the Dual-Glo^{®} Luciferase Assay System (Promega, # E2940). The main steps were as follows: 50 µL of culture medium was aspirated from each well and discarded, followed by the addition of 50 µL of Dual-Glo^{®} Luciferase Reagent, and the mixture was shaken at room temperature for 10 minutes. Then, 80 µL of the lysate reaction solution was transferred to a white opaque optiPlate-96 well plate, and the luminescence signal of Firefly luciferase (Firefly-Luc) was measured using an MD i3x multifunctional microplate reader. Subsequently, 40 µL of Dual-Glo^{®} Stop & Glo^{®} Reagent was added, and the mixture was shaken at room temperature for 10 minutes. Finally, the luminescence signal of Renilla luciferase (Renilla-Luc) was measured using the MD i3x multifunctional microplate reader. The ratio of Firefly-Luc/Renilla-Luc was used as the compound's agonist activity toward GPR40, and normalization was performed using the ratio of the solvent DMSO group. A dose-response curve was fitted using a four-parameter model with GraphPad Prism 8.0 software to calculate the EC50 value.

### 2. Results

**Table 1. In vitro activity assay results**

| Cpd ID | Nuclear factor of activated T-cells reporter gene activity assay | | Cpd ID | Nuclear factor of activated T-cells reporter gene activity assay | |
|---|---|---|---|---|---|
| | (NFAT reporter assay) | | | (NFAT reporter assay) | |
| | EC₅₀ (µM) | Efficacy (%) | | EC₅₀ (µM) | Efficacy (%) |
| TAK875 | * | 100 | Compound **27** | **** | ††† |
| Compound **1** | NA | NA | Compound **28** | *** | †† |
| Compound **2** | ** | ††† | Compound **29** | *** | †††† |
| Compound **3** | ** | †† | Compound **30** | *** | †† |
| Compound **4** | *** | ††† | Compound **32** | ** | †† |
| Compound **5** | ** | ††† | Compound **33** | * | †† |
| Compound **6** | *** | ††† | Compound **34** | **** | ††† |
| Compound **7** | * | †† | Compound **35** | *** | ††† |
| Compound **8** | **** | †† | Compound **36** | *** | †† |
| Compound **9** | **** | †† | Compound **37** | ** | ††† |
| Compound **10** | *** | ††† | Compound **38** | *** | †† |
| Compound **11** | **** | ††† | Compound **39** | *** | ††† |
| Compound **12** | **** | †††† | Compound **40** | *** | †† |
| Compound **14** | *** | †† | Compound **41** | *** | †† |
| Compound **15** | *** | †† | Compound **42** | *** | † |
| Compound **16** | *** | †† | Compound **43** | ** | †† |
| Compound **17** | ** | ††† | Compound **44** | *** | ††† |
| Compound **18** | ** | †† | Compound **45** | *** | ††† |
| Compound **19** | *** | †††† | Compound **46** | ** | ††† |
| Compound **20** | *** | †† | Compound **47** | *** | ††† |
| Compound **21** | *** | ††† | Compound **48** | * | ††† |
| Compound **22** | *** | †† | Compound **49** | ** | ††† |
| Compound **23** | ** | ††† | Compound **50** | **** | †† |
| Compound **24** | *** | ††† | Compound **51** | ** | †† |
| Compound **25** | * | †† | Compound **52** | *** | ††† |
| Compound **26** | **** | †† | | | |

| | | | | | |
|---|---|---|---|---|---|
| *: EC50 > 0.1 µM; **: 0.1 µM ≥ EC50 > 0.01 µM; ***: 0.01 µM ≥ EC50 > 0.001 µM; ****: 0.001 µM ≥ EC50; NA indicates no activity. †: 100 ≥ Efficacy(%) > 50; ††: 150 ≥ Efficacy(%) > 100; †††: 200 ≥ Efficacy(%) > 150; ††††: Efficacy(%) > 200. | | | | | |

From the above experimental results, it can be seen that the compounds of the present invention exhibit favorable GPR40 activity.

Although specific embodiments of the present invention have been described above, those skilled in the art should understand that these are merely illustrative examples, and various changes or modifications can be made to these embodiments without departing from the principles and essence of the present invention. Therefore, the scope of protection of the present invention shall be defined by the appended claims.

## Claims

1. A compound of formula I, a pharmaceutically acceptable salt thereof, a solvate thereof, or a solvate of the pharmaceutically acceptable salt thereof: 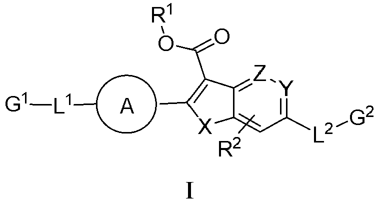
wherein X is O or NR³, and R³ is H or C₁-C₆ alkyl;
Z and Y are independently CH or N;
G¹ is H, C₁-C₆ alkyl, C₁-C₆ alkyl substituted by one or more G¹⁻¹, C₆-C₁₄ aryl, C₆-C₁₄ aryl substituted by one or more G¹⁻², 5- to 10-membered heteroaryl, 5- to 10-membered heteroaryl substituted by one or more G¹⁻³, C₁-C₆ alkoxy, C₁-C₆ alkoxy substituted by one or more G^{l-4}, C₂-C₆ alkenyl, C₂-C₆ alkenyl substituted by one or more G¹⁻⁵, C₂-C₆ alkynyl, C₂-C₆ alkynyl substituted by one or more G¹⁻⁶, C₃-C₈ cycloalkyl, C₃-C₈ cycloalkyl substituted by one or more G^{l-7}, C₃-C₈ cycloalkenyl, C₃-C₈ cycloalkenyl substituted by one or more G¹⁻⁸, 3- to 8-membered heterocycloalkyl, 3- to 8-membered heterocycloalkyl substituted by one or more G¹⁻⁹, 3- to 8-membered heterocycloalkenyl, or 3- to 8-membered heterocycloalkenyl substituted by one or more G¹⁻¹⁰;
each G¹⁻¹, each G¹⁻², each G¹⁻³, each G¹⁻⁴, each G¹⁻⁵, each G¹⁻⁶, each G¹⁻⁷, each G¹⁻⁸, each G¹⁻⁹, and each G¹⁻¹⁰ is independently deuterium, halogen, cyano, -NG¹⁻¹⁻¹G¹⁻¹⁻², -NC(=O)G¹⁻¹⁻³G¹⁻¹⁻⁴ , hydroxyl, -S(=O)₂-C₁-C₆ alkyl, C₁-C₆ alkyl, C₁-C₆ alkyl substituted by one or more G¹⁻¹⁻⁵, C₁-C₆ alkoxy, C₁-C₆ alkoxy substituted by one or more G¹⁻¹⁻⁶, -S-C₁-C₆ alkyl, -S-C₁-C₆ alkyl substituted by one or more G¹⁻¹⁻⁷, C₃-C₈ cycloalkyl, C₃-C₈ cycloalkyl substituted by one or more G¹⁻¹⁻⁸, -O-C₃-C₈ cycloalkyl, -O-C₃-C₈ cycloalkyl substituted by one or more G¹⁻¹⁻⁹, or - C(=O)NG¹⁻¹⁻¹¹G¹⁻¹⁻¹²;
or, any two adjacent G¹⁻², together with the carbon atom to which they are attached, form a 3- to 8-membered heterocycloalkyl, a 3- to 8-membered heterocycloalkyl substituted by one or more G¹⁻¹⁻⁹, a C₃-C₈ cycloalkyl, or a C₃-C₈ cycloalkyl substituted by one or more G¹⁻¹⁻¹⁰;
G¹⁻¹⁻¹, G¹⁻¹⁻², G¹⁻¹⁻³, G¹⁻¹⁻⁴, G¹⁻¹⁻¹¹, and G¹⁻¹⁻¹² are independently H, C₁-C₆ alkyl, C₃-C₈ cycloalkyl, 5- to 10-membered heteroaryl, or 5- to 10-membered heteroaryl substituted by one or more G¹⁻¹⁻¹⁰⁻¹;
each G¹⁻¹⁻¹⁰⁻¹ is independently C₁-C₆ alkyl;
each G¹⁻¹⁻⁵, each G¹⁻¹⁻⁶, each G¹⁻¹⁻⁷, each G¹⁻¹⁻⁸, each G¹⁻¹⁻⁹, and each G¹⁻¹⁻¹⁰ is independently halogen, oxo, C₁-C₆ alkyl, or C₃-C₈ cycloalkyl;
L¹ is a bond or C₁-C₆ alkylene;
ring A is C₄-C₆ cycloalkyl, C₄-C₆ cycloalkyl substituted by one or more A¹, C₄-C₆ cycloalkenyl, C₄-C₆ cycloalkenyl substituted by one or more A², 4- to 6-membered heterocycloalkyl, 4- to 6-membered heterocycloalkyl substituted by one or more A¹, 4- to 6-membered heterocycloalkenyl, or 4- to 6-membered heterocycloalkenyl substituted by one or more A⁴; the 4- to 6-membered heterocycloalkyl, the 4- to 6-membered heterocycloalkyl substituted by one or more A¹, the 4- to 6-membered heterocycloalkenyl, and the 4- to 6-membered heterocycloalkenyl substituted by one or more A⁴ have 1 or 2 heteroatoms independently selected from one or more types of N, S, and O;
each A¹, each A², each A³, and each A⁴ is independently deuterium, halogen, cyano, -NA¹⁻¹A¹⁻², -NC(=O)A¹⁻³A¹⁻⁴ , hydroxyl, C₁-C₆ alkyl, C₁-C₆ alkyl substituted by one or more A¹⁻⁵, C₁-C₆ alkoxy, or C₁-C₆ alkoxy substituted by one or more A¹⁻⁶;
A¹⁻¹, A¹⁻², A¹⁻³, and A¹⁻⁴ are independently deuterium, halogen, cyano, C₁-C₆ alkyl, or C₁-C₆ alkoxy;
each A¹⁻⁵ and each A¹⁻⁶ is independently hydrogen, deuterium, halogen, cyano, C₁-C₆ alkyl, or C₁-C₆ alkoxy;
R¹ is C₁-C₆ alkyl, C₇-C₁₂ linear alkyl, C₂-C₁₂ alkenyl, C₂-C₆ alkynyl, C₃-C₁₀ cycloalkyl, C₆-C₁₄ aryl, 5- to 10-membered heteroaryl, C₁-C₆ alkyl substituted by one or more R¹⁻¹, C₇-C₁₂ linear alkyl substituted by one or more R¹⁻¹, C₂-C₁₂ alkenyl substituted by one or more R¹⁻¹, C₂-C₆ alkynyl substituted by one or more R¹⁻¹, C₃-C₁₀ cycloalkyl substituted by one or more R¹⁻², C₆-C₁₄ aryl substituted by one or more R¹⁻³, or 5- to 10-membered heteroaryl substituted by one or more R¹⁻⁴;
each R¹⁻¹, each R¹⁻², each R¹⁻³, and R¹⁻⁴ is independently halogen, C₁-C₆ alkyl, C₃-C₈ cycloalkyl, C₆-C₁₄ aryl, C₁-C₆ alkoxy, 5- to 10-membered heteroaryl, C₁-C₆ alkyl substituted by one or more R¹⁻¹⁻¹, C₃-C₈ cycloalkyl substituted by one or more R¹⁻¹⁻¹, C₆-C₁₄ aryl substituted by one or more R¹⁻¹⁻¹, C₁-C₆ alkoxy substituted by one or more R¹⁻¹⁻¹, or 5- to 10-membered heteroaryl substituted by one or more R¹⁻¹⁻¹;
R¹⁻¹⁻¹ is halogen, C₁-C₆ alkyl, or C₆-C₁₄ aryl;
R² is hydrogen, deuterium, halogen, cyano, C₁-C₆ alkyl, or C₁-C₆ alkoxy;
L² is C₁-C₆ alkylene, C₁-C₆ alkylene substituted by one or more L²⁻¹, C₃-C₈ cycloalkylene, or C₃-C₈ cycloalkylene substituted by one or more L²⁻²;
each L²⁻¹ and each L²⁻² is independently halogen, C₁-C₆ alkyl, C₁-C₆ alkyl substituted by one or more L²⁻¹⁻¹, C₁-C₆ alkoxy, C₁-C₆ alkoxy substituted by one or more L²⁻¹⁻², C₃-C₈ cycloalkyl, C₃-C₈ cycloalkyl substituted by one or more L²⁻¹⁻³, C₂-C₆ alkynyl, or C₂-C₆ alkynyl substituted by one or more L²⁻¹⁻⁴;
each L²⁻¹⁻¹, each L²⁻¹⁻², each L²⁻¹⁻³, and each L²⁻¹⁻⁴ is independently C₃-C₈ cycloalkyl or C₃-C₈ cycloalkyl substituted by one or more L²⁻¹⁻¹⁻¹;
G² is -C(=O)G²⁻¹, -C(=O)NG²⁻²G²⁻³, or 5- to 10-membered heteroaryl;
G²⁻¹ is hydroxyl, C₁-C₆ alkyl, or -O-NH₂;
G²⁻² and G²⁻³ are independently H, -S(=O)₂-C₁-C₆ alkyl, C₃-C₈ cycloalkyl, C₁-C₆ alkyl, or C₁-C₆ alkyl substituted by one or more G²⁻²⁻¹;
each G²⁻²⁻¹ is independently carboxyl or -S(=O)₂OH;
each 5- to 10-membered heteroaryl, each 3- to 8-membered heterocycloalkenyl, and each 3- to 8-membered heterocycloalkyl independently has 1, 2, 3, or 4 heteroatoms independently selected from one or more types of N, S, and O.

2. The compound of formula I, the pharmaceutically acceptable salt thereof, the solvate thereof, or the solvate of the pharmaceutically acceptable salt thereof according to claim 1, wherein the compound is represented by formula Ia, preferably represented by formula Ia1: 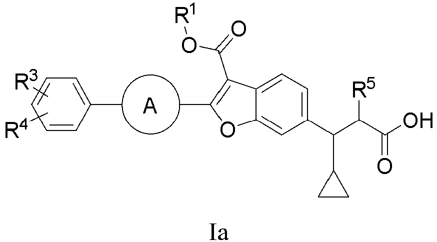 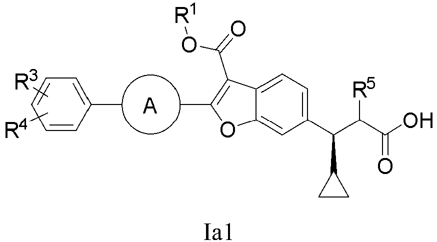 wherein
ring A is C₄-C₆ cycloalkyl, C₄-C₆ cycloalkyl substituted by one or more A¹, C₄-C₆ cycloalkenyl, C₄-C₆ cycloalkenyl substituted by one or more A², 4- to 6-membered heterocycloalkyl, 4- to 6-membered heterocycloalkyl substituted by one or more A¹, 4- to 6-membered heterocycloalkenyl, or 4- to 6-membered heterocycloalkenyl substituted by one or more A⁴; the 4- to 6-membered heterocycloalkyl, the 4- to 6-membered heterocycloalkyl substituted by one or more A¹, the 4- to 6-membered heterocycloalkenyl, and the 4- to 6-membered heterocycloalkenyl substituted by one or more A⁴ have 1 or 2 heteroatoms independently selected from one or more types of N, S, and O;
each A¹, each A², each A³, and each A⁴ is independently deuterium, halogen, cyano, -NA¹⁻¹A¹⁻², -NC(=O)A¹⁻³A¹⁻⁴, hydroxyl, C₁-C₆ alkyl, C₁-C₆ alkyl substituted by one or more A¹⁻⁵, C₁-C₆ alkoxy, or C₁-C₆ alkoxy substituted by one or more A¹⁻⁶;
A¹⁻¹, A¹⁻², A¹⁻³, and A¹⁻⁴ are independently deuterium, halogen, cyano, C₁-C₆ alkyl, or C₁-C₆ alkoxy;
each A¹⁻⁵ and each A¹⁻⁶ is independently hydrogen, deuterium, halogen, cyano, C₁-C₆ alkyl, or C₁-C₆ alkoxy;
R³ and R⁴ are independently H, deuterium, halogen, cyano, -NR³⁻¹R³⁻², -NC(=O)R³⁻³R³⁻⁴, hydroxyl, -S(=O)₂-C₁-C₆ alkyl, C₁-C₆ alkyl, C₁-C₆ alkyl substituted by one or more R³⁻⁵, C₁-C₆ alkoxy, C₁-C₆ alkoxy substituted by one or more R³⁻⁶, -S-C₁-C₆ alkyl, -S-C₁-C₆ alkyl substituted by one or more R³⁻⁷, C₃-C₈ cycloalkyl, C₃-C₈ cycloalkyl substituted by one or more R³⁻⁸, -O-C₃-C₈ cycloalkyl, -O-C₃-C₈ cycloalkyl substituted by one or more R³⁻⁹, or -C(=O)NR³⁻¹⁰R³⁻¹¹;
or, any two adjacent R³ and R⁴, together with the carbon atom to which they are attached, form a 3- to 8-membered heterocycloalkyl, a 3- to 8-membered heterocycloalkyl substituted by one or more R³⁻⁸, a C₃-C₈ cycloalkyl, or a C₃-C₈ cycloalkyl substituted by one or more R³⁻⁸;
R³⁻¹, R³⁻², R³⁻³, R³⁻⁴, R³⁻¹⁰, and R³⁻¹¹ are independently H, C₁-C₆ alkyl, C₃-C₈ cycloalkyl, 5- to 10-membered heteroaryl, or 5- to 10-membered heteroaryl substituted by one or more R³⁻¹⁰⁻¹;
each R³⁻¹⁰⁻¹ is independently C₁-C₆ alkyl;
each R³⁻⁵, each R³⁻⁶, each R³⁻⁷, each R³⁻⁸, and each R³⁻⁹ is independently halogen, oxo, C₁-C₆ alkyl, or C₃-C₈ cycloalkyl;
R¹ is C₁-C₆ alkyl, C₇-C₁₂ linear alkyl, C₂-C₁₂ alkenyl, C₂-C₆ alkynyl, C₃-C₁₀ cycloalkyl, C₆-C₁₄ aryl, 5- to 10-membered heteroaryl, C₁-C₆ alkyl substituted by one or more R¹⁻¹, C₇-C₁₂ linear alkyl substituted by one or more R¹⁻¹, C₂-C₁₂ alkenyl substituted by one or more R¹⁻¹, C₂-C₆ alkynyl substituted by one or more R¹⁻¹, C₃-C₁₀ cycloalkyl substituted by one or more R¹², C₆-C₁₄ aryl substituted by one or more R¹⁻³, or 5- to 10-membered heteroaryl substituted by one or more R¹⁻⁴;
each R¹⁻¹, each R¹⁻², each R¹⁻³, and R¹⁻⁴ is independently halogen, C₁-C₆ alkyl, C₃-C₈ cycloalkyl, C₆-C₁₄ aryl, C₁-C₆ alkoxy, 5- to 10-membered heteroaryl, C₁-C₆ alkyl substituted by one or more R¹⁻¹⁻¹, C₃-C₈ cycloalkyl substituted by one or more R¹⁻¹⁻¹, C₆-C₁₄ aryl substituted by one or more R¹⁻¹⁻¹, C₁-C₆ alkoxy substituted by one or more R¹⁻¹⁻¹, or 5- to 10-membered heteroaryl substituted by one or more R¹⁻¹⁻¹;
R¹⁻¹⁻¹ is halogen, C₁-C₆ alkyl, or C₆-C₁₄ aryl;
R⁵ is H, halogen, C₁-C₆ alkyl, C₁-C₆ alkyl substituted by one or more R⁵⁻¹, C₁-C₆ alkoxy, C₁-C₆ alkoxy substituted by one or more R⁵⁻², C₃-C₈ cycloalkyl, C₃-C₈ cycloalkyl substituted by one or more R⁵⁻³, C₂-C₆ alkynyl, or C₂-C₆ alkynyl substituted by one or more R⁵⁻⁴;
each R⁵⁻¹, each R⁵⁻², each R⁵⁻³, and each R⁵⁻⁴ is independently C₃-C₈ cycloalkyl.

3. The compound of formula I, the pharmaceutically acceptable salt thereof, the solvate thereof, or the solvate of the pharmaceutically acceptable salt thereof according to claim 2, wherein the compound of formula I, the pharmaceutically acceptable salt thereof, the solvate thereof, or the solvate of the pharmaceutically acceptable salt thereof satisfies one or more of the following conditions:
(1) ring A is 4- to 6-membered heterocycloalkyl or C₄-C₆ cycloalkenyl;
(2) R³ and R⁴ are independently H, halogen, C₁-C₆ alkyl, C₁-C₆ alkyl substituted by one or more R³⁻⁵, C₁-C₆ alkoxy, or C₁-C₆ alkoxy substituted by one or more R³⁻⁶; or, any two adjacent R³ and R⁴, together with the carbon atom to which they are attached, form a 3- to 8-membered heterocycloalkyl, for example, the 3- to 8-membered heterocycloalkyl may be dioxolanyl;
(3) each R³⁻⁵ and each R³⁻⁶ is independently halogen;
(4) R¹ is C₁-C₆ alkyl, C₁-C₆ alkyl substituted by one or more R¹⁻¹, C₇-C₁₂ linear alkyl, C₂-C₁₂ alkenyl, C₂-C₆ alkynyl, C₁-C₆ alkoxy substituted by one or more R¹⁻¹, C₃-C₁₀ cycloalkyl, C₃-C₁₀ cycloalkyl substituted by one or more R¹⁻², C₆-C₁₄ aryl, or C₆-C₁₄ aryl substituted by one or more R¹⁻³;
(5) each R¹⁻¹, each R¹⁻², and each R¹⁻³; is independently halogen, C₁-C₆ alkyl, C₁-C₆ alkyl substituted by one or more ¹⁻¹⁻¹, C₃-C₈ cycloalkyl, C₆-C₁₄ aryl, C₁-C₆ alkoxy, C₁-C₆ alkoxy substituted by one or more R¹⁻¹⁻¹, or 5- to 10-membered heteroaryl;
(6) R¹⁻¹⁻¹ is halogen, C₁-C₆ alkyl, or C₆-C₁₄ aryl;
(7) R⁵ is H or halogen;
preferably, the following conditions are satisfied:
(1) ring A is 4- to 6-membered heterocycloalkyl or C₄-C₆ cycloalkenyl;
(2) R³ and R⁴ are independently H, halogen, C₁-C₆ alkyl, C₁-C₆ alkyl substituted by one or more R³⁻⁵, C₁-C₆ alkoxy, or C₁-C₆ alkoxy substituted by one or more R³⁻⁶; or, any two adjacent R³ and R⁴, together with the carbon atom to which they are attached, form a 3- to 8-membered heterocycloalkyl, for example, the 3- to 8-membered heterocycloalkyl may be dioxolanyl;
(3) each R³⁻⁵ and each R³⁻⁶ is independently halogen;
(4) R¹ is C₁-C₆ alkyl, C₁-C₆ alkyl substituted by one or more R¹⁻¹, C₇-C₁₂ linear alkyl, C₂-C₁₂ alkenyl, C₂-C₆ alkynyl, C₁-C₆ alkoxy substituted by one or more R¹⁻¹, C₃-C₁₀ cycloalkyl, C₃-C₁₀ cycloalkyl substituted by one or more R¹⁻², C₆-C₁₄ aryl, or C₆-C₁₄ aryl substituted by one or more R¹⁻³;
(5) each R¹⁻¹, each R¹⁻², and each R¹⁻³; is independently halogen, C₁-C₆ alkyl, C₁-C₆ alkyl substituted by one or more ¹⁻¹⁻¹, C₃-C₈ cycloalkyl, C₆-C₁₄ aryl, C₁-C₆ alkoxy, C₁-C₆ alkoxy substituted by one or more R¹⁻¹⁻¹, or 5- to 10-membered heteroaryl;
(6) R¹⁻¹⁻¹ is halogen, C₁-C₆ alkyl, or C₆-C₁₄ aryl; and
(7) R⁵ is H or halogen.

4. The compound of formula I, the pharmaceutically acceptable salt thereof, the solvate thereof, or the solvate of the pharmaceutically acceptable salt thereof according to claim 2 or 3, wherein the compound of formula I, the pharmaceutically acceptable salt thereof, the solvate thereof, or the solvate of the pharmaceutically acceptable salt thereof satisfies one or more of the following conditions:
(1) ring A is 4- to 6-membered heterocycloalkyl;
(2) R³ and R⁴ are each independently H or C₁-C₆ alkoxy, and further, the C₁-C₆ alkoxy is located at the meta position;
(3) R⁵ is H;
preferably, the following conditions are satisfied:
(1) ring A is 4- to 6-membered heterocycloalkyl;
(2) R³ and R⁴ are each independently H or C₁-C₆ alkoxy, and further, the C₁-C₆ alkoxy is located at the meta position; and
(3) R⁵ is H.

5. The compound of formula I, the pharmaceutically acceptable salt thereof, the solvate thereof, or the solvate of the pharmaceutically acceptable salt thereof according to claim 2 or 3, wherein the compound of formula I, the pharmaceutically acceptable salt thereof, the solvate thereof, or the solvate of the pharmaceutically acceptable salt thereof satisfies one or more of the following conditions:
(1) in ring A, the C₄-C₆ cycloalkenyl is cyclohexenyl, and the 4- to 6-membered heterocycloalkyl is saturated cycloalkyl containing 1-2 N atoms, which may be, for example, piperidinyl;
(2) in R³ and R⁴, the "C₁-C₆ alkyl" in the C₁-C₆ alkyl and the C₁-C₆ alkyl substituted by one or more R³⁻⁵ is independently methyl, ethyl, *n*-propyl, isopropyl, *n*-butyl, isobutyl, *sec-*butyl, or *tert-*butyl;
(3) in R³ and R⁴, the "C₁-C₆ alkoxy" in the C₁-C₆ alkoxy and the C₁-C₆ alkoxy substituted by one or more R³⁻⁶ is independently methoxy, ethoxy, *n*-propoxy, isopropoxy, *n*-butoxy, isobutoxy, *sec*-butoxy, or *tert*-butoxy;
(4) in R³ and R⁴, the 3- to 8-membered heterocycloalkyl formed by any two adjacent R³ and R⁵, together with the carbon atom to which they are attached, is a 5- to 6-membered heterocycloalkyl containing 1-2 oxygen atoms, for example, the 5- to 6-membered heterocycloalkyl may be dioxolanyl;
(5) in R³ and R⁴, the halogen is fluorine or chlorine;
(6) in each R³⁻⁵ and each R³⁻⁶, the halogen is fluorine;
(7) in R¹, the "C₁-C₆ alkyl" in the C₁-C₆ alkyl and the C₁-C₆ alkyl substituted by one or more R¹⁻¹ is independently methyl, ethyl, *n*-propyl, isopropyl, *n*-butyl, isobutyl, *sec*-butyl, *tert-*butyl, -C₅H₁₁, -C₆H₁₃, 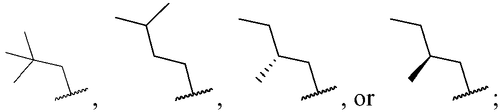
(8) in R¹, the "C₇-C₁₂ linear alkyl" in the C₇-C₁₂ linear alkyl and the C₇-C₁₂ linear alkyl substituted by one or more R¹⁻¹ is C₈-C₁₂ linear alkyl;
(9) in R¹, the "C₂-C₁₂ alkenyl" in the C₂-C₁₂ alkenyl and the C₂-C₁₂ alkenyl substituted by one or more R¹⁻¹ is C₄-C₁₀ alkenyl containing 1-2 double bonds;
(10) in R¹, the "C₂-C₆ alkynyl" in the C₂-C₆ alkynyl and the C₂-C₆ alkynyl substituted by one or more R¹⁻¹ is C₃-C₄ alkynyl containing one triple bond;
(11) in R¹, the "C₁-C₆ alkoxy" in the C₁-C₆ alkoxy and the C₁-C₆ alkoxy substituted by one or more R¹⁻¹ is methoxy, ethoxy, *n*-propoxy, isopropoxy, *n*-butoxy, isobutoxy, *sec*-butoxy, or *tert*-butoxy;
(12) in R¹, the "C₃-C₁₀ cycloalkyl" in the C₃-C₁₀ cycloalkyl and the C₃-C₁₀ cycloalkyl substituted by one or more R¹⁻² is independently cyclopropyl, cyclobutyl, cyclopentyl, or cyclohexyl;
(13) in R¹, the "C₆-C₁₄ aryl" in the C₆-C₁₄ aryl and the C₆-C₁₄ aryl substituted by one or more R¹⁻³; is phenyl;
(14) in each R¹⁻¹, each R¹⁻², each R¹⁻³, and each R¹⁻⁴, the halogen is fluorine;
(15) in each R¹⁻¹, each R¹⁻², each R¹⁻³, and each R¹⁻⁴, the "C₁-C₆ alkyl" in the C₁-C₆ alkyl and the C₁-C₆ alkyl substituted by one or more R¹⁻¹⁻¹ is independently methyl, ethyl, *n*-propyl, isopropyl, *n*-butyl, isobutyl, *sec*-butyl, or *tert-*butyl;
(16) in each R¹⁻¹, each R¹⁻², each R¹⁻³, and each R¹⁻⁴, the C₃-C₈ cycloalkyl is cyclopropyl, cyclobutyl, cyclopentyl, or cyclohexyl;
(17) in each R¹⁻¹, each R¹⁻², each R¹⁻³, and each R¹⁻⁴, the "C₆-C₁₄ aryl" in the C₆-C₁₄ aryl and the C₆-C₁₄ aryl substituted by one or more R¹⁻¹⁻¹ is phenyl;
(18) in each R¹⁻¹, each R¹⁻², each R¹⁻³, and each R¹⁻⁴, the C₁-C₆ alkoxy is independently methoxy or ethoxy;
(19) in each R¹⁻¹, each R¹⁻², each R¹⁻³, and each R¹⁻⁴, the 5- to 10-membered heteroaryl is independently 5- to 6-membered heteroaryl containing one S atom or 5- to 6-membered heteroaryl containing one N atom, further optionally thiophenyl, pyridinyl, or benzothiophenyl;
(20) in each R¹⁻¹⁻¹, the halogen is fluorine, chlorine, or bromine;
(21) in each R¹⁻¹⁻¹, the C₁-C₆ alkyl is methyl or ethyl;
(22) in each R¹⁻¹⁻¹, the C₆-C₁₄ aryl is phenyl;
(23) in R⁵, the halogen is fluorine.

6. The compound of formula I, the pharmaceutically acceptable salt thereof, the solvate thereof, or the solvate of the pharmaceutically acceptable salt thereof according to claim 2, wherein the compound of formula I, the pharmaceutically acceptable salt thereof, the solvate thereof, or the solvate of the pharmaceutically acceptable salt thereof satisfies one or more of the following conditions:
(1) A is 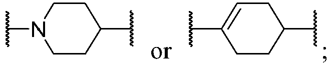
(2) R³ and R⁴ are independently H, -OCH₃, F, Cl, -CH₃, -CF₃, -OCF₃, or 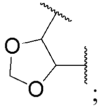
(3) R¹ is methyl, ethyl, propyl, *n*-butyl, 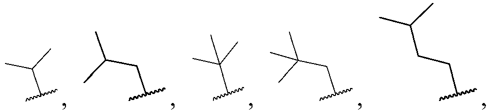 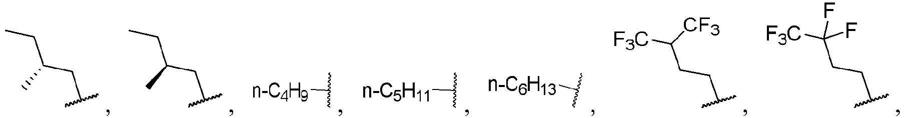 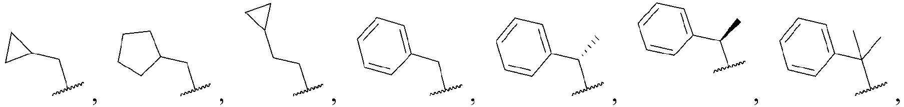 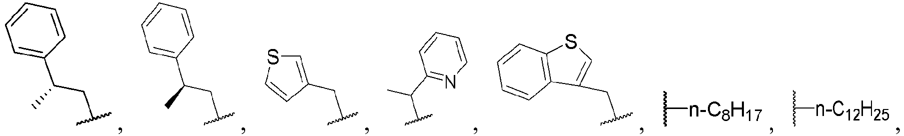 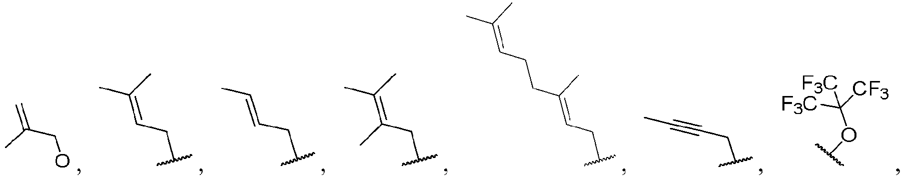 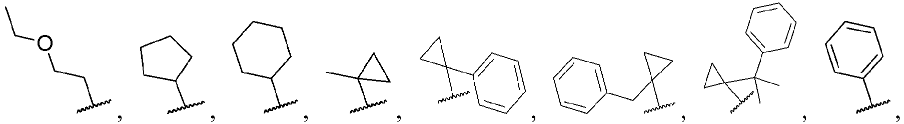 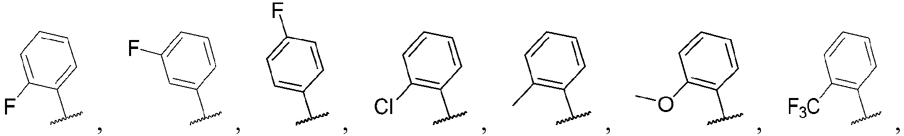 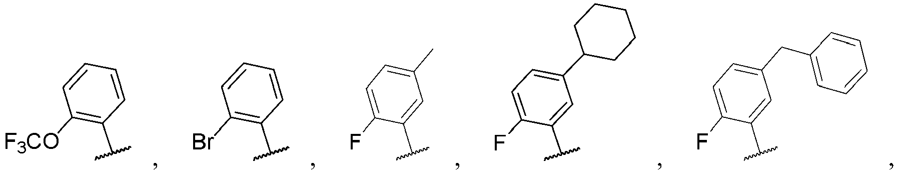 or 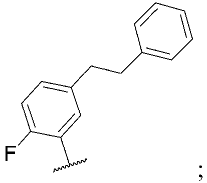
(4) R⁵ is H;
preferably, the following conditions are satisfied:
(1) A is 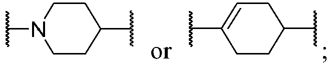
(2) R³ and R⁴ are independently H, -OCH₃, F, Cl, -CH₃, -CF₃, -OCF₃, or 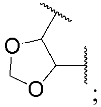
(3) R¹ is methyl, ethyl, propyl, *n*-butyl, 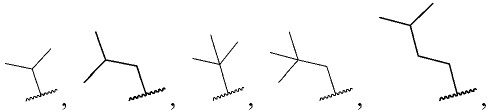 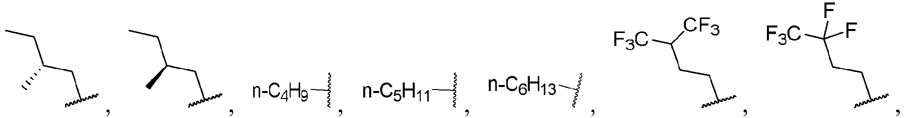 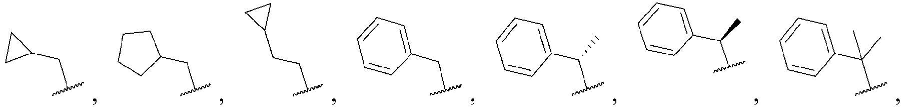 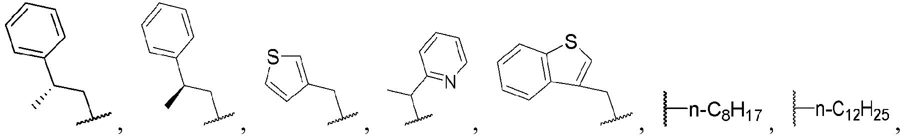 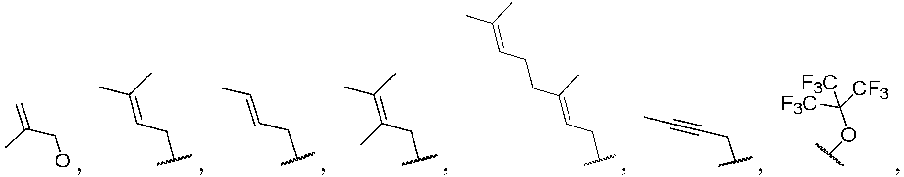 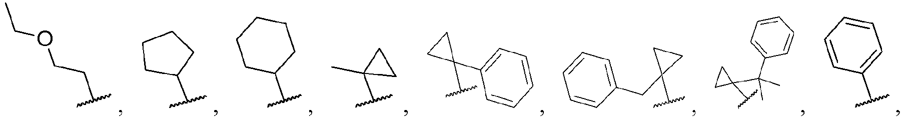 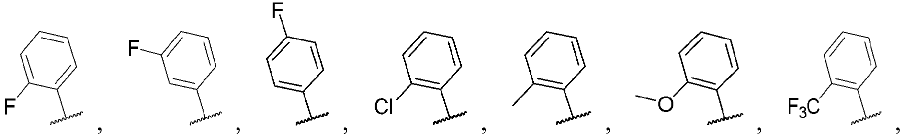 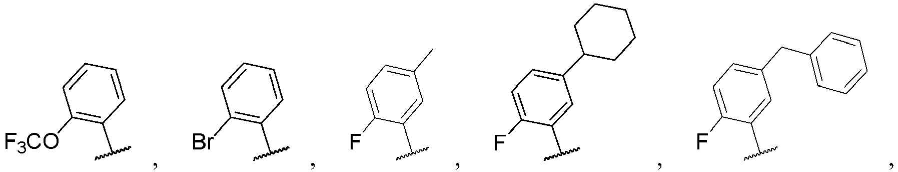 or 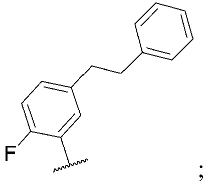 and
(4) R⁵ is H.

7. The compound of formula I, the pharmaceutically acceptable salt thereof, the solvate thereof, or the solvate of the pharmaceutically acceptable salt thereof according to claim 2, wherein the compound of formula I, the pharmaceutically acceptable salt thereof, the solvate thereof, or the solvate of the pharmaceutically acceptable salt thereof satisfies one or more of the following conditions:
(1) R¹ is C₃-C₆ alkyl, C₁-C₆ alkyl substituted by one or more R¹⁻¹, C₃-C₁₀ cycloalkyl, C₃-C₁₀ cycloalkyl substituted by one or more R¹⁻², phenyl, or phenyl substituted by one or more R¹⁻³;
(2) each R¹⁻¹ is independently fluorine or phenyl;
(3) each R¹⁻² is independently C₁-C₃ alkyl or C₁-C₃ alkyl substituted by one or more R¹⁻¹⁻¹;
(4) each R¹⁻³; is independently fluorine, C₁-C₃ alkyl, or C₁-C₃ alkyl substituted by one or more R¹⁻¹⁻¹;
(5) each R¹⁻¹⁻¹ is independently phenyl;
(6) R³ is H, methoxy, or C₁-C₆ alkyl substituted by one or more R³⁻⁵.
(7) R⁴ is methoxy or C₁-C₆ alkyl substituted by one or more R³⁻⁵;
(8) each R³⁻⁵ is fluorine;
preferably, the following conditions are satisfied:
(1) R¹ is C₃-C₆ alkyl, C₁-C₆ alkyl substituted by one or more R¹⁻¹, C₃-C₁₀ cycloalkyl, C₃-C₁₀ cycloalkyl substituted by one or more R¹⁻², phenyl, or phenyl substituted by one or more R¹⁻³;
(2) each R¹⁻¹ is independently fluorine or phenyl;
(3) each R¹⁻² is independently C₁-C₃ alkyl or C₁-C₃ alkyl substituted by one or more R¹⁻¹⁻¹;
(4) each R¹⁻³; is independently fluorine, C₁-C₃ alkyl, or C₁-C₃ alkyl substituted by one or more R¹⁻¹⁻¹;
(5) each R¹⁻¹⁻¹ is independently phenyl;
(6) R³ is H, methoxy, or C₁-C₆ alkyl substituted by one or more R³⁻⁵;
(7) R⁴ is methoxy or C₁-C₆ alkyl substituted by one or more R³⁻⁵; and
(8) each R³⁻⁵ is fluorine.

8. The compound of formula I, the pharmaceutically acceptable salt thereof, the solvate thereof, or the solvate of the pharmaceutically acceptable salt thereof according to claim 1, wherein the compound of formula I is any one of the following compounds:

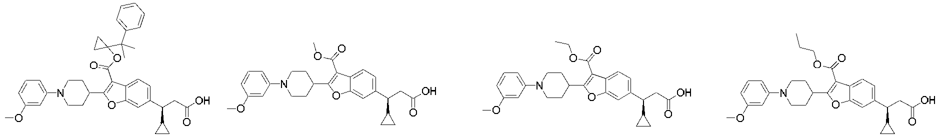

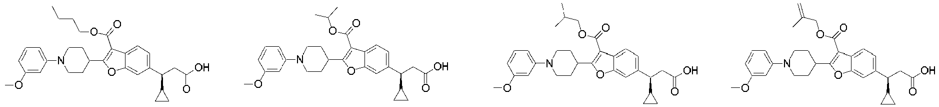

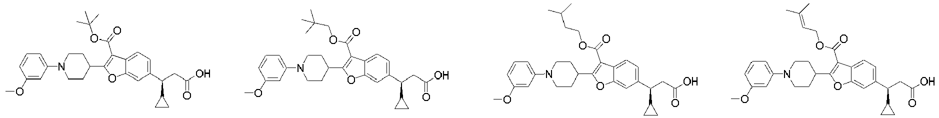

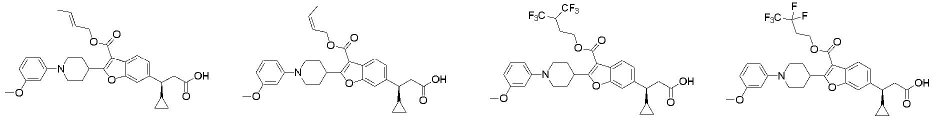

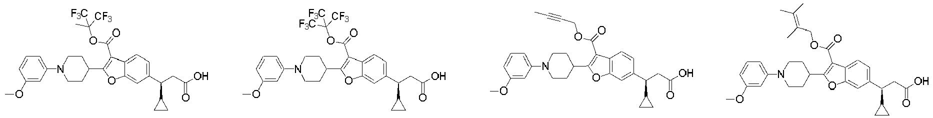

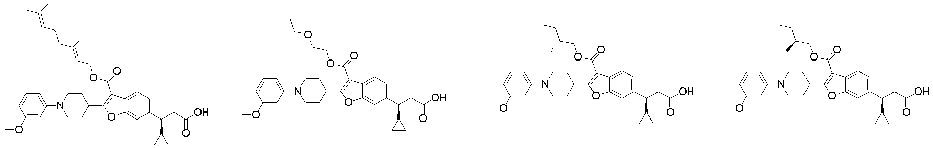

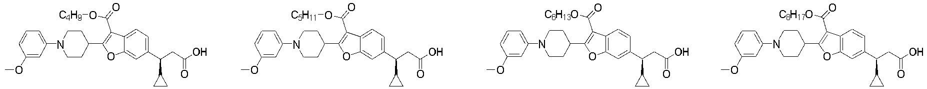

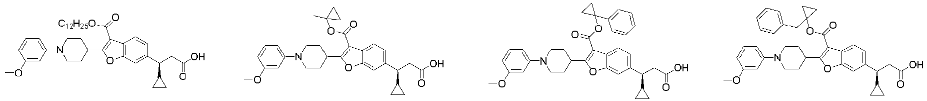

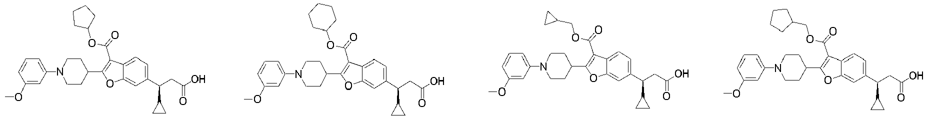

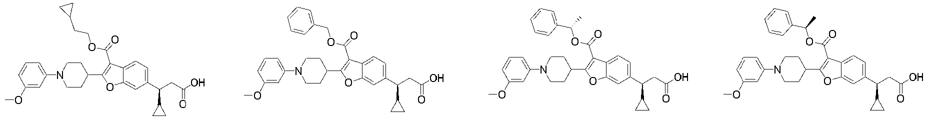

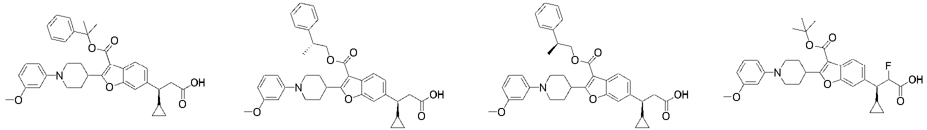

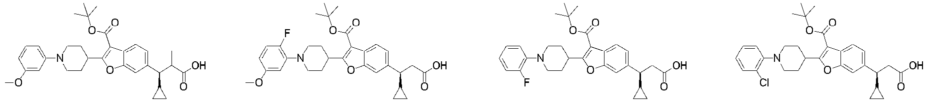

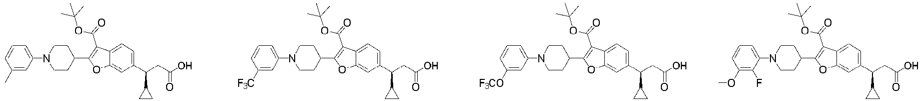

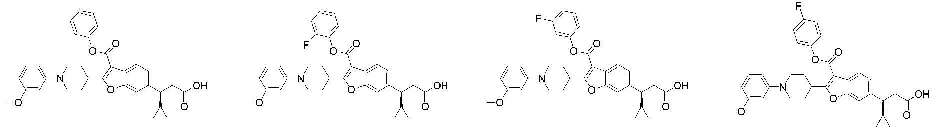

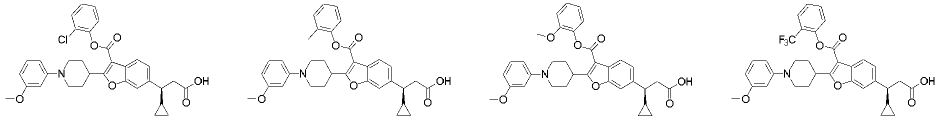

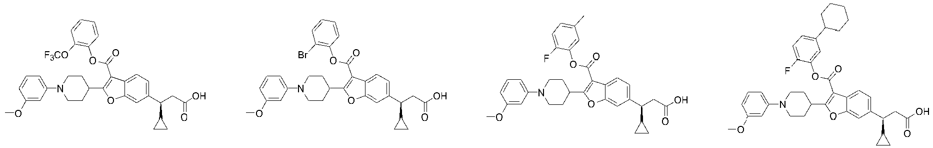

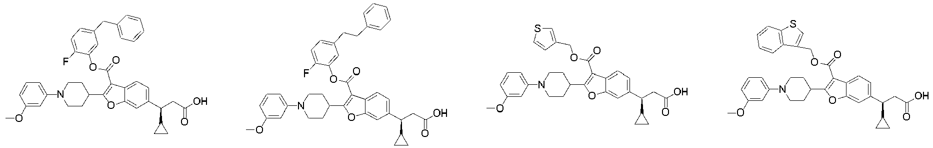

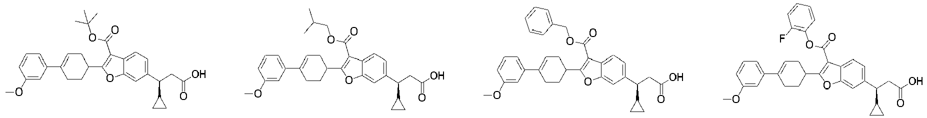

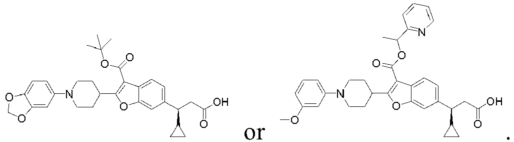

9. A pharmaceutical composition comprising the compound of formula I, the pharmaceutically acceptable salt thereof, the solvate thereof, or the solvate of the pharmaceutically acceptable salt thereof according to any one of claims 1-8, and a pharmaceutically acceptable excipient.

10. Use of the compound of formula I, the pharmaceutically acceptable salt thereof, the solvate thereof, or the solvate of the pharmaceutically acceptable salt thereof according to any one of claims 1-8 in the manufacture of a GPR40 agonist.

11. Use of the compound of formula I, the pharmaceutically acceptable salt thereof, the solvate thereof, or the solvate of the pharmaceutically acceptable salt thereof according to any one of claims 1-8 in the manufacture of a medicament for treating or preventing a GPR40-related disease;
in the use, the GPR40-related disease is, for example, diabetes.
